(19)

Europäisches Patentamt

European Patent Office

Office européen des brevets

(11) **EP 2 792 754 A1**

(12) **EUROPEAN PATENT APPLICATION**

(43) Date of publication:
**22.10.2014 Bulletin 2014/43**

(51) Int Cl.:
***C12Q 1/68*** (2006.01)

(21) Application number: **13164508.7**

(22) Date of filing: **19.04.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **UCB Pharma, S.A.
1070 Brussels (BE)**

(72) Inventors:
• **CARA TERRIBAS, Carlos Javier
28046 Madrid (ES)**

• **OLANO MARTIN, Estibaliz
48160 Derio (ES)**
• **MARTINEZ MARTINEZ, Antonio
48160 Derio (ES)**
• **SIMON BUELA, Laureano
48160 Derio (ES)**
• **GONZALEZ RUIZ, Juan Ramon
08914 Barcelona (ES)**

(74) Representative: **UCB Intellectual Property
c/o UCB Biopharma SPRL
Intellectual Property Department
Allée de la Recherche 60
1070 Brussels (BE)**

(54) **Biomarkers for epilepsy**

(57)     The present invention relates to a group of epilepsy biomarkers comprising (i) SNP rs2740574; (ii) SNP rs1799735; (iii) SNP rs1695; (iv) SNP rs6280; and the locus of the human glutathione S-transferase theta-1 (GSTT1) gene. The invention particularly relates to this group of biomarkers as marker for protection against drug-resistant epilepsy. The group of biomarkers may additionally comprise at least one clinical marker selected from the group comprising (i) a focal seizure with secondary generalization; (ii) age at confirmed diagnosis of epilepsy; (iii) atrophy of hippocampus or medial temporal sclerosis; and (iv) the presence of a brain tumor. The present invention further relates to a method for detecting protection against drug-resistant epilepsy in a subject, a method for monitoring epilepsy therapy, a method of identifying an individual as eligible for an aggressive epilepsy therapy, a composition for detecting protection against drug-resistant epilepsy in a subject, a corresponding kit and a microarray for the analysis of protection against drug-resistant epilepsy.

EP 2 792 754 A1

## SEQUENCE LISTING

```
<110>  UCB Pharma, S.A.

<120>  Biomarkers for epilepsy

<130>  B0083 EP PRIO

<160>  4

<170>  PatentIn version 3.5

<210>  1
<211>  41
<212>  DNA
<213>  Homo sapiens

<400>  1
cagccataga dacaagggca ggagagaggc gatttaatag a                    41


<210>  2
<211>  43
<212>  DNA
<213>  Homo sapiens

<400>  2
taggaagaag ggaaaagaag aggatacttc tctatctctg cag                  43


<210>  3
<211>  41
<212>  DNA
<213>  Homo sapiens

<400>  3
aggacctccg ctgcaaatac gtctccctca tctacaccaa c                    41


<210>  4
<211>  41
<212>  DNA
<213>  Homo sapiens

<400>  4
acaggtgtag ttcaggtggc tactcagctg gctcagagat g                    41
```

**Description**

**FIELD OF THE INVENTION**

The present invention relates to a group of epilepsy biomarkers comprising (i) SNP rs2740574; (ii) SNP rs1799735; (iii) SNP rs1695; (iv) SNP rs6280; and the locus of the human glutathione S-transferase theta-1 (GSTT1) gene. The invention particularly relates to this group of biomarkers as marker for protection against drug-resistant epilepsy. The group of biomarkers may additionally comprise at least one clinical marker selected from the group comprising (i) a focal seizure with secondary generalization; (ii) age at confirmed diagnosis of epilepsy; (iii) atrophy of hippocampus or medial temporal sclerosis; and (iv) the presence of a brain tumor. The present invention further relates to a method for detecting protection against drug-resistant epilepsy in a subject, a method for monitoring epilepsy therapy, a method of identifying an individual as eligible for an aggressive epilepsy therapy, a composition for detecting protection against drug-resistant epilepsy in a subject, a corresponding kit and a microarray for the analysis of protection against drug-resistant epilepsy.

**BACKGROUND OF THE INVENTION**

The World Health Organisation (WHO) and the International League Against Epilepsy (ILAE) define epilepsy as a chronic, recurrent, repetitive neurological disorder consisting of paroxysmal phenomena caused by excessive and chaotic discharges in neuronal brain cells. Its incidence has two peaks: one in childhood and adolescence and a second more marked one over the age of 60. According to the International Bureau for Epilepsy (IBE), some 50 million people suffer from epilepsy worldwide, with 20-30% of them suffering more than one seizure per month (Forsgren et al., Eur J Neurol 2005;12:245-53).

The number of new epilepsy cases per year worldwide ranges between 24 and 53 cases per 100,000 inhabitants. In Europe, from the prevalence studies carried out in different countries and years, it was calculated that 0.9 million children and adolescents, 1.9 million adults of an age between 20 and 64 years, and 0.6 million older person of 65+ years are afflicted by epilepsy (Forsgren et al., Eur J Neurol 2005;12:245-53).

Epilepsy is considered to comprise a diverse set of chronic neurological disorders characterized by seizures. These seizures may be recurrent and unprovoked, or may constitute single seizures combined with brain alterations increasing the chance of future seizures. Epileptic seizures typically result from abnormal, excessive or hypersynchronous neuronal activity in the brain.

The etiology of epilepsy can in certain cases be linked to mutations in several genes that code for protein sub units of voltage-gated and ligand-gated ion channels. A further speculated mechanism for some forms of inherited epilepsy are mutations of genes coding for sodium channel proteins. Accordingly modified or defective sodium channels may be opened for a prolonged period of time, leading to a hyper-excitability of neurons. In consequence, larger than normal amounts of the excitatory neurotransmitter glutamate can be released from the affected neurons and may trigger excessive calcium release in post-synaptic cells. The calcium release may, in turn, be neurotoxic to affected cell. Typically, the hippocampus, which comprises a large number of glutamatergic neurons is highly vulnerable to epileptic seizure, a subsequent spread of excitation, and possible neuronal death. Other possible mechanisms involve mutations leading to a dysfunction of neurotransmitter GABA, as well as mutations in non-ion channel genes.

A consequence of the occurrence of epileptic seizures is an interference of physical, emotional, and social functioning. Further consequences of repeated seizures include neuronal loss, gliosis, parenchymal microhemorrhages, excess of starch bodies, leptomeningeal thickening, and periavascular atrophy.

The classification of epileptic seizures according to the ILAE (International League Against Epilepsy, http://www.ilae-epilepsy.org., visited on 9 Febr. 2010) based on the semiotics of the seizure and the characteristics of the electroencephalogram (EEG), is grouped into: partial seizures (involving a specific area of the cerebral cortex), generalised seizures (involving the entire cerebral cortex) and unclassified seizures. A second classification put forth by the ILAE is based on the differentiation between generalised syndromes; focal syndromes, meaning those related to a location; undetermined syndromes and special syndromes. There are three classes within each category: symptomatic (there is a known etiology causing the seizure), idiopathic (related to genetic disorders), cryptogenic (a symptomatic cause is suspected but it has not been determined).

According to the ILAE Good Clinical Practice Guidelines (Glauser et al., Epilepsia 2006; 47(7):1094-120) when epilepsy is diagnosed initial treatment is monotherapy with an antiepileptic drug (AED) to prevent the patient from having another epileptic seizure. If the seizures continue, the dose is increased and if the seizures persist or if there are signs of intoxication (high doses), the drug is substituted for another monotherapy AED. If there is therapeutic failure with the second AED, it can be substituted for a third monotherapy AED or treatment may be initiated with two AEDs (polytherapy).

Approximately 50% of cases achieve early remission of the seizures with the first treatment. However, around 30% of the patients have no early remission of the seizures or attacks despite suitable treatment with AEDs, and they present difficult to control epilepsy (DICE) or drug-resistant epilepsy (French et al., Neurology 2004;62:1261-73). DICE is associated with premature mortality and an increase in morbidity (Tomson et al., ancet Neurol 2008; 7:1021-31).

According to a recent definition carried out by the ILAE (Kwan et al., Epilepsia 2009) drug-resistant epilepsy can be defined as failure of adequate trials of two tolerated and appropriately chosen and used AED schedules (whether as monotherapies or in combination) to achieve sustained seizure freedom. The sustained absence is defined by the absence of all types of crisis for 12 months or three times the interval between pre-crisis intervention therapy, the longest period. Refractoriness must be considered only if the diagnosis is certain and there is therapeutic failure despite performing an adequate treatment (by both the physician and patient).

The reason why a patient diagnosed with epilepsy becomes a patient resistant to antiepileptic treatments is not completely understood. It has been considered that the non-responsiveness may be present from the start or it may come about with the progression of the disease, or even both. Therefore, some patients with DICE could be identified from the start of the course of the disease, which would enable better initial management (Kwan et al., N Engl J Med 2000; 342:314-9).

Several studies have considered the characterisation of patients with DICE, trying to identify which factors are associated with non-responsiveness or could predict the progress of this condition. Some of these factors are: early-onset epilepsy, cryptogenic or symptomatic aetiology, family history of epilepsy, multiple seizure types, large number of seizures before starting treatment, inadequate response to first AED treatment, complex febrile seizures or febrile epileptic state and generalised epileptiform activity on the surface of the electroencephalogram (EEG) (Shorvon, Epilepsia 1996;37:S1-S3; Perucca, CNS Drugs 1998;10:171-9). Moreover, it has been hypothesized that genetic factors may also play a specific role in the resistance to multiple AEDs (French, Epilepsia 2007; 48:S3-S7). This was demonstrated in a genotypic study carried out by Siddiqui et al. (N Engl J Med 2003; 348:1442-8), which showed a genetic polymorphism in an AED transporter protein (ABCB1) in which it was observed that the patients who responded to treatment compared to those who did not had a greater likelihood of presenting the CC genotype than the TT genotype in the ABCB13435 position. In this context also transporters, e.g. glutathione S-transferase, which are able to facilitate or inhibit the entrance of antiepileptic drugs into cells or the crossing of the blood-brain barrier, as well as mutations in genes encoding such transporters, may be of elevated importance. Other studies detected a genetic association with the targets of the drugs (Tate et al., Proc Natl Acad Sci 2005; 102:5507-12; Escayg et al., Am J Hum Genet 2001;68:866-73) and with the components of immune response system (Pirmohamed et al., Neurology. 2001;56:890-6). Despite this recent progress, an improvement in the understanding of genetic influence in epilepsy is required in order to develop new therapeutic strategies for patients with epilepsy. There is thus a need for diagnostic markers and indirect markers as essential tools to be able to predict which patients will not develop or be protected against difficult to control epilepsy.

## OBJECTS AND SUMMARY OF THE INVENTION

The present invention addresses this need and provides a group of biomarkers wherein said group of biomarkers comprises: (i) SNP rs2740574; (ii) SNP rs1799735; (iii) SNP rs1695; (iv) SNP rs6280; and the locus of the human glutathione S-transferase theta-1 (GSTT1) gene. The inventors found that the group of biomarkers (i) SNP rs2740574; (ii) SNP rs1799735; (iii) SNP rs1695; (iv) SNP rs6280; and the locus of the human glutathione S-transferase theta-1 (GSTT1) gene allows to predict a subform of epilepsy. In particular, it was found that these markers can predict which patients will not develop or be protected against difficult to control epilepsy/drug-resistant epilepsy. In particular, a multivariate model was obtained that includes the genes CYPP3A4 (SNP rs2740574), GSTM3 (SNP rs1799735), GSTP 1 (SNP rs1695), DRD3 (SNP rs6280) and GSTT1. In the sample analysed, a genetic score that presents a predictive power of approximately 60% (AUC=0.599) could be constructed with these genes. The validation of this score using cross-validation suggests that the capacity of this score is 56% (AUC [bootstrap]: 0.56). This result is within the range of typical genetic studies where the variability that can explain a set of SNPs tends to be low. When additional clinical variables are introduced, the predictive power of the model even increases to 75% (AUC: 0.755), thus showing a high prediction capacity. The inclusion of SNPs in a model to predict the protection against difficult to control epilepsy is considered as an at least 5-10% improvement in the predictive capacity. The inclusion of clinical variables further improves the results and allows for the definition of a specific patient collective or patient group to be diagnosed. The invention thus permits to effectively detect a complex disease that has subtypes at both clinical and genetic levels. The demonstration of the existence of a genetic profile related subtype of patients more protected against the risk of drug-resistant epilepsy further allows to understand the predisposing, precipitating and perpetuating factors of drug-resistant epilepsy, to ameliorate the associated symptoms and, in particular, to choose an appropriate therapeutic approach. Furthermore, the addition of a genomic profile including transportation protein polymorphisms (e.g. GSTT1) may be advantageous when deciding on the type of antiepileptic drug to be used during treatment regimes. Thus, the current invention provides for the first time molecular and clinical elements, which are highly relevant in the protection of patients from drug-resistant epilepsy.

In a preferred embodiment of the present invention SNP rs2740574 is represented by a single polymorphic change at position 21 of SEQ ID NO: 1, where in one or two alleles the wildtype nucleotide A is replaced by indicator nucleotide G; SNP rs1799735 is represented by a single polymorphic change at position 21 of SEQ ID NO: 2, where in one or two alleles indicator nucleotides AGG are inserted; SNP rs1695 is represented by a single polymorphic change at position 21 of SEQ ID NO: 3, where in one or two alleles wildtype nucleotide A is replaced by indicator nucleotide G; SNP rs6280 is represented by a single polymorphic change at position 21 of SEQ ID NO: 4, where in one or two alleles wildtype nucleotide C is replaced by indicator nucleotide T; and the human glutathione S-transferase theta-1 (GSTT1) gene in one or two alleles is completely deleted, partially deleted and/or non functional.

In a particularly preferred embodiment, the group of biomarker as defined herein above constitutes a marker for protection against drug-resistant epilepsy.

In a further preferred embodiment, said group of biomarkers additionally comprises at least one clinical marker selected from the group comprising:

    (i) a focal seizure with secondary generalization;
    (ii) age at confirmed diagnosis of epilepsy;
    (iii) atrophy of hippocampus or medial temporal sclerosis; and
    (iv) the presence of a brain tumor.

In a further aspect the present invention relates to a method for detecting protection against drug-resistant epilepsy in a subject comprising the steps of:

    (a) isolating a nucleic acid from a subject's sample; and
    (b) determining the status of a biomarker as defined herein above;

wherein the presence of indicator nucleotides, and the presence of a deleted, partially deleted and/or non-functional GSTT1 gene as defined herein above is indicative for a protection against drug-resistant epilepsy.

In a preferred embodiment of said method the subject has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor.

In a further particular embodiment of the above mentioned method said subject has an age of more than 30 years, more than 40 years, more than 45 years, more than 50 years, more than 55 years or more than 60 years at a confirmed diagnosis of epilepsy.

In a preferred embodiment of any of the mentioned methods, the determination of the nucleotide sequence and/or molecular structure is carried out through allele-specific oligonucleotide (ASO)-dot blot analysis, primer extension assays, iPLEX SNP genotyping, Dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, SNP microarray based analysis, restriction enzyme fragment length polymorphism (RFLP) analysis, targeted resequencing analysis and/or whole genome sequencing analysis.

In a further preferred embodiment, any of the mentioned methods comprises the determination of the haplotype for two copies of the chromosome comprising the SNPs rs2740574; rs1799735; rs1695; and rs6280 and the GSTT1 gene in a subject.

In yet another preferred embodiment, said method for detecting protection against drug-resistant epilepsy in a subject comprises as additional step the determination of a focal seizure with secondary generalization in a subject, the determination of atrophy of hippocampus or medial temporal sclerosis in a subject, the determination of the presence of a brain tumor in a subject and/or the determination of the subject's age at confirmed diagnosis of epilepsy.

In another aspect the present invention relates to a method for monitoring epilepsy therapy of a subject comprising the step of determining the status of a biomarker as defined herein above before and during an epilepsy treatment, optionally also after an epilepsy treatment.

In a preferred embodiment of the method for monitoring epilepsy said subject has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor.

In a further particular embodiment of the above mentioned methods for monitoring epilepsy said subject has an age of more than 30 years, more than 40 years, more than 45 years, more than 50 years, more than 55 years or more than 60 years at a confirmed diagnosis of epilepsy.

In yet another preferred embodiment, said method for monitoring epilepsy further comprises the determination of a focal seizure with secondary generalization in a subject, the determination of atrophy of hippocampus or medial temporal sclerosis in a subject, and/or the determination of the presence of a brain tumor in a subject.

In a preferred embodiment of said method the treatment is a treatment with at least one anticonvulsant selected from the group of lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel and zonisamide, or combinations thereof.

In yet another aspect, the present invention relates to a method of identifying an individual as eligible for an aggressive epilepsy therapy comprising:

(a) determining in a sample obtained from an individual the status of a biomarker as defined herein above;

(b) optionally determining a focal seizure with secondary generalization in a subject, determining atrophy of hippocampus or medial temporal sclerosis in a subject, determining the presence of a brain tumor in a subject and/or determining the subject's age at confirmed diagnosis of epilepsy;

(c) identifying the individual as eligible to receive an aggressive epilepsy therapy where the individual's sample is classified as showing the presence of indicator nucleotides, and the occurrence of a deleted, partially deleted and/or non-functional GSTT1 gene as defined herein above; optionally also showing focal seizure with secondary generalization in a subject, showing no atrophy of hippocampus or medial temporal sclerosis in a subject, showing no brain tumor in a subject and/or wherein the subject's age at confirmed diagnosis of epilepsy is above 45 years.

In a preferred embodiment of the present invention a sample as mentioned above is a mixture of tissues, organs, cells and/or fragments thereof, or a tissue or organ specific sample, such as a tissue biopsy from tongue, pancreas, liver, spleen, ovary, muscle, joint tissue, neural tissue, gastrointestinal tissue, or a body fluid such as blood, serum, saliva, cerebrospinal liquid, liquor or urine.

In another aspect, the present invention relates to a composition for detecting protection against drug-resistant epilepsy in a subject, comprising a nucleic acid affinity ligand for a biomarker as defined herein above.

In a preferred embodiment of the invention said oligonuceotide is a oligonucleotide specific for an indicator nucleotide as defined herein above or the corresponding wildtype nucleotide, or it is specific for the GSTT1 gene. It is particularly preferred that an oligonucleotide has a sequence complementary to an indicator nucleotide as defined herein above, or to the corresponding wildtype nucleotide.

In a further aspect the present invention relates to a kit for detecting protection against drug-resistant epilepsy in a subject, comprising a nucleic acid affinity ligand for a biomarker as defined herein above.

In a preferred embodiment, said kit further comprises an enzyme for primer elongation, nucleotides and/or labeling agents.

In yet another aspect, the present invention relates to a microarray for the analysis of protection against drug-resistant epilepsy in a subject, comprising at least one probe selective for an indicator nucleotide or the corresponding wildtype nucleotide, and the GSTT1 gene as defined herein above.

In a last aspect the present invention relates to a use of a group of biomarkers as defined herein above for detecting protection against drug-resistant epilepsy in a subject, or for screening a population of subjects for the presence of drug-resistant epilepsy. In a specific embodiment of said use, the subject or population of subjects has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. In yet another specific embodimentThe method of claim 24 wherein said subject has an age of more than 30 years, more than 40 years, more than 45 years, more than 50 years, more than 55 years or more than 60 years at a confirmed diagnosis of epilepsy.

## BRIEF DESCRIPTION OF THE FIGURES

**Fig. 1** shows an association analysis SNP by SNP under distinct models of inheritance.

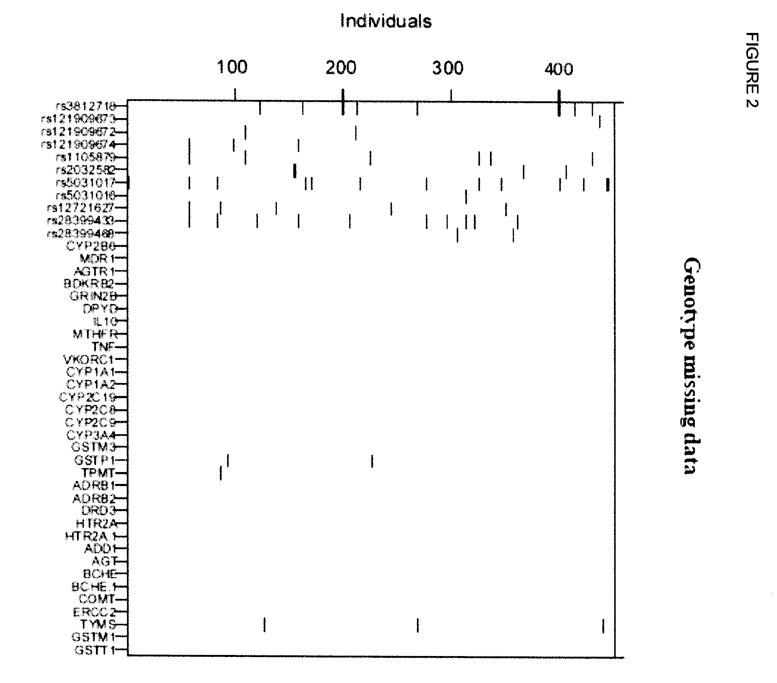

**Fig. 2** provides information of missing genotypes for every individual and SNP.

**Fig. 3** shows the distribution of the number of risk alleles in patients with CE and DICE.

**Fig. 4** shows the area under the ROC curve for the weighted and unweighted models for the prediction of risk of DICE.

**Fig. 5** shows the area under the curve (AUC) for the genetic model plus clinical variables. The genes include in the score are: CYP3A4, GSTM3, GSTP1, DRD3, GSTT1. The clinical variables associated to patients with controlled epilepsia are: focal seizures with secondary generalization (CRFO), result of the cerebral MRI: atrophy of the hippocampus (medial temporal sclerosis), result of the cerebral MRI: tumour and age at confirmed diagnostic of epilepsy.

**Fig. 6** shows a nomogram for the prediction of the probability of CE according to the clinical variables.

**Fig. 7** shows a nomogram for the prediction of the likelihood of CE according to clinical and genetic variables.

**DETAILED DESCRIPTION OF THE INVENTION**

The present invention relates to a group of biomarkers relevant for epilepsy. Although the present invention will be described with respect to particular embodiments, this description is not to be construed in a limiting sense.

Before describing in detail exemplary embodiments of the present invention, definitions important for understanding the present invention are given.

It must be noted that as used herein and in the appended claims, the singular forms "a", "an", and "the", include plural referents unless the context clearly indicates otherwise. For example, the reference to "the method" includes reference to equivalent steps and methods known to those of ordinary skill in the art that could be modified or substituted for the methods described herein.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group, which preferably consists of these embodiments only.

Furthermore, the terms "first", "second", "third" or "(a)", "(b)", "(c)", "(d)" or "(i)", "(ii)", "(iii)", "(iv)", "(v)", "(vi)", "(vii)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)", "(v)", "(vi)", "(vii)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application or claims as set forth herein above or below.

It is to be understood that this invention is not limited to the particular methodology, protocols, proteins, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

As has been set out above, the present invention concerns in one aspect a group of biomarkers comprising: (i) SNP rs2740574; (ii) SNP rs1799735; (iii) SNP rs1695; (iv) SNP rs6280; and the locus of the human glutathione S-transferase theta-1 (GSTT1) gene. The term "biomarker", as used herein, relates to any nucleic acid sequence of any length, or a derivative thereof, which comprises a polymorphic variant as defined in SNP rs2740574; (ii) SNP rs1799735; (iii) SNP rs1695; (iv) SNP rs6280. The term further refers to the locus of the human glutathione S-transferase theta-1 (GSTT1) gene, which may also comprise a polymorphic modification such as a deletion, partial deletion or non-functionality. A biomarker may, for instance, be represented by a nucleic acid molecule of a length of e.g. 1 nt, 2 nt, 3 nt, 4 nt, 5 nt, 10 nt, 15 nt, 20 nt, 25 nt, 30 nt, 35 nt, 40 nt, 45 nt, 50 nt, 60 nt, 70 nt, 80 nt, 90 nt, 100 nt, 200 nt, 300 nt, 400 nt, 500 nt, 1000 nt, 2000 nt, or more or any length in between these lengths. The representing nucleic acid may be any suitable nucleic acid molecule, e.g. a DNA molecule, e.g. a genomic DNA molecule or a cDNA molecule, or a RNA molecule, or a derivative thereof. The biomarker may further be represented by translated forms of the nucleic acid, e.g. a peptide or protein as long as the polymorphic modification leads to a corresponding modification of the peptide or protein. Corresponding information may be readily available to the skilled person from databases such as the NCBI SNP repository and NCBI Genbank.

The "SNP rs2740574" relates to a base change of the human CYP3A4 (1576) gene (NG_008421.1) at position 99382096 of chromosome 7 (Contig NT_007933.15 with contig position 37414939) of assembly GRCh37.p5 of the Reference SNP(refSNP) of NCBI as of 16 December 2012.

The "SNP rs1799735" relates to an insertion modification of the human GSTM3 gene (NM_000849.4.) at position 110280254 of chromosome 1 (Contig NT_032977.9 with contig position 80252172) of assembly GRCh37.p5 of the Reference SNP(refSNP) of NCBI as of 16 December 2012.

The "SNP rs1695" relates to a base change of the human GSTP1 gene (NG_012075.1) at position 67352689 of chromosome 11 (Contig NT_167190.1 with contig position 12658484) of assembly GRCh37.p5 of the Reference SNP(refSNP) of NCBI as of 16 December 2012.

The "SNP rs6280" relates to a base change of the human DRD3 gene (NG_008842.1) at position 113890815 of chromosome 3 (Contig NT_005612.16 with contig position 20385961) of assembly GRCh37.p5 of the Reference SNP(refSNP) of NCBI as of 16 December 2012.

The SNPs as described herein may be present on the Watson or the Crick strand, with presence of the corresponding base. I.e. if, for example, a polymorphism is present on the Watson strand as A, it is present on the Crick strand as T, if the polymorphism is present on the Watson strand as T, it is present on the Crick strand as A, if the polymorphism is present on the Watson strand as G, it is present on the Crick strand as C, and if the polymorphism is present on the Watson strand as C, it is present on the Crick strand as G, and vice versa. Also the insertion or deletion of bases may be detected on the Watson and/or the Crick strand, with correspondence as defined above. For analytic purposes the strand identity may be defined, or fixed, or may be choose at will, e.g. in dependence on factors such the availability of binding elements, GC- content etc. Furthermore, for the sake of accuracy, the SNP may be defined on both strands (Crick and Watson) at the same time, and accordingly be analyzed. A "polymorphic site" as used herein relates to the position of a polymorphism or SNP as described herein within the genome or portion of a genome of a subject, or within a genetic element derived from the genome or portion of a genome of a subject.

The "locus of the human glutathione S-transferase theta-1 (GSTT1) gene" as used herein relates to a genetic region comprising about positions 24376139 to 24384284 on chromosome: 22 (22q11.23) with NC_000022.10 of GRCh37.p10 as of 17 December 2012. The locus may further comprise 5' and/or 3' flanking regions of the indicated position, e.g. 100 bp, 200 bp, 300 bp, 400 bp, 500 bp, 600 bp, 700 bp, 800 bp, 900 bp, 1 kb, 1.5 kb, 2 kb, 2.5 kb, 3 kb, 4 kb or more or any value in between these values.

In a preferred embodiment, SNP rs2740574 is represented by a single polymorphic change at position 21 of SEQ ID NO: 1, where in one or two alleles the wildtype nucleotide A is replaced by indicator nucleotide G. In a further preferred embodiment, SNP rs1799735 is represented by a single polymorphic change at position 21 of SEQ ID NO: 2, where in one or two alleles indicator nucleotides AGG (indicator triplet) are inserted. In another preferred embodiment, SNP rs1695 is represented by a single polymorphic change at position 21 of SEQ ID NO: 3, where in one or two alleles wildtype nucleotide A is replaced by indicator nucleotide G. In yet another preferred embodiment SNP rs6280 is represented by a single polymorphic change at position 21 of SEQ ID NO: 4, where in one or two alleles wildtype nucleotide C is replaced by indicator nucleotide T. In yet another preferred embodiment, the GSTT1 gene locus is deleted, partially deleted and/or modified such that a non-functional GSTT1 expression product is generated.

The term "wildtype sequence" as used herein refers to the sequence of an allele, which does not show the associated epilepsy phenotype according to the present invention, preferably which does not show the associated phenotype of protection against drug-resistant epilepsy. The term may further refer to the sequence of the non phenotype-associated allele with the highest prevalence within a population, preferably within a Caucasian population.

The term "indicator sequence" as used herein refers to the sequence of an allele, which shows an association with an epilepsy phenotype according to the present invention. Preferably, it shows an association with the phenotype of protection against drug-resistant epilepsy. In specific embodiments of the present invention, an indicator sequence may not only be the above indicated allelic sequence but also an independent, further variation from the wildtype sequence as defined herein. The term "indicator nucleotide" as used herein refers to the identity of the nucleotide at the position of a SNP or polymorphic site as defined herein below, associated with an epilepsy phenotype according to the present invention, preferably associated with the phenotype of protection against drug-resistant epilepsy. The term in particular refers to a non-wildtype nucleotide at position 21 of SEQ ID NO: 1, 2, 3 or 4, e.g. as defined herein below.

The term "allele" or "allelic sequence" as used herein refers to a particular form of a gene or a particular nucleotide, preferably a DNA sequence at a specific chromosomal location or locus. In certain embodiments of the present invention a SNP as defined herein may be found at or on one of two alleles in the human genome of a single subject. In further, specific embodiments, a SNP as defined herein may also be found at or on both alleles in the human genome of a single subject. The presence of an indicator nucleotide or an indicator triplet as defined herein on both alleles, or the modification of the GSTT1 gene on both alleles may have a higher predictive value than the presence of an indicator nucleotide or an indicator triplet on one allele only, the other allele comprising a wildtype genotype. Further information on the relevance of one or two alleles in case of the specific SNPs may be derived from Table 6 in the Examples portion. The presence of indicator nucleotides or indicator triplets on one or two alleles is preferably connected with a p-value regarding the significance of prediction for the underlying medical aberration, e.g. epilepsy, preferably protection against drug-resistant epilepsy.

SEQ ID NO: 1 as mentioned herein defines a sequence around SNP rs2740574, wherein at position 21 the wildtype nucleotide A is replaced by an indicator nucleotide, preferably by the nucleotide G. The SNP shows a MAF/MinorAlleleCount of C=0.2015/440.

SEQ ID NO: 2 as mentioned herein defines a sequence around SNP rs1799735, wherein at position 21 indicator nucleotides, preferably the indicator nucleotides AGG, are inserted.

SEQ ID NO: 3 as mentioned herein defines a sequence around SNP rs1695, wherein at position 21 the wildtype nucleotide A is replaced by an indicator nucleotide, preferably by the indicator nucleotide G. The SNP shows a MAF/MinorAlleleCount for G=0.3246/709.

SEQ ID NO: 4 as mentioned herein defines a sequence around SNP rs6280, wherein at position 21 the wildtype nucleotide C is replaced by an indicator nucleotide, preferably by the indicator nucleotide T. The SNP shows a MAF/MinorAlleleCount: C=0.4515/986.

A person skilled in the art would be able to derive the exact position, nucleotide sequence, and indicator sequence from the above identified rs-nomenclature, e.g. from suitable database entries and associated information systems, e.g. the Single Nucleotide Polymorphism database (dbSNP) which is incorporated herein by reference. The information may also be retrievable in case of changes to the nomenclature, or to the surrounding sequence elements, e.g. based on history functions of a suitable database.

In a specific embodiment, the present invention refers to a isolated nucleic acid molecule selected from the group comprising: SEQ ID NO: 1 as mentioned herein or a complementary sequence thereof, SEQ ID NO: 2 as mentioned herein or a complementary sequence thereof, SEQ ID NO: 3 as mentioned herein or a complementary sequence thereof, SEQ ID NO: 4 as mentioned herein or a complementary sequence thereof, and a sequence covering the GSTT1 locus or a fragment thereof as defined herein above. In a preferred embodiment, the isolated nucleic acids comprise the entire group of SEQ ID NO: 1, 2, 3, 4 and a sequence covering the GSTT1 locus or a fragment thereof. The term "isolated nucleic acid molecule" a used herein refers to a nucleic acid entity, e.g. DNA, RNA etc, wherein the entity is substantially free of other biological molecules, such as, proteins, lipids, carbohydrates, other nucleic acids or other material, such as cellular debris and growth media. Generally the term "isolated" is not intended to refer to the complete absence of such material, or to the absence of water, buffers, or salts, unless they are present in amounts which substantially interfere with the methods of the present invention.

In specific embodiments of the present invention the envisaged nucleic acid molecules comprise sequences of SEQ ID NO: 1 to 4, essentially consist of sequences of SEQ ID NO: 1 to 4, or consist of sequences of SEQ ID NO: 1 to 4. For example, the sequences may comprise adjacent regions in the 3' and/or 5' context of SEQ ID NO: 1 to 4, as defined herein, e.g. stretch for about additional 20, 25, 30, 40, 50, 60, 70, 80, 90, 100, 200, 300, 400, 500, 600, 700, 800, 900, 1000, 1500, 2000, 3000, 4000, 5000, 6000, 7000, 10 000 or more nucleotides into 3' and/or 5' direction starting from the herein above indicated genomic positions. In specific embodiments, the nucleic acids may comprise the indicator nucleotide or indicator sequence as described herein.

In further embodiments of the present invention the envisaged nucleic acid molecules may comprise, essentially consist of, or consist of fragments of SEQ ID NO: 1 to 4 which at least have to comprise the polymorphic sites at position 21 of SEQ ID NO: 1, 3 or 4, or the polymorphic sites at positions 21, 22 and 23 of SEQ ID NO: 2. In specific embodiments, the nucleic acids may comprise the indicator nucleotide or indicator sequence as described herein. For example, the present invention relates to sequences of about 900, 800, 700, 600, 500, 400, 300, 200, 100, 90, 80, 70, 60, 50, 40, 30, 20, or 10 or less nucleotides length or any value in between, which have to comprise at least the polymorphic sites at position 21 of SEQ ID NO: 1, 3 or 4, or the polymorphic sites at positions 21, 22 and 23 of SEQ ID NO: 2. The fragments may extend for the indicated length towards the 5' or 3' direction, or in both, the 5' and 3' direction. Preferred are fragments of a length of 20, 50 or 100 nucleotides and less with the SNPs at around position 10 or a corresponding position, i.e. in the center of the sequence. The envisaged nucleic acid molecules may comprise an additional SNP or may not comprise an additional SNP. Such additional SNP may preferably have no diagnostic relevance.

In a further preferred embodiment, the locus of the human glutathione S-transferase theta-1 (GSTT1) gene comprises the human glutathione S-transferase theta-1 (GSTT1) gene in one or two alleles, wherein said gene is completely deleted, partially deleted and/or non functional on one or both alleles. The term "completely deleted" as used herein means that at least the entire open reading frame of the GSTT1 gene is removed or not present in one allele or in both alleles of a human cell. The deletion may further comprise 5' and/or 3' flanks of the open reading frame, e.g. promoter structures, terminator structures, or remotely located structures such as enhancers. The term "partially deleted" as used herein refers to a non-complete removal or a non-complete absence of the GSTT1 gene in one allele or in both alleles of a human cell. The partial deletion may be directed to a part of the open reading frame of the GSTT1, e.g. 5%, 10%, 15%, 20%, 25%, 30%, 35%, 40%, 45%, 50%, 55%, 60%, 65%, 70%, 75%, 80%, 85%, 90%, 95% or even 99%, or any value in between these values, of the number of encoded amino acids or of the encoding bases of the open reading. The partial deletion of the gene may alternatively also be directed to functional gene elements such as promoter or terminator regions, or even enhancer regions. The effect of a partial deletion of the GSTT1 gene is a full or partial reduction in the presence of the GSTT1 expression product, i.e. GSTT1 protein. The expression product may accordingly be present in an amount of 0%, 5%, 10%, 15%, 20%, 25%, 30%, 35%, 40%, 45%, 50%, 55%, 60%, 65%, or 70%, or any value in between these values. The term "non-functional" as used herein means that the GSTT1 expression product is not provided in a fashion as to fulfill its typical biological role or activity. The non-functional expression product may be due to a complete or partial deletion of the GSTT1 gene or open reading frame in one allele or in both alleles of a human cell. The non-functionality may further be due to secondary inhibition processes such as the presence of miRNAs, antisense nucleic acids, siRNAs or other inhibitory elements leading to a degradation of GSTT1 transcripts or the inhibition or translation etc. It is preferred that both alleles of the GSTT1 gene are deleted, partially deleted or non-functional. In a specific embodiment, the presence or absence of a GSTT1 mutation may indicate which type of antiepileptic drug or medicament should be used. E.g. in case of a non-functional GSTT1 a drug may be employed which is not or only partially dependent on GSTT1 transportation, and/or whose pharmacokinetics or pharmocodynamics are not or only partially influenced by the absence of GSTT1. Further details may be derived from suitable literature sources, e.g. from Saruwatari et al., 2010, Pharmaceuticals, 3, 2709-2732.

In very specific embodiments, the present invention also envisages one or more SNPs being in linkage disequilibrium with a SNP as defined herein, or with the modification of the GSTT1 locus as defined herein.

In a particularly preferred embodiment, the group of biomarkers as defined herein above constitutes a marker for protection against drug-resistant epilepsy. The term "drug-resistant epilepsy" as used herein refers to an epilepsy form in which a patient has no early remission of epileptic seizures or attacks despite a treatment with a monotherapy with an antiepileptic drug (AED). This disease form, which is equivalent to a difficult to control epilepsy (DICE), is further associated with premature mortality and an increase in morbidity, and/or with progressive neuronal, cognitive, and psychosocial deterioration. In a specific embodiment, the drug-resistant epilepsy is understood as drug resistant epilepsy according to a definition of the ILAE (e.g. based on Kwan et al., Epilepsia 2009), wherein said drug-resistant epilepsy is defined as an epilepsy form in which there is a failure in the treatment after use of two AEDs, chosen, properly used (monotherapy or in combination) and well tolerated, to get a free state in a sustained crisis. The sustained absence means that there is an absence of all types of crisis for 12 months or three times the interval between pre-crisis intervention therapy, the longest period. In a specific embodiment, the drug-resistant epilepsy may also be a refractory epilepsy. Such an epilepsy form is understood as refractoriness based on a confirmed diagnosis wherein there is therapeutic failure despite performing an adequate treatment, e.g. by both the physician and patient. In specific embodiments of the invention, a drug-resistant epilepsy may be present in a subject from the onset, i.e. without any previous AED treatment, e.g. based on constitutive factors involved in intractability.

The term "protection against drug-resistant epilepsy" as used herein means that a patient or subject will not develop a drug-resistant epilepsy upon treatment with an antiepileptic drug (AED). The protection may, in certain embodiments, be considered as occurrence of remission of epileptic seizures or attacks after a treatment with a monotherapy with an antiepileptic drug (AED) or after a treatment with a combination of AED. In a very specific embodiment, it is a success in the treatment after use of two AEDs, chosen, properly used (monotherapy or in combination) and well tolerated, to get a free state in a sustained crisis as defined herein above. In further embodiments, the protection against drug-resistant epilepsy may be associated with a lack of epilepsy forms associated with progressive neuronal, cognitive, and psychosocial deterioration. It is an advantageous aspect of the present invention that the detection of a protection against drug-resistant epilepsy or even refractory epilepsy allows for a different treatment or therapy management in comparison to patients, who are not protected against drug-resistant epilepsy.

Examples of suitable antiepileptic drugs (AED) include one or more of the anticonvulsants lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel and zonisamide.

In a further embodiment of the invention, the above mentioned group of biomarkers may be combined with at least one clinical marker selected from the group comprising: (i) a focal seizure with secondary generalization; (ii) age at confirmed diagnosis of epilepsy; (iii) atrophy of hippocampus or medial temporal sclerosis; and (iv) the presence of a brain tumor. One or more of these clinical markers may be tested and/or measured at a subject or patient. The group of markers for the protection against drug-resistant epilepsy may accordingly comprise the following combinations:

(1) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of patient; or

(2) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (ii) age at confirmed diagnosis of epilepsy; or

(3) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (iii) atrophy of hippocampus or medial temporal sclerosis; or

(4) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (iv) the presence of a brain tumor; or

(5) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of patient and (ii) age at confirmed diagnosis of epilepsy; or

(6) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of patient and (iii) atrophy of hippocampus or medial temporal sclerosis; or

(7) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of a patient and (iv) the presence of a brain tumor; or

(8) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (ii) age at confirmed diagnosis of epilepsy and (iii) atrophy of hippocampus or medial temporal sclerosis; or

(9) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (ii) age at confirmed diagnosis of epilepsy and (iv) the presence of a brain tumor; or

(10) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (iii) atrophy of hippocampus or medial temporal sclerosis and (iv) the presence of a brain tumor; or

(11) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of a patient, (ii) age at confirmed diagnosis of epilepsy and (iii) atrophy of hippocampus or medial temporal sclerosis; or

(12) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of a patient, (ii) age at confirmed diagnosis of epilepsy and (iv) the presence of a brain tumor; or

(13) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of a patient, (iii) atrophy of hippocampus or medial temporal sclerosis and (iv) the presence of a brain tumor; or

(14) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (ii) age at confirmed diagnosis of epilepsy, (iii) atrophy of hippocampus or medial temporal sclerosis and (iv) the presence of a brain tumor; or

(15) SNP rs2740574; SNP rs1799735; SNP rs1695; SNP rs6280; and information on the locus of the human glutathione S-transferase theta-1 (GSTT1) gene, in particular on the deletion, partial deletion and/or non-functionality of GSTT1 and (i) a focal seizure with secondary generalization of a patient, (ii) age at confirmed diagnosis of epilepsy, (iii) atrophy of hippocampus or medial temporal sclerosis and (iv) the presence of a brain tumor, which is a particularly preferred combination of markers.

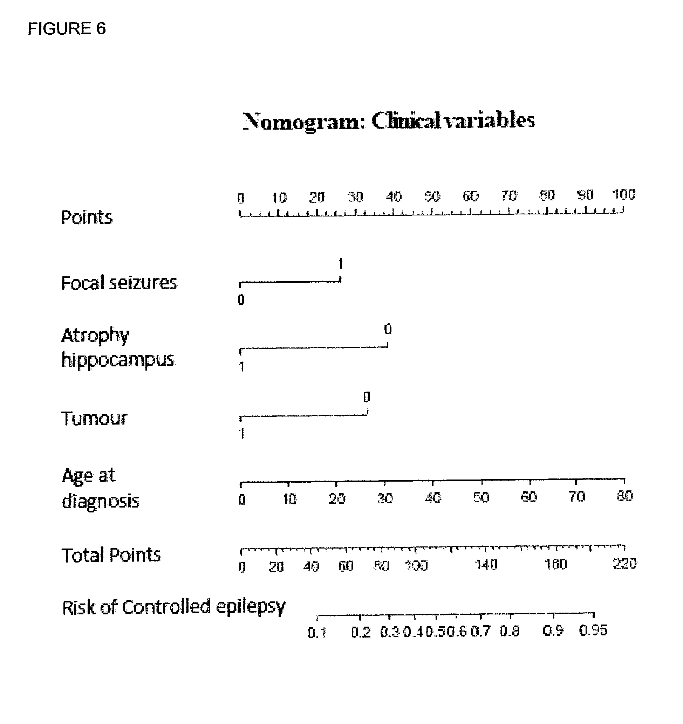

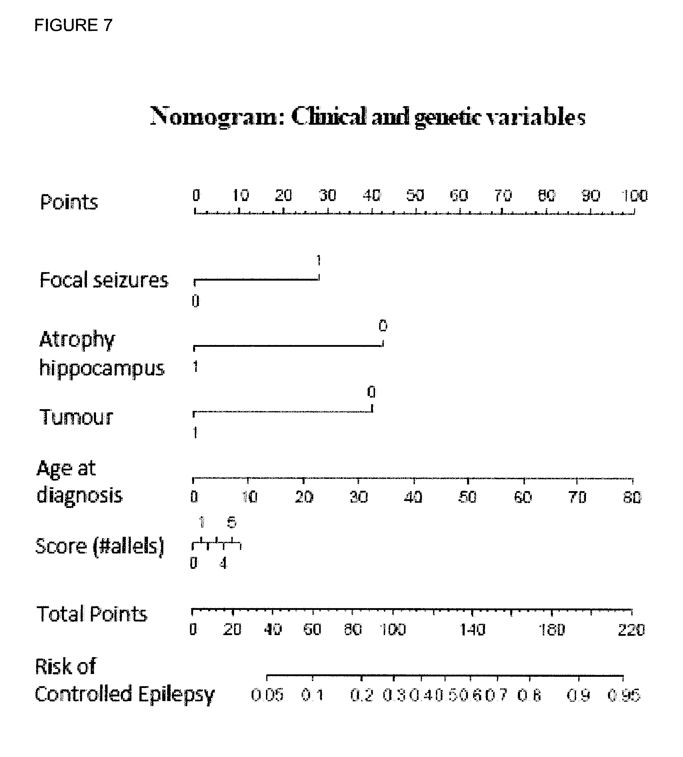

The term "focal seizure with secondary generalization" as used herein refers to a partial seizure or localized seizure which affects only a part of the brain at onset. A secondary generalization may subsequently occur through activation of the upper brain stem or by direct cortico-cortical spread through the commissures (corpus callosum, hippocampal commissure or anterior commissure). Clues to the fact that this seizure is not a primary generalized seizure may be found either in a premonitory "aura" (e.g. visceral or emotional symptoms leading up to the seizure) or reports from observers of unusual motor events (e.g. blinking, twitching, sniffing, picking at the clothes or lip smacking) immediately prior to losing consciousness. These symptoms may further be the result of electrical activity in the focal area prior to generalization. Focal seizures with secondary generalization may accordingly be determined by using suitable methods such as electroencephalography (EEG). The presence or detection of a focal seizure with secondary generalization is generally believed to contribute to a protection against drug-resistant epilepsy.
Further details may be derived from nomograms as depicted in Fig. 6 and 7 of the description.

The term "age at confirmed diagnosis of epilepsy" as used herein refers to the patient's age when first being diagnosed with epilepsy by a competent professional, e.g. a physician or neurologist. The diagnosis may be performed according to a suitable reference table or questionnaire to be classified as confirmed diagnosis. Further details may be derived from suitable literature sources such as Benbadis et al., 2001, Intracranial EEG and localization studies, in: Wyllie E. "The treatment of epilepsy: principles and practice", 3rd edition, Philadelphia, Lippincott Williams & Wilkins. A lower age at confirmed diagnosis of epilepsy, e.g. in the range of about 0 to 45 years is generally believed to lead to a lesser extent of protection against drug-resistant epilepsy, whereas a higher age at confirmed diagnosis of epilepsy, e.g. in the range of about 46 to 80 years or more is generally believed to lead to a higher extent of protection against drug-resistant epilepsy. Further details may be derived from nomograms as depicted in Fig. 6 and 7 of the description.

The term "atrophy of hippocampus or medial temporal sclerosis" as used herein refers to the patient's brain damage impacting both memory and spacial navigation. It is often associated with memory-loss conditions and may be caused by trauma, oxygen deprivation and encephalitis. Atrophy of hippocampus or medial temporal sclerosis may accordingly be determined by using suitable methods such as MRI based analyses, e.g. voxel-based morphometry. Further details may be derived from suitable literature sources such as Yasuda et al., 2010, Expert Rev Neurother., 10(6), 975-984. The absence of an atrophy of hippocampus or medial temporal sclerosis is generally believed to contribute to a protection against drug-resistant epilepsy. Further details may be derived from nomograms as depicted in Fig. 6 and 7 of the description.

The term "presence of a brain tumor" as used herein relates to the detection of any form of brain tumor or brain cancer. The tumor may a malignant or benign tumor. Brain tumors accordingly include all tumors inside the cranium or in the central spinal canal. Brain tumors may be caused by an abnormal and uncontrolled cell division in the brain or in lymphatic tissue, in blood vessels, in the cranial nerves, in the meninges, skull, pituitary gland, or pineal gland. Within the brain itself, the involved cells may be neurons or glial cells such as astrocytes, oligodendrocytes, and ependymal cells. Examples of typical brain tumors include gliomas, meningiomas, pituitary adenomas, and nerve sheath tumors. Brain tumors may be detected and/or diagnosed according to any suitable methodology, e.g. based on the use of CT- ,MRI- and PET-scanning or imaging methods. The absence of a brain tumor is generally believed to contribute to a protection against drug-resistant epilepsy. Further details may be derived from nomograms as depicted in Fig. 6 and 7 of the description.

In a preferred embodiment of the invention the probability for a given subject or individual, i, of being protected against drug-resistant epilepsy given both clinical covariates *age, seiz, atrophy* and *tumor* and the *genetic score (score)* may be computed on the basis of the following formula (I):

$$\mathrm{prob}(Y|\mathrm{age}_i, \mathrm{seiz}_i, \mathrm{atrophy}_i, \mathrm{tumor}_i, \mathrm{score}_i) =$$
$$= \frac{1}{\exp^{-(1.45+0.04\times\mathrm{age}_i+0.91\times\mathrm{seiz}_i-1.41\times\mathrm{atrophy}_i-1.33\times\mathrm{tumor}_i+0.06\times\mathrm{score}_i)}+1}$$

where:

| | |
|---|---|
| Y: | protection against drug-resistant epilepsy, |
| age: | age at con_rmed diagnosis of epilepsy, |
| seiz: | having focal seizure with secondary generalization, |
| atrophy: | having atrophy of hippocampus or medial temporal sclerosis, |
| tumor: | having the presence of a brain tumor, |
| score: | sum of variant alleles of SNPs: rs2740574, rs1799735, rs1695, rs6280; and the locus of the human glutathione S-transferase theta-1 (GSTT1) |

In further specific embodiments, deviations from one, more or all of the values mentioned in the formula may be applicable. For example, the value 1.45 may vary by about ± 15%, e.g. by about ± 1%, 2%, 3%, 4%, 5%, 6%, 7%, 8%, 9%, 10%, 11 %, 12%, 13%, 14%, or 15%; the value 0.04 may vary by about ± 10%, e.g. by about ± 1%, 2%, 3%, 4%, 5%, 6%, 7%, 8%, 9%, 10%, the value 0.91 may vary by about ± 15%, e.g. by about ± 1%, 2%, 3%, 4%, 5%, 6%, 7%, 8%, 9%, 10%, 11 %, 12%, 13%, 14%, or 15%; the value 1.41 may vary by about ± 15%, e.g. by about ± 1%, 2%, 3%, 4%, 5%, 6%, 7%, 8%, 9%, 10%, 11%, 12%, 13%, 14%, or 15%, the value 1.33 may vary by about about ± 15%, e.g. by about ± 1%, 2%, 3%, 4%, 5%, 6%, 7%, 8%, 9%, 10%, 11%, 12%, 13%, 14%, or 15% or the value 0.06 may vary by about ± 10%, e.g. by about ± 1%, 2%, 3%, 4%, 5%, 6%, 7%, 8%,9%,10%.

In another specific embodiment of the present invention, the group of biomarkers indicating a protection against drug-resistant epilepsy as mentioned herein above may be present in a subject or a group of subjects, which has been or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. In further specific embodiments, the subject as mentioned above may have an age of more than 30 years, more than 35 years, more than 40 years, more than 45 years, more than 50 years, more than 55 years, more than 60 years, more than 65 years, more than 70 years, more than 75 years, or more than 80 years at a confirmed diagnosis of epilepsy.

In a further aspect the present invention relates to a method for detecting protection against drug-resistant epilepsy in a subject comprising the steps of: (a) isolating a nucleic acid from a subject's sample; and (b) determining the status of a biomarker as defined herein above; wherein the presence of indicator nucleotides as defined herein above, and the presence of a deleted, partially deleted and/or non-functional GSTT1 gene as defined herein above is indicative for a protection against drug-resistant epilepsy.

The terms "isolating" as used herein refer to separating a nucleic acid molecule from at least one other undesired component or impurity found in a composition. The term "isolating" includes "purifying" and "clarifying." No particular level of isolation of a nucleic acid molecule is required, however in some embodiments, at least 50%, 70%, 80%, 90%, 95% or 99% (w/w) of an impurity is separated from the nucleic acid molecule. The separation may be carried out with respect to impurities such as proteins, lipids, further nucleic acids, and other forms of cellular debris, viral debris, contaminating bacterial debris, media components, and the like.

The term "detecting protection against drug-resistant epilepsy" as used herein means that a protection against drug-resistant epilepsy as defined herein above may be determined in a human being. In particular, a subject or patient may be considered to be protected against drug-resistant epilepsy if an indicator nucleotide in SNP rs2740574; rs1799735; rs1695; and rs6280 has been found on one chromosome or in one haplotype and if the GSTT1 gene is found deleted, partially deleted and/or non-functional as defined herein above in one chromosome or in one haplotype. In a further embodiment, a subject or patient (being a diploid organism comprising two copies of each autosome) may be considered to be protected against drug-resistant epilepsy if an indicator nucleotide in SNP rs2740574; rs1799735; rs1695; and rs6280 has been found on one chromosome or in one haplotype and if an indicator nucleotide in rs2740574; rs1799735; rs1695;or in rs6280 has additionally been found in the second (corresponding or equivalent) chromosome or in two haplotypes and if the GSTT1 gene is found deleted, partially deleted and/or non-functional as defined herein above in one chromosome or in one haplotype, or if the GSTT1 gene is additionally found deleted, partially deleted and/or non-functional as defined herein above in the second (corresponding or equivalent) chromosome or in both haplotypes. In a further specific embodiment, a subject or patient may be considered to be protected against drug-resistant epilepsy if an indicator nucleotide in SNP rs2740574; rs1799735; rs1695; and rs6280 has been found on two (corresponding or equivalent) chromosomes or in two haplotype and if the GSTT1 gene is found deleted, partially deleted and/or non-functional as defined herein above in the two (corresponding or equivalent) chromosomes or in both haplotypes.

The term "determining the status of a biomarker" as used herein refers to any suitable method or technique of detecting the identity of the nucleotide at position 21 of SEQ ID NO: 1, 3 or 4 or of detecting the identity of the nucleotides at positions 22, 23, and 24 of SEQ ID NO: 2, or of detecting the functionality or activity of the GSTT1 gene, or of detecting a deletion or partial deletion of the GSTT1 gene. The detection is preferably a detection of the identity of the nucleotide at position 21 of SEQ ID NO: 1, 3 and 4 and of the nucleotides at positions 22, 23, and 24 of SEQ ID NO: 2 as well as a determination of the functionality or activity of the GSTT1 gene, or the detection of deletion or partial deletion of the GSTT1 gene. The determination method may preferably be a sequencing technique or a technique based on complementary nucleic acid binding. The context of the indicated positions, as well as the strand may differ, e.g. from patient to patient, or from sample to sample etc.

A "subject's sample" as used herein may be any sample derived from any suitable part or portion of a subject's body. The sample may, in one embodiment, be derived from pure tissues or organs or cell types, or derived from very specific locations, e.g. comprising only one type of tissue, cell, or organ. In further embodiments, the sample may be derived from mixtures of tissues, organs, cells, or from fragments thereof. Samples may, for example, be obtained from organs or tissues such as the gastrointestinal tract, the vagina, the stomach, the heart, the tongue, the pancreas, the liver, the lungs, the kidneys, the skin, the spleen, the ovary, a muscle, a joint, the brain, the prostate, the lymphatic system or an organ or tissue known to the person skilled in the art. In further embodiments of the invention the sample may be derived from body fluids, e.g. from blood, saliva, ejaculate, liquor etc. In further embodiments of the present invention the above mentioned sample is be a mixture of tissues, organs, cells and/or fragments thereof, or a tissue or organ specific sample, such as a tissue biopsy from vaginal tissue, tongue, pancreas, liver, spleen, ovary, muscle, joint tissue, neural or brain tissue, gastrointestinal tissue, , or a body fluid, blood, , saliva, or liquor. Preferred is the use of blood or saliva samples.

The sample used in the context of the present invention should preferably be collected in a clinically acceptable manner, more preferably in a way that nucleic acids and/or proteins are preserved.

In further embodiments of the present invention the sample may contain one or more than one cell, e.g. a group of histologically or morphologically identical or similar cells, or a mixture of histologically or morphologically different cells. Preferred is the use of histologically identical or similar cells, e.g. stemming from one confined region of the body.

In a specific embodiment a sample may be obtained from the same subject at different points in time, obtained from different organs or tissues of the same subject, or form different organs or tissues of the same subject at different points in time. For example, a sample of specific tissue and of one or more samples of a neighbouring region of the same tissue or organ may be taken.

In a preferred embodiment of the present invention, the subject to be tested may have been, or may be, or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. A method for detecting protection against drug-resistant epilepsy may accordingly be carried out in a subject or group of subjects, which (i) is afflicted by a focal seizure with secondary generalization, and/or (ii) is not afflicted by atrophy of hippocampus or medial temporal sclerosis and/or (iii) is not afflicted by a brain tumor. A subject may, thus, for example be afflicted by a focal seizure with secondary generalization, and not be afflicted by atrophy of hippocampus; a subject may alternatively be afflicted by a focal seizure with secondary generalization, and not be afflicted by a brain tumor; a subject may alternatively be afflicted by a focal seizure with secondary generalization, and not be afflicted by atrophy of hippocampus and not be afflicted by a brain tumor; a subject may alternatively not be afflicted by atrophy of hippocampus and not be afflicted by a brain tumor; a subject may alternatively be afflicted by a focal seizure with secondary generalization, and not be afflicted by atrophy of hippocampus and not be afflicted by a brain tumor. In further specific embodiments, the subject as mentioned above may have an age of more than 30 years, more than 35 years, more than 40 years, more than 45 years, more than 50 years, more than 55 years, more than 60 years, more than 65 years, more than 70 years, more than 75 years, or more than 80 years at a confirmed diagnosis of epilepsy. Although the method is clearly applicable at every age of a subject, a more advanced age of a subject indicates a higher probability of being protected against drug-resistant epilepsy. Further details and combinations of proportions of a subject or group of subjects which may be tested according to a method of the invention may, for example, be derived from the nomogram of Fig. 6. In specific embodiments the present inventon relates to all combinations of values as derivable from Fig. 6.

In a further preferred embodiment of the present invention the mentioned determination of the nucleotide sequence may be carried out through allele-specific oligonucleotide (ASO)-dot blot analysis, primer extension assays, iPLEX SNP genotyping, Dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, SNP microarray based analysis, restriction enzyme fragment length polymorphism (RFLP) analysis, targeted resequencing analysis and/or whole genome sequencing analysis.

The term "allele-specific oligonucleotide (ASO)-dot blot analysis" as used herein refers to the employment of a short piece of synthetic DNA, which is typically complementary to the sequence of a polymorphic target site, in dot blot assay or, alternatively, in a Southern blot assay. The allele specific oligonucleotide may be an oligonucleotide of 15-21 bases in length, e.g. spanning 15 to 21 nucleotides around position 21 of SEQ ID NO: 1 to 4 or the complementary sequence thereof, or spanning 15 to 21 nucleotides of the ORF of the GSTT1 gene, or of border sequences of said GSTT1 ORF. The ASO may vary in length, may be chose from either of the two nucleic acid strands and the specificity of its binding in the dot blot or Southern blot may be modified by suitable buffer, hybridizing or washing conditions, which would be known to the person skilled in the art. The ASO may be labeled with any suitable label, e.g. with a radioactive, enzymatic, or fluorescent tag.

The term "primer extension assay" as used herein refers to a two step process that first involves the hybridization of a probe to the bases immediately upstream of the polymorphic nucleotide(s) followed by a mini-sequencing reaction, in which DNA polymerase extends the hybridized primer by adding a base that is complementary to the polymorphic nucleotide. The incorporated base may subsequently be detected and can thus determine the SNP allele. The primer extension method may be used in the context of further assay formats, e.g. detection techniques including MALDI-TOF Mass spectrometry and ELISA-like methods.

The term "iPLEX SNP genotyping" as used herein refers to a method involving the use of a MassARRAY mass spectrometer and extension probes designed in such a way that 40 different SNP assays can be amplified and analyzed in a PCR cocktail. The extension reaction preferably uses ddNTPs and the detection of the SNP allele is typically dependent on the actual mass of the extension product. Further details would be known to the person skilled in the art.

The term "Dynamic allele-specific hybridization (DASH) genotyping" refers to a technique taking advantage of the differences in the melting temperature in DNA that results from the instability of mismatched base pairs. Preferably, in a first step, a genomic segment may be amplified and attached to a bead through a PCR reaction, e.g. with a biotinylated primer. In the second step, the amplified product may be attached to a streptavidin column and washed, e.g. preferably with NaOH, to remove the unbiotinylated strand. Subsequently, an allele-specific oligonucleotide may be added in the presence of a molecule that fluoresces when bound to double-stranded DNA. The intensity may subsequently be measured as temperature is increased until the Tm can be determined. A SNP will typically result in a lower than expected Tm. In a preferred embodiment the process may be carried out on an automated basis.

The term "use of molecular beacons" as used herein refers to a detection of a polymorphism by using specifically designed single-stranded oligonucleotide probe comprising complementary regions at each end and a probe sequence located in between. This design typically allows the probe to take on a hairpin structure or stem-loop structure. The probe may preferably comprise at one end a fluorophore and at the other end a fluorescence quencher and, in certain embodiments, be engineered such that only the probe sequence is complementary to the genomic DNA that will be used in the assay. If the probe sequence of the molecular beacon encounters its target DNA, it will anneal, hybridize and fluoresce. If, however, the probe sequence encounters a modified target sequence with a non-complementary nucleotide, the molecular beacon may preferably stay in its natural hairpin state and no fluorescence may be observed, thereby allowing a distinction between a wildtype situation and modification thereof. In preferred embodiments of the invention more than one such molecular beacon may be used, e.g. one for a wildtype sequence, and a further one for a sequence including an indicator nucleotide. Thereby the presence of at least the wildtype nucleotide and the indicator nucleotide may be determined.

The term "tetra primer ARMS PCR" as used herein refers to the method involving two pairs of primers to amplify two alleles in one PCR reaction. The primers are typically designed such that the two primer pairs overlap at a polymorphic site or SNP location but each match perfectly to only one of the possible SNPs. As a result, if a given allele is present in the PCR reaction, the primer may pair specific to that allele and may subsequently produce a product but not to the alternative allele with a different SNP. The two primer pairs may, in further embodiments, also be designed such that their PCR products are of a significantly different length allowing, for example, for easily distinguishable bands by gel electrophoresis.

The term "flap endonuclease invader assay" as used herein refers to the use of a flap endonuclease cleavase which is combined with two specific oligonucleotide probes that, together with the target DNA, can form a tripartite structure recognized by the cleavase The first probe, i.e. the invader oligonucleotide is preferably complementary to the 3' end of the target DNA. The last base of the invader oligonucleotide may be a non-matching base that overlaps the SNP nucleotide in the target DNA. The second probe may be an allele-specific probe which is complementary to the 5' end of the target DNA, but may also extend past the 3' side of the SNP nucleotide. The allele-specific probe may contain a base complementary to the SNP nucleotide. If the target DNA contains the desired allele, the invader and allele-specific probes may bind to the target DNA forming the tripartite structure. The cleavase may subsequently cleave and release the 3' end of the allele-specific probe. In preferred embodiments, the invader assay may be coupled with a fluorescence resonance energy transfer (FRET) system to detect the cleavage event.

The term "quantitative real-time PCR assay" as used herein refers to an assay preferably performed with a Taqman enzyme or a similar activity, concurrently with a PCR reaction, wherein the results can be read in real-time as the PCR reaction proceeds. The assay typically requires forward and reverse PCR primers that will amplify a region that includes the polymorphic site, preferably primers binding in the 5' or 3' region with respect to position 501 of any one of SEQ ID NO: 1 to 14. Allele discrimination may, in specific embodiments also be achieved using FRET combined with one or two allele-specific probes that hybridize to the SNP polymorphic site. The probes may have a fluorophore linked to their 5' end and a quencher molecule linked to their 3' end. While the probe is intact, the quencher may remain in close proximity to the fluorophore, eliminating the fluorophore's signal. During the PCR amplification step, if the allele-specific probe is perfectly complementary to the SNP allele, it may bind to the target DNA strand and then get degraded by 5'-nuclease activity of the Taq polymerase as it extends the DNA from the PCR primers. If the allele-specific probe is not perfectly complementary, it may have a lower melting temperature and not bind as efficiently.

The term "oligonucleotide ligase assay" as used herein refers to an enzymatic reaction catalyzed by DNA ligase which may be used to interrogate a SNP by hybridizing two probes directly over the SNP or polymorphic site, whereby ligation can occur if the probes are identical to the target DNA. Typically, two probes are designed: an allele-specific probe which hybridizes to the target DNA so that its 3' base is situated directly over the SNP nucleotide and a second probe that hybridizes the template upstream (downstream in the complementary strand) of the SNP or polymorphic site providing a 5' end for the ligation reaction. Ligated or unligated products may subsequently be detected by gel electrophoresis, MALDI-TOF mass spectrometry or by capillary electrophoresis.

The term "PCR-single strand conformation polymorphism (SSCP) analysis" as used herein refers a method, capable of identifying sequence variations in a single strand of DNA, typically between 150 and 250 nucleotides in length. The method is based on the fact that single-stranded DNA (ssDNA) folds into a tertiary structure. The conformation is typically sequence dependent and most single base pair mutations will alter the shape of the structure. When applied to a gel, the tertiary shape may determine the mobility of the ssDNA, which provides a mechanism to differentiate between polymorphic alleles. In preferred embodiments the method first involves a PCR amplification of a target DNA. The double-stranded PCR products may be denatured using heat and formaldehyde to produce ssDNA. The ssDNA may be applied to a non-denaturing electrophoresis gel and allowed to fold into a tertiary structure.

The term "SNP microarray based analysis" as used herein refers to the employment of high-density oligonucleotide SNP arrays comprising, for example, 100, 1000, or more than 10000 probes arrayed on a chip, allowing for many SNPs to be interrogated simultaneously. Target DNA may be hybridized to the array, preferably by using several redundant probes to interrogate each SNP. In specific embodiments, probes may be designed to have the SNP site in several different locations as well as containing mismatches to the SNP allele. In further embodiments, the differential amount of hybridization of the target DNA to each of these redundant probes may also allow the determination of homozygous and heterozygous alleles, e.g. to detect whether one or two of the alleles of SEQ ID NO: 1 to 4 show the indicator sequence or not, or whether parts or fragments of the GSTT1 gene or ORF are present in a cell. An examples of a SNP microarrays, which is also envisaged by the present invention is the Affymetrix Human SNP 5.0 GeneChip.

The term "restriction enzyme fragment length polymorphism (RFLP) analysis" used herein refers to the performance of a digestion on a genomic sample and the determination of fragment lengths, e.g. through a gel assay, allowing to ascertain whether or not the enzymes cut at expected restriction sites. The RFLP analysis may preferably be carried out on the basis of PCR amplified fragments around position 21 of any one of SEQ ID NO: 1 to 4, or PCR amplified fragments covering parts or portions of the ORF, or the entire ORF of the GSTT1 gene. The corresponding primer binding sites and the length may be determined in dependence on the availability of suitable restriction sites.

The term "targeted resequencing analysis" as used herein refers to capturing and sequencing of the regions of interest, wherein the capturing may be done in solution or on an array and the sequencing can be performed by any first, second or third generation sequencing platform. Further details and features would be known to the person skilled in the art.

The term "whole genome sequencing analysis" as used herein refers to the determination of the sequence of the entire genome of a subject, preferably of both alleles of a polymorphic site based on high-throughput sequencing technology, e.g. Next-generation sequencing technologies such as pyrosequencing and the like. The techniques may, in certain embodiments, also be used for the sequencing of portions of the genome, e.g. small regions of interest. This technique may, in preferred embodiments, also be used for the determination of haplotypes in chromosomic regions or on specific chromosomes.

In a further embodiment of the present invention the method for detecting protection against drug-resistant epilepsy may comprise the determination of the haplotype for two copies of the chromosome comprising the SNPs rs2740574; rs1799735; rs1695; and rs6280 and the GSTT1 gene in a subject. The term "haplotype" as used herein refers to a 5' to 3' sequence of nucleotides found at a polymorphic site as defined herein above in a locus on a single chromosome from a single subject. A haplotype according to the present invention does not comprise a combination of two or more SNPs as defined herein on one chromosome, and thus merely refers to a single stranded sequence reflecting a polymorphic site on one autosome as described according to the present invention. For example, the present invention encompasses haplotypes as mentioned in Table 6. Particularly preferred are haplotypes showing a p-value of $\leq 0.15$, as derivable from Table 6.

In a further embodiment, the method for detecting protection against drug-resistant epilepsy in a subject as defined herein above may comprise as additional step the determination of a focal seizure with secondary generalization in a subject, the determination of atrophy of hippocampus or medial temporal sclerosis in a subject, the determination of the presence of a brain tumor in a subject and/or the determination of the subject's age at confirmed diagnosis of epilepsy. The determination of these clinical markers may be carried out as described herein above. The detection of a focal seizure with secondary generalization may indicate an increase in the likelihood for protection against drug-resistant epilepsy. Furthermore, formula (I) and variants thereof as mentioned above may be used as basis for the determination of likelihood of being protected against drug-resistant epilepsy. Further details may also be derived from nomograms as depicted in Fig. 6 and 7 of the description.

Furthermore, a lower age at confirmed diagnosis of epilepsy, e.g. in the range of about 0 to 45 years may indicate an decrease in the likelihood for protection against drug-resistant epilepsy, whereas a higher age at confirmed diagnosis of epilepsy, e.g. in the range of about 46 to 80 years or more may indicate an increase in the likelihood for protection against drug-resistant epilepsy. Furthermore, formula (I) and variants thereof as mentioned above may be used as basis for the determination of likelihood of being protected against drug-resistant epilepsy. Further details may also be derived from nomograms as depicted in Fig. 6 and 7 of the description.

The detection of the absence of an atrophy of hippocampus or medial temporal sclerosis may indicate an increase in the likelihood for protection against drug-resistant epilepsy. Furthermore, formula (I) and variants thereof as mentioned above may be used as basis for the determination of likelihood of being protected against drug-resistant epilepsy. Further details may also be derived from nomograms as depicted in Fig. 6 and 7 of the description.

The absence of a brain tumor may indicate an increase in the likelihood for protection against drug-resistant epilepsy. Furthermore, formula (I) and variants thereof as mentioned above may be used as basis for the determination of likelihood of being protected against drug-resistant epilepsy Further details may also be derived from nomograms as depicted in Fig. 6 and 7 of the description.

In a further aspect the present invention relates to a method for monitoring epilepsy therapy in a subject comprising the step of determining the status of a biomarker as defined herein above before and during an epilepsy treatment of said subject.

The term "monitoring" as used herein relates to the accompaniment of an epilepsy therapy during a certain period of time, typically during 6 months, 1 year, 2 years, 3 years, 5 years, 10 years, or any other period of time. The term "accompaniment" means that states of disease as defined herein and, in particular, changes of these states of disease may be detected by comparing the status of a biomarker of the present invention in a sample in any type of a periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The term "before an epilepsy treatment" as used herein means that a patient or patient's sample may analyzed after an initial diagnosis of epilepsy and the commencement of an epilepsy treatment. The corresponding period of time may be 1 hour, 12 hours, 1 day, 2 days, 3 days, 4 days, 5 days, 6 days, 1 week, 2 weeks, 3 weeks, 4 weeks, 2 months, 3 months, 4 months, 5 months, 6 months, or more or any period of time in between these values. The term "during an epilepsy treatment" as used refers to the determination during the entire or during a part of a therapeutic treatment. For instance, the determination may be carried out between administration steps, or at a defined interval of 1 hour, 12 hours, 1 day, 2 days, 3 days, 4 days, 5 days, 6 days, 1 week, 2 weeks, 3 weeks, 4 weeks, 2 months, 3 months, 4 months, 5 months, 6 months, or more or any period of time in between these values. In a specific embodiment, the monitoring may alos be carried out after the epilepsy treatment, e.g. 1 hour, 12 hours, 1 day, 2 days, 3 days, 4 days, 5 days, 6 days, 1 week, 2 weeks, 3 weeks, 4 weeks, 2 months, 3 months, 4 months, 5 months, 6 months, or more or any period of time in between these values after the termination of the epilepsy treatment. Changes of the status of biomarkers as defined herein above may provide the medical professional with indications regarding the protection against drug-resistant epilepsy and may lead to a modification of administration, the inclusion of other or more or less medicaments, a combination with further medicaments or any other suitable decision to increase the health of a patient.

In a further specific embodiment said subject to be monitored has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. In further embodiments, the subject as mentioned above may have an age of more than 30 years, more than 35 years, more than 40 years, more than 45 years, more than 50 years, more than 55 years, more than 60 years, more than 65 years, more than 70 years, more than 75 years, or more than 80 years at a confirmed diagnosis of epilepsy. Although the method is clearly applicable at every age of a subject, a more advanced age of a subject indicates a higher probability of being protected against drug-resistant epilepsy. In a further, alternative embodiment, the method further comprises the determination of a focal seizure with secondary generalization in a subject, the determination of atrophy of hippocampus or medial temporal sclerosis in a subject, and/or the determination of the presence of a brain tumor in a subject as defined herein above. It is preferred to combine the determination of a focal seizure with secondary generalization in a subject, the determination of atrophy of hippocampus or medial temporal sclerosis in a subject, and the determination of the presence of a brain tumor in a subject with the determination of the biomarkers as described above.

In a preferred embodiment, the treatment may be carried out with any suitable medicament, preferably with any suitable anticonvulsant. Preferred examples of suitable anticonvulsants include lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel and zonisamide. Also envisaged is a combination of any two or more of the mentioned anticonvulsants.

In a specific embodiment of the present invention, the likelihood of a subject to be protected against drug-resistant epilepsy may be determined according to the method for detecting protection against drug-resistant epilepsy on the basis of the status of a biomarker as defined herein above. The determination may be carried out in a subject which has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. A calculation of the likelihood may preferably be performed according to formula (I) as depicted herein above. Probability values obtainable with said formula (I) may range between 0 and 1, with 0.5 and more providing an indication that the subject is protected against drug-resistant epilepsy, while a value of below 0.5 may be interpreted as meaning that a subject is not protected against drug-resistant epilepsy. The obtainable likelihood values for the protection against drug-resistant therapy may be used for a definition and/or control of therapy regimes and options. For example, in case of a high likelihood for the protection against drug-resistant therapy, i.e. above 0.5, a therapy with any suitable drug known to the skilled person may be carried out. In case of a low likelihood for the protection against drug-resistant therapy, i.e. above 0.5, a therapy with any suitable drug as known to the skilled person may be carried out.

In a further aspect the present invention relates to a method of identifying an individual as eligible for an aggressive epilepsy therapy comprising:

(a) determining in a sample obtained from an individual the status of a biomarker as defined herein above; and
(b) identifying the individual as eligible to receive an aggressive epilepsy therapy where the individual's sample is classified as showing the presence of indicator nucleotides, and the occurrence of a deleted, partially deleted and/or non-functional GSTT1 gene as defined herein above. The term "aggressive epilepsy" as used herein refers to a therapeutic form, which relies on an increased dosis in comparison to a standard or comparable therapeutic form, e.g. a dosis increased by 10%, 15%, 20%, 25% or any other suitable value, e.g. any value in between the mentioned values. An aggressive epilepsy therapy may further be a combination therapy of two or more independent anticonvulsants. The method may preferably be performed on a subject or group of subjects, which has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. In further embodiments, the subject as mentioned above may have an age of more than 30 years, more than 35 years, more than 40 years, more than 45 years, more than 50 years, more than 55 years, more than 60 years, more than 65 years, more than 70 years, more than 75 years, or more than 80 years at a confirmed diagnosis of epilepsy.

# EP 2 792 754 A1

In a further specific embodiment of the present invention the method of identifying an individual as eligible for an aggressive epilepsy therapy as described above comprises the following steps:

(a) determining in a sample obtained from an individual the status of a biomarker as defined herein above;
(b) determining a focal seizure with secondary generalization in a subject, determining atrophy of hippocampus or medial temporal sclerosis in a subject, determining the presence of a brain tumor in a subject and/or determining the subject's age at confirmed diagnosis of epilepsy; and
(c) identifying the individual as eligible to receive an aggressive epilepsy therapy where the individual's sample is classified as showing the presence of indicator nucleotides, and the occurrence of a deleted, partially deleted and/or non-functional GSTT1 gene as defined herein above and where the individual additionally shows focal seizure with secondary generalization, where the individual additionally shows no atrophy of hippocampus or medial temporal sclerosis, where the individual additionally shows no brain tumor and/or wherein the subject's age at confirmed diagnosis of epilepsy is above 45 years. The determination of elegebility for an aggressive epilepsy therapy may be based on the calculation of the likelihood which can be performed according to formula (I) as depicted herein above. Probability values obtainable with said formula (I) may range between 0 and 1, with 0.5 and more providing an indication that the subject is eligible for an aggressive epilepsy therapy, while a value of below 0.5 may be interpreted as meaning that a subject is not protected against drug-resistant epilepsy and thus not eligible for an aggressive epilepsy therapy.

In another aspect the present invention relates to a composition for detecting protection against drug-resistant epilepsy in a subject, comprising a nucleic acid affinity ligand for a biomarker as defined in claim 1 or 2.

The term "nucleic acid affinity ligand" as used herein refers to a nucleic acid molecule being able to bind to a polymorphic site as defined above. Preferably, the affinity ligand is able to bind a nucleic acid molecule comprising the sequence of any one of SEQ ID NO: 1 to 4, or fragments thereof, which comprise the polymorphic site as defined herein above, wherein said sequence of SEQ ID NO: 1 to 4 comprises the respective indicator nucleotide as described herein above. In further embodiments of the present invention the nucleic acid affinity ligand may also be able to specifically bind to a nucleic acid molcule, e.g. a DNA molecule comprising, essentially consisiting of or consisting of a sequence being at least 90%, 91%, 92%, 93%, 94%, 95%, 96%, 97%, 98% or 99% or 99.5% or 99.6%, 99.7%, 99.8%, or 99.9% identical to the sequence of SEQ ID NO: 1, 2, 3 or 4, or fragments thereof, which comprise the polymorphic site as defined herein above, wherein said sequence of SEQ ID NO: 1 to 4 comprises the respective indicator nucleotide(s) as described herein above, or to any fragments of said sequences.

In further embodiments of the present invention a nucleic acid affinity ligand according to the present invention may also be able to specifically bind to a nucleic acid molecule comprising the sequences of SEQ ID NO: 1, 2, 3 or 4, with the exception that the sequences do not comprise the indicator nucleotide sequence as defined herein above, i.e. to corresponding wildtype sequences which do not comprise the respective indicator nucleotide as described herein above. In further embodiments of the present invention the nucleic acid affinity ligand may also be able to specifically bind to a nucleic acid molecule comprising, essentially consisting of, or consisting of a sequence being at least 90%, 91%, 92%, 93%, 94%, 95%, 96%, 97%, 98% or 99% or 99.5% or 99.6%, 99.7%, 99.8%, or 99.9% identical to the sequence of SEQ ID NO: 1, 2, 3 or 4 with the exception that the sequences do not comprise the indicator nucleotide sequence as defined herein above, i.e. to corresponding wildtype sequences which do not comprise the respective indicator nucleotide as described herein above, or fragments thereof. In even further embodiments, the present invention relates to nucleic acid affinity ligands binding a nucleic acid molecule comprising a sequence complementary to any one of the sequence of SEQ ID NO: 1 to 4, or fragments thereof, which comprises the polymorphic site as defined herein above, which may comprise or may not comprise the indicator nucleotide.

In a further embodiment, the nucleic acid affinity ligand may be able to bind to a nucleic acid molecule comprising the GSTT1 gene, or a part of the GSTT1 gene, e.g. a GSTT1 ORF fragment or the GSTT1 ORF, or to nucleic acid molecule comprising boundary regions of the GSTT1 gene, or to genomic copies of the GSTT1 gene, or the GSTT1 transcripts or mRNA molecules. In further specific embodiments said nucleic acid affinity ligand as defined herein above may be a short nucleic acid molecule, e.g. a RNA, DNA, PNA, CNA, HNA, LNA or ANA molecule or any other suitable nucleic acid format known to the person skilled in the art, being capable of specifically binding to the sequence of SNPs (e.g. indicator or wildtype sequence) of SEQ ID NO: 1 to 4, or to GSTT1 related sequences, or boundary sequences within the genomic region around GSTT1.

In further specific embodiments said nucleic acid affinity ligand may comprise any suitable functional component known to the skilled person, e.g. a tag, a fluorescent label, a radioactive label, a dye, a binding or recognition site for a protein or antibody or peptide, a further stretch of DNA useful for PCR approaches, a stretch of DNA useful as recognition site for restriction enzymes etc. The nucleic acid affinity ligand may further be provided in the form of a catalytic RNA specifically binding to and cleaving a nucleic acid molecule comprising a sequence comprising the SNP according to the present invention, e.g. either the indicator nucleotide or the wildtype nucleotide or a different nucleotide at the polymorphic site.

In further embodiments the present invention envisages pairs of nucleic acid affinity ligand of which one is able to specifically bind to a nucleic acid molecule comprising the wildtype sequence of any one of SEQ ID NO: 1 to 4, and the other is specifically able to bind to a nucleic acid molecule comprising the sequence of any one of SEQ ID NO: 1 to 4 including the indicator nucleotide as defined herein above. Such pairs may further be distinguished by, for example, differential labels, different dyes, or any other different functionality as described herein. Furthermore, more than two pairs, e.g. for each of SEQ ID NO: 1 to 4 a pair or a sub-group thereof may be provided, which are also distinguished by differential dyes, labels, or other functionalities. In yet another embodiment, the present invention envisages pairs of nucleic acid affinity ligands of which one is able to specifically bind to the wildtype sequence of GSTT1, e.g. within the open reading frame, and the other is specifically able to bind to a nucleic acid molecule comprising boundary sequences of GSTT1, or sequences bridging sectors or fragments of GSTT1 which may only appear upon deletion or partial deletion of GSTT1 or regulatory elements thereof.

In further specific embodiments the present invention also relates to non-nucleic acid affinity ligands specific for one or more polymorphic sites as defined herein above. Such affinity ligands may be peptides, aptamer like elements, antibodies, DNA motif recognizing proteins, e.g. restriction enzymes, or combinations of these with nucleic acids. In further specific embodiments, the composition may also comprise peptide binding or protein binding molecule, e.g. an antibody or a peptide affinity molecule, which is able to bind a GSTT1 protein or a fragment thereof.

The composition according to the present invention may additionally comprise further ingredients necessary or useful for the detection of protection against drug-resistant epilepsy, such as buffers, dNTPs, a polymerase, ions like bivalent cations or monovalent cations, hybridization solutions, etc.

In yet another preferred embodiment of the present invention the affinity ligand as mentioned herein above may be an oligonucleotide specific for one or more polymorphic sites as defined herein above, or a probe specific for one or more polymorphic sites as defined herein above. The term "oligonucleotide specific for one or more polymorphic sites" as used herein refers to a nucleic acid molecule, preferably a DNA molecule of a length of about 12 to 38 nucleotides, preferably of about 15 to 30 nucleotides. The oligonucleotide may have, for example, a length of 16, 17, 18, 19, 20, 21, 22, 23, 24, 25, 26, 27, 28, 29, or 30 nucleotides. These molecules may preferably be complementary to at least 2, 3, 4, 5, 6, 7, 8, 9, or 10 nucleotides on or around the indicator nucleotide(s) of SEQ ID NO: 1, 2, 3 or 4, comprising the complementary sequence of said indicator nucleotide(s) as defined herein above in connection with SEQ ID NO: 1 to 4. In further embodiments, the molecules may preferably be complementary to at least 2, 3, 4, 5, 6, 7, 8, 9, or 10 nucleotides on or around the polymorphic site as defined herein above in connection with SEQ ID NO: 1 to 4, however comprising the wildtype sequence. In further embodiments, the oligonucleotide may be specific for the GSTT1 gene, a fragment thereof, the GSTT1 ORF, or a product obtainable upon deletion or partial deletion of the GSTT1 gene.

In preferred embodiments of the present invention said oligonucleotide as defined herein above may have a sequence complementary to a sequence including the indicator nucleotide(s) of the SNPs of the present invention as defined herein above. In further embodiments the oligonucleotide may also have a complementary sequences towards the counter strand of said sequence including the indicator nucleotide of the SNPs of the present invention as defined herein above.

In further embodiments the present invention also relates to oligonucleotide molecules specifically binding in the vicinity of the polymorphic site as indicated herein above in the context of SEQ ID NO: 1 to 4, or of GSTT1. These oligonucleotides may be designed in the form of a pair of primers allowing the amplification of stretch of DNA, e.g. of a length of 50 bp, 75 bp, 100 bp, 150 bp, 200 bp, 250 bp, 300 bp, 400 bp, 500 bp, 750 bp, 1000 bp, or more around and including the polymorphic site of the SNPs of the present invention. Suitable sequence information may be derived from the sequence of SEQ ID NO: 1 to 4, the herein above indicated genomic sequence localization, which allows the skilled person to obtain the necessary context DNA sequence from data repositories, e.g. the human genome of build 37.5, or 3' and 5' extensions of the sequences of SEQ ID NO: 1 to 4.

The term "probe specific for one or more polymorphic sites as defined herein above" as used herein refers piece of DNA, which is capable of specifically binding to a polymorphic site according to the present invention. The probe may, for example, be designed such that it only binds to a sequence comprising the indicator nucleotide, or the wildtyp sequence, or a complementary strand thereof. In other embodiments the probe may be capable of binding to a polymorphic site according to the present invention, i.e. be able to bind to the wildtype sequence, the indicator nucleotide comprising sequence or any other variant at that position as defined herein above. The specificity of the probe may further be adjusted, for example in hybridization experiments, by the changing the concentration of salts, modifying the temperature of the reaction, adding further suitable compounds to the reaction etc. The probe may also be designed such that it binds outside of the polymorphic site, e.g. within the sequence of SEQ ID NO: 1 to 4, or a complementary sequence thereof, or a sequence of the GSTT1 gene.

The probe according to the present invention may, in further embodiments, comprise, essentially consist of, or consist of a nucleic acid molecule being at least 90%, 91%, 92%, 93%, 94%, 95%, 96%, 97%, 98% or 99% or 99.5% or 99.6%, 99.7%, 99.8%, or 99.9% identical to the sequence of SEQ ID NO: 1 to 4, or to fragments thereof, which comprise the polymorphic site as defined herein above, wherein said sequence of SEQ ID NO: 1 to 4 comprises the respective indicator nucleotide as described herein above, or to any fragments of said sequences, or to the corresponding wildtype sequences as defined herein above, or to the complementary sequences of these sequences, or to nucleic acid molecules comprising the GSTT1 sequences or fragments thereof.

A probe according to the present invention may have any suitable length, e.g. a length of 15, 20, 30, 40, 50, 100, 150, 200, 300, 500, 1000 or more than 1000 nucleotides. The probe may further be suitable modified, e.g. by the addition of labels, e.g. fluorescent labels, dyes, radioactive labels etc.

In further embodiments, the probe may also be functionally adjusted to a detection method as described herein above.

In yet another aspect the present invention relates to a kit for detecting protection against drug-resistant epilepsy in a subject, comprising an oligonucleotide specific for one or more polymorphic sites as defined herein above, or a probe specific for one or more polymorphic sites as defined herein above. In a particularly preferred embodiment said oligonucleotide has a sequence complementary to an indicator nucleotide as defined herein above, or complementary to a GSTT1 gene fragment indicative for a deletion or partial delection. In further embodiments the kit as defined herein above may comprise accessory ingredients such as PCR buffers, ions like bivalent cations or monovalent cations, hybridization solutions etc. It is particularly preferred that the kit comprises an enzyme for primer elongation, nucleotides and/or lablelling agents. An enzyme for primer elongation may, for example, be a polymerase such as Taq polymerase, Pfu polymerase etc. Nucleotides may preferably be dNTPs, or derivatives thereof. A labeling agent may be, for example, an agent leading to the labeling with a radioactive label, an enzymatic label, a fluorescent label, a chemiluminescent or a bioluminescent label. The term "enzymatic label" relates to labels, which comprise enzymatic activities. A typical, preferred example is the horseradish peroxidase enzyme (HRP). This enzyme complex subsequently may catalyze the conversion of a suitable substrate, e.g. a chemiluminescent substrate into a sensitized reagent which ultimately lead to the emission of light or production of a color reaction. The term "radioactive label" relates to labels emitting radioactive radiation, preferably composed of radioactive isotopes. The term "radioactive isotope" in the context of the label relates to any such factor known to the person skilled in the art. More preferably, the term relates to $^3$H, $^{14}$C, $^{32}$P, $^{33}$P, $^{35}$S or $^{125}$I. The term "chemiluminescent label" relates to a label, which is capable of emitting light (luminescence) with a limited emission of heat as the result of a chemical reaction. Preferably, the term relates to luminol, cyalume, oxalyl chloride, TMAE (tetrakis (dimethylamino) ethylene), pyragallol, lucigenin, acridinumester or dioxetane. The term "bioluminescent label" relates to a label, which is capable of emitting light due to a biochemical reaction. Typically, the term refers to the production of light due to the reaction of a luciferin and a luciferase. In such a reaction scheme, the luciferase catalyzes the oxidation of luciferin resulting in light and an inactive oxyluciferin. The term "fluorescent label" relates to chemically reactive derivatives of a fluorophores. Typically common reactive groups include amine reactive isothiocyanate derivatives such as FITC and TRITC (derivatives of fluorescein and rhodamine), amine reactive succinimidyl esters such as NHS-fluorescein, and sulfhydryl reactive maleimide activated fluors such as fluorescein-5-maleimide. Reaction of any of these reactive dyes with another molecule results in a stable covalent bond formed between a fluorophore and a labelled molecule. Following a fluorescent labeling reaction, it is often necessary to remove any nonreacted fluorophore from the labeled target molecule.

In further embodiments the kit may also comprise accessory ingredients like secondary affinity ligands, e.g. secondary antibodies, detection dyes, or other suitable compound or liquids necessary for the performance of a nucleic acid detection. Such ingredients as well as further details would be known to the person skilled in the art and may vary depending on the detection method carried out. Additionally, the kit may comprise an instruction leaflet and/or may provide information as to the relevance of the obtained results.

In yet another aspect the present invention relates to a microarray for the analysis of protection against drug-resistant epilepsy in a subject, comprising at least one probe selective for an indicator nucleotide or the corresponding wildtype nucleotide, and the GSTT1 gene as defined herein above. In a standard setup such a microarray comprises immobilized probes to detect a nucleic acid comprising a polymorphic site as defined herein above. The probes on the array may, for example, be complementary to one or more parts of the sequence of SEQ ID NO: 1 to 4, or to the GSST1 gene, and/or to corresponding wildtype sequences. Typically, cDNAs, PCR products, and oligonucleotides may be used as probes. The microarray may comprise probes of one or more SEQ ID NO: 1 to 4 and GSTT1, or corresponsing wildtype sequences, or sequences representing deleted or partially deleted GSTT1, or all of these biomarkers or any combination of said markers. Furthermore, any type of fragment or sub-portion of any of the markers sequences may be combined with any further fragment or sub-portion of any of said sequences SEQ ID NO: 1 to 4, or GSTT1, or corresponding wildtype sequences.

There is virtually no limitation on the number of probes which are spotted on a DNA array. Also, a marker can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays.

In a further aspect the present invention relates to the use of a group of biomarkers as defined herein above for detecting protection against drug-resistant epilepsy in a subject. Further envisaged is the use of the SNPs rs2740574; rs1799735; rs1695; and rs6280 and the GSTT1 locus in combination with a clinical marker as defined herein above for detecting protection against drug-resistant epilepsy in a subject. In a particular embodiment, it is envisaged to use the SNPs rs2740574; rs1799735; rs1695; and rs6280 and the GSTT1 locus in combination with the detection of a focal seizure with secondary generalization, or the determination of age at confirmed diagnosis of epilepsy, or atrophy of hippocampus or medial temporal sclerosis, or the determination of the presence of a brain tumor for detecting protection against drug-resistant epilepsy in a subject.

Also envisaged is the use of a group of biomarkers as defined herein above for screening a population of subjects for the presence of drug-resistant epilepsy. A "population of subjects" may, for instance, be a natural population such as Caucasians, or Africans etc. The population may further be comprised of individuals living in a certain area, or having any other feature in common. In a particular embodiment, it is envisaged to use the SNPs rs2740574; rs1799735; rs1695; and rs6280 and the GSTT1 locus in combination with the detection of a focal seizure with secondary generalization, or the determination of age at confirmed diagnosis of epilepsy, or atrophy of hippocampus or medial temporal sclerosis, and/or the determination of the presence of a brain tumor for screening a population of subjects for the presence of drug-resistant epilepsy.

The subject or population of subjects as mentioned above may have been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor. The subject may thus belong to a patient group or patient collective, which is afflicted by a focal seizure with secondary generalization, and/or which is not afflicted by atrophy of hippocampus or medial temporal sclerosis and/or which is not afflicted by a brain tumor. Furthermore, said subject may have an age of more than 30 years, more than 40 years, more than 45 years, more than 50 years, more than 55 years or more than 60 years at a confirmed diagnosis of epilepsy.

In a further embodiment the present invention relates to lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel or zonisamide, or combinations thereof, for use in the treatment of epilepsy in a subject showing at the polymorphic site of SNP rs2740574 a single polymorphic change represented by position 21 of SEQ ID NO: 1, where in one or two alleles the wildtype nucleotide A is replaced by indicator nucleotide G; showing at the polymorphic site of SNP rs1799735 a single polymorphic change represented by position 21 of SEQ ID NO: 2, where in one or two alleles indicator nucleotides AGG are inserted; showing at the polymorphic site of SNP rs1695 a single polymorphic change represented by position 21 of SEQ ID NO: 3, where in one or two alleles wildtype nucleotide A is replaced by indicator nucleotide G; showing at the polymorphic site of SNP rs6280 a single polymorphic change represented by position 21 of SEQ ID NO: 4, where in one or two alleles wildtype nucleotide C is replaced by indicator nucleotide T; and showing in one or two alleles a complete deletion, partial deletion and/or non-functionality of the human glutathione S-transferase theta-1 (GSTT1) gene. In a further optional embodiment, the subject additionally shows a focal seizure with secondary generalization, no atrophy of hippocampus or medial temporal sclerosis, no brain tumor and/or the subject's age at confirmed diagnosis of epilepsy is above 45 years.

In yet another embodiment, the present invention relates to lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel or zonisamide, or combinations thereof, for use in the treatment of epilepsy in a subject, comprising (i) assaying a sample from said subject, preferably a sample as defined herein above, (ii) determining, e.g. on or with the sample, whether the subject shows at the polymorphic site of SNP rs2740574 a single polymorphic change, e.g. represented by position 21 of SEQ ID NO: 1, where in one or two alleles the wildtype nucleotide A is replaced by indicator nucleotide G; at the polymorphic site of SNP rs1799735 a single polymorphic change, e.g. represented by position 21 of SEQ ID NO: 2, where in one or two alleles indicator nucleotides AGG are inserted; at the polymorphic site of SNP rs1695 a single polymorphic change, e.g. represented by position 21 of SEQ ID NO: 3, where in one or two alleles wildtype nucleotide A is replaced by indicator nucleotide G; at the polymorphic site of SNP rs6280 a single polymorphic change, e.g. represented by position 21 of SEQ ID NO: 4, where in one or two alleles wildtype nucleotide C is replaced by indicator nucleotide T; and whether in one or two alleles a complete deletion, partial deletion and/or non-functionality of the human glutathione S-transferase theta-1 (GSTT1) gene is given, and optionally determining in addition whether the subject shows a focal seizure with secondary generalization, shows atrophy of hippocampus or medial temporal sclerosis, has or previously had a brain tumor and/or determining the subject's age at confirmed diagnosis of epilepsy; (iii) administering an effective amount of lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel or zonisamide, or combinations thereof to the subject if the subject shows at the polymorphic site of SNP rs2740574 a single polymorphic change represented by position 21 of SEQ ID NO: 1, where in one or two alleles the wildtype nucleotide A is replaced by indicator nucleotide G; at the polymorphic site of SNP rs1799735 a single polymorphic change represented by position 21 of SEQ ID NO: 2, where in one or two alleles indicator nucleotides AGG are inserted; at the polymorphic site of SNP rs1695 a single polymorphic change represented by position 21 of SEQ ID NO: 3, where in one or two alleles wildtype nucleotide A is replaced by indicator nucleotide G; at the polymorphic site of SNP rs6280 a single polymorphic change represented by position 21 of SEQ ID NO: 4, where in one or two alleles wildtype nucleotide C is replaced by indicator nucleotide T; and shows in one or two alleles a complete deletion, partial deletion and/or non-functionality of the human glutathione S-transferase theta-1 (GSTT1) gene and wherein optionally the subject shows a focal seizure with secondary generalization, shows no atrophy of hippocampus or medial temporal sclerosis, has or had previously no brain tumor and/or wherein the subject's age at confirmed diagnosis of epilepsy is above 45 years.

The following examples and figures are provided for illustrative purposes. It is thus understood that the examples and figures are not to be construed as limiting. The skilled person in the art will clearly be able to envisage further modifications of the principles laid out herein.

EXAMPLES

**Example** 1

***Study design***

Cross-sectional, epidemiological, case-control, multicenter study to analyze predictive factors related to DICE in patients with epilepsy. An algorithm will be designed with multiple variables, including genetic, environmental, clinical and diagnostic factors, in a total of 564 patients.

The study population were patients with epilepsy, 18 years of age or older, males and females, who attended Epilepsy Units or Specialised Consultancies. Patients were included in the study consecutively, in the order in which they attended the center and when they met the inclusion criteria. The patients were divided into two groups:

G.1: DICE patients ($\geq$ 1 epileptic seizure per month, in the last 12 months after)

G.2: controlled patients (1 year without epileptic seizures for one year or for three times between crisis interval [the longest period], after the first or second AED in monotherapy)

The recruitment period was 10 months. The participating investigators collected the requested information in the case report form (CRF).

Once the informed consent was obtained from each patient, demographic, environmental and clinical data as well as diagnostic factors were obtained by reviewing the medical records of the patients. If data was not available in the medical records, the patients were asked directly during the visit.

The genetic determinants associated to DICE susceptibility and AED tolerability were identified using a DNA microarray (biochip) specifically created for this study, which included 61 different polymorphisms involved in the metabolism, transport and elimination of the main AEDs, as well as mutations that affect molecular routes possibly related to the etiopathogeny of epilepsy. The complete list of single-nucleotide polymorphisms included in the biochip can be found in Annex 1.

***Study population***

1. *Definition of the study population: selection criteria*

The study included patients diagnosed with epilepsy who met the selection criteria specified below.

2. *Inclusion criteria*

Inclusion criteria:

- Patients (male or female) aged 18 and older.
- Patients diagnosed with focal epilepsy, based on clinical study, EEG and brain NMR.

- Patients who suitably comply with their current antiepileptic treatments.
- Patients who given their written informed consent to participate in the study.

G.1: DICE Group

- Patients with an average of one or more epileptic seizures per month in the last 12 months.
- Patients in whom control of epileptic seizures has failed after trying two suitable antiepileptic drugs (AEDs) in mono- or combination therapy, excluding those drugs that were discontinued because of poor tolerability.

G.2: Controlled epilepsy group

- Patients who have not experienced an epileptic seizure in the last year or for three times between crisis interval [the longest period], after the first or second AED in monotherapy)
- Patients who have achieved epilepsy control with the first or second AED used, excluding those medications that were not tolerated

3. *Exclusion criteria*

- Patients with a history of chronic drug or alcohol abuse in the last 6 months.
- Patients whose medical or psychiatric condition, or history of progressive neurological disease, could, in the opinion of the investigator, put them at risk or compromise the subject's capacity to participate in the study
- Patients with a history of non-epileptic events in the three years prior to entering the study.
- Patients with a clinically significant systemic disease, such as cancer or kidney or liver failure.
- Patients with a low level of AED compliance.

**Example 2**

***Rationale for the sample***

The main focus of this study was the identification of factors associated with Difficult to Control Epilepsy (DICE). There is a consensus between studies that the percentage of patients diagnosed with epilepsy who present DICE is approximately 30%.[4] According to these data, the inclusion of 650 patients in the current study would enable the ratio of interest to be estimated with a 95% confidence interval and 4% precision.
The calculation of the sample size was carried out with the PASS1 programme, version 2020 (Hintze J. 2001. NCSS and PASS. Number Cruncher Statistical Systems. Kaysville, Utah. www.ncss.com).

***Sample size to determine the predictability of genetic polymorphisms are difficult to control epilepsy***

It was created a predictor that includes clinical and genetic variables with a predictive capability of 75% (AUC: 0.755) that presented a sensitivity and specificity of 68.3% and 68.8%, respectively. In other words, rate of false positives (RFP) is approximately 30% and the rate of true positives (RTP) is 70%. In the validation sample we hope to have these values at least. However, to apply our predictor with a reasonable TFP, we will assume that to be applied in practice we will require a RFP = 10%. With this information, it is required a sample size of 55 individuals with DICE and 320 with controlled epilepsy. If we keep the RFP equal to 10% and increased the RTP to 90%, we would need 50 individuals in each group (reference - The Statistical evaluation of medical tests for classification and prediction. MS Pepe, Oxford University Press, 2003).

***Variables***

1. *Primary and secondary endpoints*

In the different Epilepsy Units and Specialised Consultancies participating in the study, socio-demographic, environmental and clinical data and factors related to epilepsy were complied, and patients were asked for a saliva sample for the genetic determination of factors associated to the susceptibility of DICE and tolerability to AEDs.

## 2. *Socio-demographic and environmental data*

- Date of birth (month and year)
- Gender: (man/woman)
- Lifestyle habits:

  ■ Smoking habit (smoker, ex-smoker, non-smoker) (in the case of smoker or ex-smoker, specify number of cigarettes/day) Alcohol habit (yes/no), in case of "yes", specify drink units (DUs)
  *1 DU= 1 beer = 1 glass of wine =1/2 glass of liquor= ½ whisky*
  ■ Caffeine consumption (yes/no), in case of "yes", specify coffee, cola drinks or energy drinks (mg)
  *mg caffeine: 1 cup of coffee (125 ml)=120 mg; 1 can of cola (330 ml)=35 mg; 1 can of energy drink (250 ml)= 80 mg*
  ■ Use of cellular phone (yes/no), in case of "yes", specify average number of min/day and monthly bill (euros)

- Living situation (urban area/rural area)
- Occupational status (works/doesn't work), in case of "works" (night shift/no night shift)

## 3. *Clinical particulars*

- Weight (kg)
- Height (cm)
- Perinatal history
- Psychomotor development (normal/abnormal, specify anomaly)
- Concomitant diseases and treatments (depression, autoimmune disorders)
- Other concomitant diseases
- Other concomitant treatments (route of administration, dose, start date)

## 4. *Factors related to epilepsy*

- Epilepsy and seizures

  ■ History of the epilepsy (date of first seizure, type of seizure, date of first diagnosis), number of seizures before the diagnosis
  ■ Type of seizure (partial seizure [specify type] or unclassified seizures)
  ■ History of febrile seizures (yes/no, in case of "yes": date of first seizure, date of last seizure, total number of seizures, type of seizures)
  ■ Other risk factors (cranial trauma with loss of consciousness, confusion or other neurological deficit; cranial trauma associated with lesion in cerebral imaging; meningitis or encephalitis, brain surgery; cerebral stroke, cerebral haemorrhaging)
  ■ Etiology of the epilepsy (symptomatic, cryptogenic, undetermined and idiopathic [gene identified, yes/no])
  ■ Types of epilepsy (temporal, frontal, occipital, parietal, undetermined location)
  ■ Medical assistance due to epilepsy (number of visits to neurologist, emergency ward and/or hospital admissions)
  ■ Status epilepticus (yes/no, in case of "yes": convulsive or nonconvulsive and number)
  ■ Evolution (only for DICE patients). Remission periods ≥6 months (yes/no, number, waking seizures/night)
  ■ Cognitive status (neuropsychological assessment [yes/no, specify findings])
  ■ Family history of epilepsy, febrile seizures or isolated seizures (siblings, parents, grandparents, aunts/uncles, cousins)

- Antiepileptic treatment (AEDs)

  ■ Prior treatment (date of first AED, AEDs received since diagnosis of epilepsy, reason for change of treatment [lack of efficacy or tolerability], maximum dose [mg])

■ Current treatment (active substance, maximum dose [mg], start date [month/year])

- Electroencephalogram (EEG)
- Brain nuclear magnetic resonance (NMR)

5. *Genetic factors related to susceptibility to DICE and tolerability of AEDs*

In case to have a saliva sample the SNPs described om Annex 1 were evaluated by using *ad hoc* DNA microarray.

6. *Diagnostic criteria for the study disease*

Patient selection for this study was based on the consecutive selection of patients with epilepsy who attended Epilepsy Units and Specialised Consultancies throughout Spain.

## Example 3

### *Statistical analysis*

The analyses were carried out using the available data, without employing substitution techniques for absent values, and the number of missing data in each analysis was described. Nevertheless, to generate multivariate models, the processing of absent values was assessed in terms of the number of missing data in the set of predictive variables.
Descriptive analyses was carried out separately for all the evaluation criteria, using tables of absolute and relative frequency in the case of discrete quantitative and qualitative variables, and using statistical values of mean, standard deviation, extreme values and quartiles in the case of continuous quantitative variables.

### *Definition of the dependent variable*

The objective of this study was to identity the patients who were resistant to currently available antiepileptic treatments (AEDs), also known as patients with Difficult to Control Epilepsy (DICE). With this aim two patient groups were selected: those who respond and those who do not respond to currently available anti-epileptic treatments (AEDs).

### *Independent variables*

Two types of variables were considered:

1) relevant clinical and environmental information recorded during the course of the study,
2) determination of single nucleotide polymorphisms (SNP)'

A selection of variables before starting the generation of models was realized, in order to avoid redundancy and "overfitting" (which generates noise or "random order") of the model. The selection was achieved by univariate analysis, using SPSS, version 15.0 (SPSS Inc. Headquarters, Chicago, IL, USA).

### *Selection of clinical and environmental variables*

The selection of clinical and environmental variables was carried out to identify the most important and predictive characteristics to be analysed in the model. This approach was based on the marginal association between each variable and the clinical phenotype (response/no response to current epilepsy treatments).
Depending on the type of variables a different univariate test was used, according to:

1) Continous or categorical variables.
2) Data normality

All the possibilities of a categorical dependent variable were represented in the following table 1:

**Table 1:** Possibilities for categorical dependent variables.

| Dependent variable | Independent variable | | |
|---|---|---|---|
| | Categorical variable c =2 | Multi-category c>2 | Quantitative variable |
| **Categorical variable c=2** | Chi-square test | Chi-square test | Student's t-test (normal distribution) Mann-Whitney U test (non-normal distribution) |
| **Multi-category c>2** | Chi-square test | Chi-square test | ANOVA (normal distribution) Kruskal-Wallis (non-normal distribution) |

**Example** 4

***SNP Selection***

Several criteria were used to identify the most important and predictive SNPs to be included in the model, using the Helix Tree® software. The following steps were taken:

-Step 1:

The associated allele frequencies were calculated in order to eliminate monomorphic SNPs and those with a low allele frequency (<5%).

-Step 2:

For those SNPs selected in step one, a linkage disequilibrium and deviations from Hardy-Weinberg Equilibrium (HWE) were calculated. When a complete linkage disequilibrium among SNPs was observed (r2 >0.8) only the one with the lowest p-value for allelic association between SNPs and phenotypes was included in the regression model. Additionally, those SNPs which significantly deviate from Hardy-Weinberg equilibrium were eliminated from the regression model, a genotyping error or a sampling error could account for that.

-Step 3:

In the final step an association between the phenotype and those SNPs selected in previous step was performed by univariate analysis. Clinical and SNPs variables were typically filtered on the basis of a p-value cut-off from the univariate analysis. However a correction for multiple testing False Discovery Rate (FDR) was applied. The corrected number represents the expected proportion false discoveries for a given p-value cut-off. After the FDR correction, the cut-off 0.05 was used.

After completing this phase a list of clinical variables and SNP was available to start the generation of the model.

***Generation of model/models***

Once the SNP and clinical variables were selected, a binary logistic regression model was constructed, using SPSS version 15.0 (SPSS Inc. Headquarters, Chicago, IL, USA) and R packages Design[20].

Binary logistic regression is a form of regression which is used when the dependent is a dichotomous variable (responder/no responder) and the independents are of any type, clinical (categorical or quantitative variables) and SNPs (categorical variables). Logistic regression applies maximum likelihood estimation after transforming the dependent into a logit variable (the natural log of the odds of the dependent occurring or not). In this way, logistic regression estimates the odds of a certain event occurring.

The selection of variables in the logistic regression model was stepwise. Two types of models, backward and forward mode, were generated in parallel. Both approaches provided similar results, proving that the results were correct.

***Evaluation of the goodness-off-fit of the model***

The goodness of fit of the obtained models was evaluated using Hosmer- Lemeshow statistic and the accuracy was assessed by calculating the bootstrapped area under the Receiver Operating Characteristic (ROC) curve (AUC) with 95% confidence intervals. Bootstrapping on the model's AUC was conducted 500 times using 100% random sampling by replacement, using R package Boot. To measure the impact of the SNPs and variables included in the logistic regression model of the analyzed phenotypes, the sensitivity, specificity, and positive likelihood ratio (LR+ = sensitivity / (1-specificity)) was computed by means of ROC curves.

AUC - ROC curves of the different obtained models were compared by using the method by Delong et al[21] implemented in the software Analyse-it (Analyse-it Software Ltd. Leeds, UK).

### *Set of analysis*

All the analyses were carried out from a single sample of assessable patients which included all those patients who met the inclusion criteria and for whom 100% of the data on principle variables was available, i.e. clinical, environmental and genetic factors related to Difficult to Control Epilepsy (DICE).

### *Ethical requirements*

The investigator conducted the study according to the principles of the Declaration of Helsinki. Copies of the Declaration of Helsinki and subsequent amendments were provided upon express request or could be obtained from the World Medical Association website: http://www.wma.net/en/30publications/10policies/b3/index.html.

The study was conducted according to the protocol and standard operating procedures (SOPs) to ensure compliance with Good Clinical Practice (GCP) guidelines as described in the Tripartite Harmonised ICH Guidelines for Good Clinical Practice of 1996.

According to international standards regarding epidemiological studies, as defined in International Guidelines for Ethical Review of Epidemiological Studies (Council for the International Organizations of Medical Sciences-CIOMS-Geneva, 1991) and Spanish Epidemiology Society (SEE) recommendations on the review of ethical aspects of epidemiological studies, this type of study must, except for in certain specific cases, be reviewed by an independent committee. Therefore, this study was submitted for evaluation to an Independent Ethics Committee and the Spanish Medicines Agency (AEM) was notified.

### *Risk/benefit analysis for subjects*

The patients included in the study were not subject to experimental procedures. Their participation did not involve any additional risk.

### *Patient information sheet and consent form*

The investigator explained to each patient (or legally authorised representative) the nature of the study, its purposes, procedures, estimated duration, the potential risks and benefits related to participation in the study, as well as any other inconvenience that it could entail. All participants were advised that their participation in the study was voluntary and that they could withdraw from the study at any time without this affecting their subsequent medical treatment or their relationship with the doctor who treated them in any way.

The patient had enough time to read and understand the explanations on the patient information sheet before signing and dating the informed consent form and received a copy of the signed documents. No patient were included in the study without first granting his/her informed consent.

### *Independent Ethics Committee (IEC)*

The protocol, the proposed informed consent form and other information for the patients were reviewed by an Independent Ethics Committee (IEC). All changes to the protocol, other than administrative changes, required an amendment to the protocol which was approved by this committee.

Changes in the conduct of the study which were not contemplated in amendments were classified as violations. Violations could also disqualify a center for continuing to participate in the study.

### *Investigator recruitment*

The sponsor selected the investigators according to their capacity to satisfactorily complete the study in accordance with the protocol. A sample was selected comprising the most representative group for Spain.

*Patients recruitment*

To avoid selection bias, each participating doctor consecutively included a total of 10 patients (5 patients in G.1 and 5 patients in G.2) who consecutively attended their office. After the informed consent was obtained, all the necessary data were recorded in the CRF. The necessary data were obtained in a single visit.

**Visit schedule**

The study was carried out in a single visit. The parameters evaluated are detailed in Table 2.

Table 2: Study visit, duties and procedures at the center.

| VARIABLES | Single Visit | |
|---|---|---|
| **Inclusion/exclusion criteria** | X | |
| **Information and informed consent** | X | |
| **Information and informed consent for genetic studies** | X | 5 |
| **Socio-demographic and environmental data:** | X | |
| Age, gender, lifestyle habits, living situation and occupational status | | |
| **Clinical data:** | X | |
| Weight, height, perinatal history, psychomotor development, concomitant diseases and treatments | | 10 |
| **Factors related to epilepsy:** | X | |
| Epilepsy and seizure, antiepileptic treatment, EEG, MRI | | |
| **Genetic tolerability and susceptibility** (saliva sample for SNP determination) | X | 15 |

Example 4

**Study population**

*Final analysable sample*

A total of 567 patients were recruited, 564 (99.5%) of whom could be assessed as they fulfilled all the selection criteria and a sufficient saliva sample was available for DNA analysis. In total, 290 patients (51.4%) with difficult-to-control epilepsy (DICE) and 274 patients (48.6%) with controlled epilepsy (CE) were included in the study (see Table 3).

**Table 3:** Inclusion and exclusion criteria and patients included in the analysis, by patient group.

| Variable | | DICE (N = 292) | CE (N =275) | Total (N = 567) |
|---|---|---|---|---|
| Does the patient meet all the inclusion criteria? | No | 1 (0.34 %) | 0 (0.00 %) | 1 (0.18 %) |
| | Yes | 291 (99.66 %) | 275 (100.00 %) | 566 (99.82 %) |
| Does the patient meet any exclusion criteria? | No | 1 (0.34 %) | 0 (0.00 %) | 1 (0.18 %) |
| | Yes | 291 (99.66 %) | 275 (100.00 %) | 566 (99.82 %) |
| Insufficient DNA sample | No | 0 (0.00 %) | 1 (0.36 %) | 1 (0.18 %) |
| | Yes | 292 (100.00 %) | 274 (99.64 %) | 566 (99.82 %) |
| Assessable patients | No | 2 (0.68 %) | 1 (0.36 %) | 3 (0.53 %) |
| | Yes | 290 (99.32 %) | 274 (99.64 %) | 564 (99.47 %) |

**Clinical profile of the patient**

*1. Biodemographic profile and anthropometric data*

The assessable population was characterised by a mean age (SD) of 42.9 (14.4) years. Female patients were in the majority (52.3%). The total population presented a mean weight of 72.2 (14.8) kg and a mean height of 166.9 (9.2) cm. The variables described above did not show any significant differences between the two groups.

*2. Perinatal history and psychomotor development*

Information was collected about psychomotor development and perinatal history. This consisted of birth history, problems during pregnancy, medication during pregnancy and perinatal problems. The DICE group showed a tendency, although not significant, towards a higher number of cases of abnormal delivery compared to the CE group (17.5% vs 11.8%, p = 0.057). Problems during pregnancy and the medication used in pregnancy were similar in both groups, 6.1% and 0.5% of the total population respectively. On the other hand, there were significant differences between the groups with regards to perinatal problems and psychomotor development, with significantly more cases found in the DICE group: 11.5% vs 6.6% (p = 0.044) and 13.0% vs 6.3% (p = 0.007), respectively. The perinatal problems taken into account were: APGAR score, prematurity, overdue birth, hypoglycaemia, neonatal seizures, periventricular haemorrhage, resuscitation and perinatal distress. These last two perinatal problems were shown to be more present in the DICE group compared to CE patients, with the tendency being non-significant for resuscitation (7.1% vs 3.7%, p = 0.077) and significant for perinatal distress (10.8% vs 5.1%, p = 0.014). The rest of the perinatal problems were shown to be similar between the two groups of patients, with a prevalence of less than 1.4% in each group of patients.

*3. Concomitant illnesses*

Both groups showed a similar proportion of patients with depression prior to the diagnosis of epilepsy: 4.9% of DICE patients and 5.9% of CE patients. At inclusion, the proportion of patients with depression was significantly higher in the DICE group (14.3% vs 8.1%, p = 0.021). It was observed that the increase in depression between the initial diagnosis and inclusion was significant for DICE patients (p < 0.001).

Likewise, it was found that a significantly higher proportion of EC patients received treatment for the cardiovascular system (39.8% vs 18.6%, p < 0.001). This is because cardiac disorders tend, although not significantly, to occur more often in EC patients (4.8% vs 2.1%, p = 0.076), while vascular disorders are significantly more common in EC patients (16.5% vs 6.6%, p < 0.001). Likewise, a significantly higher percentage of EC patients was observed to have treatments for the digestive tract and metabolism (19.0% vs 10.0%, p = 0.002), which is explained by a non-significant tendency towards a higher proportion of EC patients with metabolic and nutritional disorders (12.5% vs 7.9%, p = 0.073). There are no significant differences between the two groups of patients concerning disorders of the blood and the lymphatic system (2.3% of the total population). However, it was seen that the percentage of EC patients in treatment for diseases of the blood and haematopoietic organs was significantly higher than for DICE patients (10.9% vs 5.9%, p = 0.029).

For the other concomitant illnesses assessed, the two groups were shown to be similar, with no significant differences between them, with the following percentages of the total population: autoimmune diseases (7.1%), allergies (8.9%), eye disorders (3.0%), respiratory disorders (4.3%), gastrointestinal disorders (3.7%), hepatobiliary disorders (2.0%), renal and urinary disorders (3.4%), disorders of the reproductive organs and the breast (1.8%), disorders of the nervous system (8.0%), musculoskeletal disorders (6.8%), disorders of the skin and subcutaneous tissue (1.4%), neoplastic disorders (3.7%), endocrine disorders (5.2%) and psychiatric disorders (6.4%). It should be noted that psychiatric disorders are more common in the DICE group (8.3% vs 4.4%, p = 0.062), whereas a non-significant tendency is observed for endocrine disorders to be more common in EC patients (7.0% vs 3.4%, p = 0.058).

As for the majority of concomitant illnesses, the treatments for these illnesses do not show significant differences between the two groups of patients, with the following percentages of the total population for the treatments assessed: hormonal preparations (4.1%), sense organ treatments (0.7%), antineoplastic drugs and immunomodulators (0.5%), antiparasitic drugs and insect repellents (0.5%), antiallergic agents (0.2%) and treatments for the musculoskeletal system (3.5%), respiratory system (2.8%), genitourinary system and sex hormones (2.1%), nervous system (17.4%). Nevertheless, a non-significant tendency was observed for treatments of the nervous system to be more common in the DICE group (20.3% vs 14.2%, p = 0.056).

In general, on the basis of the results for concomitant illnesses and treatments, it was observed that the patients from the DICE group were prone to present illnesses related to the nervous system, while the patients from the CE group were prone to other types of illness, not related to the nervous system. Nevertheless, on evaluating the whole set of concomitant illnesses and treatments, the two groups of patients were similar.

**Main variable of analysis**

1. *Clinical factors*

Nine clinical factors were taken into account: history of epilepsy, history of febrile seizures and other risk factors, aetiology and type of epilepsy, medical care, status epilepiticus and cognitive status, family history, previous and current anti-epileptic treatment, electroencephalogram (EEG), and cerebral magnetic resonance imaging (MRI).

*2. History of epilepsy*

The history of epilepsy in the patient showed that there were significant differences in time since first seizure, type of first seizure and number of seizures prior to diagnosis. The DICE group showed a mean time since the first seizure that was significantly higher than the CE patients (25.1 (15.1) vs 16.1 (14.1) years, p < 0.001). For the first seizure, patients with CE showed a significantly higher proportion of simple partial seizures (14.0% vs 8.1%, p = 0.028) or of generalised seizures (48.7% vs 39.8%, p = 0.036), while patients with DICE showed a higher proportion of complex partial seizures (48.6% vs 34.7%, p = 0.001). The DICE group showed a significantly higher mean number of seizures prior to diagnosis (10.2 (24.3) vs 6.0 (12.7), p < 0.001). Once the diagnosis of epilepsy had been confirmed, the mean duration of the disease was significantly higher for the DICE group compared to the CE patients (23.3 (14.5) years vs 14.0 (12.7) years, p < 0.001).

In terms of the different types of seizure that a patient suffers during the illness - partial (simple, complex or focal with secondary generalisation) or generalised - significant differences are found between CE and DICE patients. In general, a significantly higher proportion of patients who had suffered generalised seizures was found in the CE group (26.8% vs 16.2%, p = 0.002). On the other hand, the proportion of patients who had ever suffered a partial seizure was significantly higher in the DICE group (99.0% vs 93.3%, p < 0.001). Taking the three types of partial seizure separately, the proportion of patients who had suffered a simple partial seizure was similar for both groups (37.0% of the total population), whereas focal partial seizure with secondary generalisation was significantly more common in patients with CE than DICE (65.7% vs 50.5%, p < 0.001). On the other hand, complex partial seizures occurred at some point in the illness in a significantly higher proportion of DICE patients (87.1% vs 58.2%, p < 0.001).

Considering patents who presented at least one partial seizure in the last 12 months, a significantly higher proportion was found of DICE patients who presented more than 40 events in this time, either simple partial seizures (46.7% vs 1.1%, p < 0.001), complex partial seizures (36.8% vs 3.8%, p < 0.001) or focal partial seizures with secondary generalisation (8.4% vs 0.0%, p < 0.001). Likewise, when analysing patients with 40 or less partial seizures in the last 12 months, it is also found that the DICE group shows a higher mean number of events, be it for simple partial seizures [13.0 (12.9) vs 0.2 (0.6) events, p < 0.001], complex partial seizures [17.8 (11.6) vs 0.8 (4.4) events, p < 0.001] or focal partial seizures with secondary generalisation [3.5 (6.5) vs 0.3 (1.6) events, p < 0.001]. Considering patients who presented at least one generalised seizure in the last 12 months, none presented more than 40 events in the last year. Nevertheless, taking into account patients with 40 or fewer generalised seizures, it was found that the DICE group showed a significantly higher mean number of events [2.5 (4.6) vs 0.4 (1.3) events, p < 0.001].

Evaluating the time of day (daytime, during sleep or both) when seizures occur, it is found that there is no difference between the two groups of patients for simple partial seizures, focal partial seizures with secondary generalisation and generalised seizures, given that the majority occur in the daytime for simple partial seizures (81.4% of the total population) and focal partial seizures with secondary generalisation (65% of the total population) and during sleep for generalised seizures (51.3% of the total population). On the contrary, for complex partial seizures, a significantly higher proportion was noted of patients with DICE having seizures at any time of day (daytime or during sleep) [16.6% vs 4.3%, p = 0.022].

Regarding the location in the brain where seizures occur, significant differences are only found for simple partial seizures, which are more frequently located in the temporal lobe for the DICE group (62.0% vs 37.7%, p = 0.008), whereas for CE patients they are located in a significantly higher proportion in the frontal lobe (43.4% vs 22.5%, p = 0.013). It was also observed that the confirmation and localisation of seizures by video-electroencephalogram was significantly higher in the DICE group than in the CE group (47.9% vs 9.9%, p < 0.001).

*3. History of febrile seizures and other risk factors*

The history of febrile seizures was collected, and significant differences were observed in their prevalence, with a higher proportion of patients with DICE having a history of febrile seizures compared to the CE group (12.5% vs 7.0%, p = 0.028). Nevertheless, no significant difference was found between the groups in terms of mean age at the first febrile seizure [1.9 (2.2) years in the total population], mean age at the last seizure [3.9 (3.4) years in the total population] and mean total number of febrile seizures [4.3 (6.8) events in the total population]. Considering the type of febrile seizure suffered, it was observed that patients with CE showed a tendency, although not significant, to present simple febrile seizures (76.5% vs 50.0%, p = 0.079).

Other risk factors for epilepsy that were considered: head injury (with loss of consciousness, confusion or other neurological disorder), head injury associated with lesion visible by cerebral imaging, meningitis or encephalitis, brain surgery, stroke and cerebral haemorrhage. Meningitis or encephalitis, stroke and cerebral haemorrhage were significantly different in the two groups of patients. Meningitis or encephalitis were suffered by a significantly higher proportion of patients with DICE (9.0% vs 3.4%, p = 0.007), whereas stroke and cerebral haemorrhage were suffered by a significantly higher proportion of patients with EC (9.2% vs 1.4%, p < 0.001 and 7.7% vs 2.9%, p = 0.012 respectively). In cases of stroke, 64% of the population suffered it in the left hemisphere, with 62.3% occurring in the temporal lobe; most of them were simple (83.3%), with no significant differences between groups. Likewise, there were no differences between the groups concerning location of cerebral haemorrhage (lobular location for 87% of the total population). The two groups were shown to be similar in the remaining risk factors, with the proportions of the total population being: head injury with loss of consciousness (12.8%), head injury associated with lesion (4.2%), brain surgery (11.5%).

### 4. *Aetiology and type of epilepsy*

The causes of epilepsy were recorded, and significant differences were found between DICE and CE patients. Symptomatic aetiology was found in a significantly higher proportion of patients with DICE (59.9% vs 47.4%), whereas the CE patients presented a significantly higher proportion of cryptogenic (43.0% vs 36.0%) and idiopathic aetiologies (5.2% vs 1.0%) [p = 0.003]. Among the various aetiological symptoms, it was observed that the DICE group showed a significantly higher proportion of cases in which the symptom was hippocampal sclerosis (38.4% vs 11.9%, p < 0.001), whereas the group with CE showed a significantly higher proportion of cases in which the symptom was vascular (24.6% vs 6.4%, p < 0.001) or post-traumatic (13.5% vs 6.4%, p = 0.038). The type of epilepsy was evaluated, and it was observed that temporal epilepsy was the most common in both groups of patients, with the proportion being significantly higher in the DICE than in the CE group (69.0% vs 52.2%, p < 0.001) [Table 136 of the statistical report].

### 5. *Medical care*

Information was collected on medical care due to epilepsy in the last 12 months, and it was found that the patients with DICE compared to EC were significantly more likely to have visited the neurologist [4.1 (3.2) vs 2.0 (2.8) consultations], and been admitted to hospital as an emergency (stay < 24 h) [0.9 (2.9) vs 0.1 (0.4) times] or for longer stays (> 24 h) [0.5 (2.7) vs 0.0 (0.2) times] (p < 0.001 in all cases).

### 6. *Status epilepticus and cognitive status*

Status epilepticus, the evolution of epileptic seizures and the cognitive status of patients were recorded. For status epilepticus, a significantly higher proportion was observed for patients with DICE (11.1% vs 3.4%, p = 0.001). Status epilepticus can be either convulsive or non-convulsive, with the mean number of both types being similar for both groups of patients [in the total population, 1.7 (1.8) for convulsive status, 2.2 (2.8) for non-convulsive status]. Considering the progress of the disease, it was found that 28.6% of patients with DICE presented periods of remission longer than 6 months.

Cognitive status was monitored by neuropsychological assessment, estimation in patients without neuropsychological assessment and intelligence quotient (IQ) in patients with learning difficulties. A higher proportion of the patients in the DICE group was observed to have learning difficulties (17.6% vs 5.8%) or to have had a neuropsychological assessment (12.3% vs 2.8%) compared with the CE group [p < 0.001 in both cases]. Both groups of patients were similar in IQ values for patients with learning difficulties, which were mild (IQ 50-99) for the majority of the population [79.5% of the total population].

### 7. *Family history*

The proportion of patients with family members (brothers and sisters, parents, aunts and uncles, grandparents and cousins) with epilepsy, febrile seizures or isolated seizures was evaluated. In general, no significant differences were found in any case. The percentages of the total population with various family members with epilepsy were: brothers and sisters (10.4%), grandparents (11.6%), aunts and uncles (8.2%) and cousins (12.1%). It should be noted that there is a tendency, although not significant, for a higher proportion of patients with CE to have parents with epilepsy (10.8% vs 3.9%, p = 0.065). There also tends to be a higher proportion of CE patients who have parents with seizures (7.0% (21.6%) vs 2.0% (9.8%), p = 0.061).

### 8. *Previous and current anti-epileptic treatment*

### *Previous anti-epileptic treatment*

The number and type of pharmacological treatments for epilepsy used by the patient over the course of the disease were assessed, along with the maximum dose and the reasons for change. The group of patients with DICE showed a significantly higher mean number of previous anti-epileptic drugs (AEDs) than the group of patients with CE (4.3 (2.7) vs 1.8 (1.2), p < 0.001). It was observed that patients in the DICE group had had up to 14 previous AEDs, whereas the maximum number of previous AEDs found in the CE group was 6. Furthermore, it was also observed that a significantly higher percentage of patients with CE (60.2%) controlled the onset of epileptic seizures with the first AED tried, compared to patients with DICE (11.5%) [p < 0.001].

Nineteen (19) different drugs were recorded as used in the total population. Table 4 below presents the 17 AEDs analysed, those used by more than one patient in each group: valproate, carbamazepine, phenytoin, phenobarbital, lamotrigine, topiramate, levetiracetam, oxcarbazepine, clobazam, gabapentin, zonisamide, pregabalin, clonazepam, vigabatrin, primidone, lacosamide and tiagabine. It was found that 16 of them were used significantly more in the DICE than in the CE group (p < 0.05). The only exception was oxcarbazepine, for which a non-significant tendency towards higher use in the DICE group was observed (p = 0.072). Differences were found in the mean maximum dose for 4 AEDs, being higher for patients with DICE than with CE: valproate, carbamazepine, phenobarbital (p < 0.011) and phenytoin (a non-significant tendency, p = 0.061). Considering the change of AED, it was observed that the reason for change from 9 of them was lack of efficacy (patients with DICE) and tolerance (patients with CE): valproate, carbamazepine, phenytoin, phenobarbital, lamotrigine, oxcarbazepine, clobazam, vigabatrin (p < 0.05) and levetiracetam (a non-significant tendency, p = 0.057). For 3 of the AEDs, the reason for change showed no significant differences between the two groups of patients: topiramate, zonisamide and clonazepam. For the 5 remaining AEDS, no statistical test could be applied due to the small number of patients.

**Table 4:** Previous anti-epileptic treatment by study group and in the total population

| | | DICE (n = 279) | CE (n = 186) | Total (n = 465) |
|---|---|---|---|---|
| Valproate | Yes | 127 (45.5%) | 66 (35.5%) | 193 (41.5%) |
| | p-value (chi-squared) | 0.031 | | |
| Maximum dose | Mean | 1,587.5 | 1,362.9 | 1,503.6 |
| | Standard deviation | 610.4 | 405.8 | 552.6 |
| | Median | 1,500.0 | 1,500.0 | 1,500.0 |
| | (Q1; Q3) | (1,225.0; 1,900.0) | (1,000.0; 1,500.0) | (1,000.0; 1,525.0) |
| | (Min; Max) | (200; 4,500) | (500; 2,000) | (200; 4,500) |
| | p-value (Student's t) | 0.011 | | |
| Reason for change | | | | |
| | Lack of efficacy | 92 (78.6%) | 36 (62.1%) | 128 (73.1%) |
| | Tolerance | 11 (9.4%) | 21 (36.2%) | 32 (18.3%) |
| | Both | 14 (12.0%) | 1 (1.7%) | 15 (8.6%) |
| | p-value (chi-squared) | <0.001 | | |
| Carbamazepine | Yes | 131 (47.0%) | 59 (31.7%) | 190 (40.9%) |
| | p-value (chi-squared) | 0.001 | | |
| Maximum dose | Mean | 1,032.4 | 758.5 | 942.2 |
| | Standard deviation | 351.5 | 327.8 | 366.3 |
| | Median | 1,200.0 | 800.0 | 1,000.0 |
| | (Q1; Q3) | (800.0; 1,200.0) | (500.0; 1,100.0) | (600.0; 1,200.0) |
| | (Min; Max) | (100; 2,200) | (100; 1,200) | (100; 2,200) |
| | p-value (Student's t) | <0.001 | | |
| Reason for change | | | | |
| | Lack of efficacy | 89 (71.2%) | 19 (38.0%) | 108 (61.7%) |
| | Tolerance | 29 (23.2%) | 29 (58.0%) | 58 (33.1 %) |
| | Both | 7 (5.6%) | 2 (4.0%) | 9 (5.1%) |
| | p-value (chi-squared) | <0.001 | | |

(continued)

|  |  | DICE (n = 279) | CE (n = 186) | Total (n = 465) |
|---|---|---|---|---|
| Phenytoin | Yes | 120 (43.0%) | 58 (31.2%) | 178 (38.3%) |
|  | p-value (chi-squared) | 0.010 |  |  |
| Maximum dose | Mean | 330.6 | 297.1 | 319.6 |
|  | Standard deviation | 102.9 | 96.3 | 101.7 |
|  | Median | 300.0 | 300.0 | 300.0 |
|  | (Q1; Q3) | (300.0; 350.0) | (300.0; 300.0) | (300.0; 300.0) |
|  | (Min; Max) | (100; 900) | (70; 600) | (70; 900) |
|  | p-value (Student's t) | 0.061 |  |  |
| Reason for change |  |  |  |  |
| Lack of efficacy |  | 89 (74.8%) | 29 (54.7%) | 118 (68.6%) |
| Tolerance |  | 16 (13.4%) | 19 (35.8%) | 35 (20.3%) |
| Both |  | 14 (11.8%) | 5 (9.4%) | 19 (11.0%) |
|  | p-value (chi-squared) | 0.003 |  |  |
| Phenobarbital | Yes | 101 (36.2%) | 34 (18.3%) | 135 (29.0%) |
|  | p-value (chi-squared) | <0.001 |  |  |
| Maximum dose | Mean | 136.5 | 124.0 | 132.9 |
|  | Standard deviation | 60.8 | 60.0 | 60.6 |
|  | Median | 100.0 | 100.0 | 100.0 |
|  | (Q1; Q3) | (100.0; 200.0) | (100.0; 150.0) | (100.0; 187.5) |
|  | (Min; Max) | (20; 350) | (15; 300) | (15; 350) |
|  | p-value (Student's t) | 0.344 |  |  |
| Reason for change |  |  |  |  |
| Lack of efficacy |  | 76 (81.7%) | 20 (62.5%) | 96 (76.8%) |
| Tolerance |  | 14 (15.1%) | 8 (25.0%) | 22 (17.6%) |
| Both |  | 3 (3.2%) | 4 (12.5%) | 7 (5.6%) |
|  | p-value (chi-squared) | 0.046 |  |  |
| Lamotrigine | Yes | 99 (35.5%) | 23 (12.4%) | 122 (26.2%) |
|  | p-value (chi-squared) | <0.001 |  |  |
| Maximum dose | Mean | 277.2 | 248.7 | 271.6 |
|  | Standard deviation | 125.1 | 154.2 | 130.9 |
|  | Median | 275.0 | 200.0 | 200.0 |
|  | (Q1; Q3) | (200.0; 400.0) | (100.0; 400.0) | (200.0; 400.0) |
|  | (Min; Max) | (50; 600) | (50; 600) | (50; 600) |
|  | p-value (Student's t) | 0.397 |  |  |
| Reason for change |  |  |  |  |
| Lack of efficacy |  | 73 (79.3%) | 10 (47.6%) | 83 (73.5%) |
| Tolerance |  | 14 (15.2%) | 10 (47.6%) | 24 (21.2%) |
| Both |  | 5 (5.4%) | 1 (4.8%) | 6 (5.3%) |
|  | p-value (chi-squared) | 0.004 |  |  |
| Topiramate | Yes | 106 (38.0%) | 12 (6.5%) | 118 (25.4%) |

(continued)

| | | | DICE (n = 279) | CE (n = 186) | Total (n = 465) |
|---|---|---|---|---|---|
| Maximum dose | | p-value (chi-squared) | 0.001 | | |
| | | Mean | 300.4 | 350.0 | 305.8 |
| | | Standard deviation | 144.8 | 126.9 | 143.2 |
| | | Median | 300.0 | 400.0 | 300.0 |
| | | (Q1; Q3) | (200.0; 400.0) | (200.0; 400.0) | (200.0; 400.0) |
| | | (Min; Max) | (30; 800) | (200; 600) | (30; 800) |
| | | p-value (Mann-Whitney U) | 0.247 | | |
| Reason for change | | | | | |
| | Lack of efficacy | | 64 (64.0%) | 7 (63.6%) | 71 (64.0%) |
| | Tolerance | | 29 (29.0%) | 2 (18.2%) | 31 (27.9%) |
| | Both | | 7 (7.0%) | 2 (18.2%) | 9 (8.1%) |
| | | p-value (chi-squared) | 0.378 | | |
| Levetiracetam | | Yes | 94 (33.7%) | 15 (8.1%) | 109 (23.4%) |
| | | p-value (chi-squared) | <0.001 | | |
| Maximum dose | | Mean | 2,206.3 | 1,633.3 | 2,115.8 |
| | | Standard deviation | 749.2 | 549.9 | 748.9 |
| | | Median | 2,000.0 | 2,000.0 | 2,000.0 |
| | | (Q1; Q3) | (2,000.0; 3,000.0) | (1,000.0; 3,000.0) | (1,500.0; 3,000.0) |
| | | (Min; Max) | (500; 3,000) | (500; 2,500) | (500; 3,000) |
| | | p-value (Student's t) | 0.006 | | |
| Reason for change | | | | | |
| | Lack of efficacy | | 67 (72.8%) | 6 (46.2%) | 73 (69.5%) |
| | Tolerance | | 16 (17.4%) | 6 (46.2%) | 22 (21.0%) |
| | Both | | 9 (9.8%) | 1 (7.7%) | 10 (9.5%) |
| | | p-value (chi-squared) | 0.057 | | |
| Oxcarbazepine | | Yes | 52 (18.6%) | 23 (12.4%) | 75 (16.1%) |
| | | p-value (chi-squared) | 0.072 | | |
| Maximum dose | | Mean | 1,440.0 | 1,261.9 | 1,378.7 |
| | | Standard deviation | 478.5 | 510.4 | 492.9 |
| | | Median | 1,500.0 | 1,200.0 | 1,200.0 |
| | | (Q1; Q3) | (1,200.0; 1,800.0) | (750.0; 1,800.0) | (1,200.0; 1,800.0) |
| | | (Min; Max) | (300; 2,400) | (600; 2,100) | (300; 2,400) |
| | | p-value (Mann-Whitney U) | 0.197 | | |
| Reason for change | | | | | |
| | Lack of efficacy | | 38 (79.2%) | 9 (45.0%) | 47 (69.1%) |
| | Tolerance | | 8 (16.7%) | 11 (55.0%) | 19 (27.9%) |
| | Both | | 2 (4.2%) | 0 (0.0%) | 2 (2.9%) |

(continued)

| | | DICE (n = 279) | CE (n = 186) | Total (n = 465) |
|---|---|---|---|---|
| | p-value (chi-squared) | 0.004 | | |
| Clobazam | Yes | 51 (18.3%) | 4 (2.2%) | 55 (11.8%) |
| Maximum dose | p-value (chi-squared) | <0.001 | | |
| | Mean | 24.2 | 25.0 | 24.3 |
| | Standard deviation | 12.8 | 5.0 | 12.4 |
| | Median | 20.0 | 25.0 | 20.0 |
| | (Q1; Q3) | (18.8; 40.0) | - | (20.0; 35.0) |
| | (Min; Max) | (2; 60) | (20; 30) | (2; 60) |
| | p-value (Mann-Whitney U) | 0.683 | | |
| Reason for change | | | | |
| Lack ofefficacy | | 37 (86.0%) | 0 (0.0%) | 37 (80.4%) |
| Tolerance | | 3 (7.0%) | 3 (100.0%) | 6 (13.0%) |
| Both | | 3 (7.0%) | 0 (0.0%) | 3 (6.5%) |
| | p-value (chi-squared) | <0.001 | | |
| Gabapentin | Yes | 52 (18.6%) | 2 (1.1%) | 54 (11.6%) |
| Maximum | p-value (chi-squared) | <0.001 | | |
| dose | Mean | 1,773.8 | 2,700.0 | 1,815.9 |
| | Standard deviation | 658.5 | 1,272.8 | 699.5 |
| | Median | 1,800.0 | 2,700.0 | 1,800.0 |
| | (Q1; Q3) | (1,200.0; 2,400.0) | | (1,200.0; 2,400.0) |
| | (Min; Max) | (300; 3,200) | (1,800; 3,600) | (300; 3,600) |
| | p-value (Mann-Whitney U) | 0.256 | | |
| Reason for change | | | | |
| Lack of efficacy | | 45 (93.8%) | 1 (100.0%) | 46 (93.9%) |
| Tolerance | | 2 (4.2%) | 0 (0.0%) | 2 (4.1%) |
| Both | | 1 (2.1%) | 0 (0.0%) | 1 (2.0%) |
| | p-value | NA | | |
| Zonisamide | Yes | 52 (18.6%) | 2 (1.1%) | 54 (11.6%) |
| Maximum dose | p-value (chi-squared) | <0.001 | | |
| | Mean | 341.7 | 300.0 | 339.9 |
| | Standard deviation | 145.2 | 0.0 | 142.3 |
| | Median | 400.0 | 300.0 | 400.0 |
| | (Q1; Q3) | (200.0; 450.0) | (300.0; 300.0) | (200.0; 400.0) |
| | (Min; Max) | (50; 700) | (300; 300) | (50; 700) |
| | p-value (Mann-Whitney U) | 0.599 | | |
| Reason for change | | | | |
| Lack of efficacy | | 33 (64.7%) | 1 (50.0%) | 34 (64.2%) |
| Tolerance | | 13 (25.5%) | 0 (0.0%) | 13 (24.5%) |

(continued)

| | | DICE (n = 279) | CE (n = 186) | Total (n = 465) |
|---|---|---|---|---|
| Both | | 5 (9.8%) | 1 (50.0%) | 6 (11.3%) |
| | p-value (chi-squared) | 0.190 | | |
| Pregabalin | Yes | 47 (16.8%) | 2 (1.1%) | 49 (10.5%) |
| | p-value (chi-squared) | <0.001 | | |
| Maximum dose | Mean | 403.6 | 300.0 | 398.9 |
| | Standard deviation | 150.0 | 0.0 | 148.0 |
| | Median | 300.0 | 300.0 | 300.0 |
| | (Q1; Q3) | (300.0; 600.0) | (300.0; 300.0) | (300.0; 600.0) |
| | (Min; Max) | (150; 600) | (300; 300) | (150; 600) |
| | p-value (Mann-Whitney U) | 0.476 | | |
| Reason for change | | | | |
| Lack of efficacy | | 33 (73.3%) | 2 (100.0%) | 35 (74.5%) |
| Tolerance | | 10 (22.2%) | 0 (0.0%) | 10 (21.3%) |
| Both | p-value | 2 (4.4%) NA | 0 (0.0%) | 2 (4.3%) |
| Clonazepam | Yes | 39 (14.0%) | 6 (3.2%) | 45 (9.7%) |
| | p-value (chi-squared) | <0.001 | | |
| Maximum dose | Mean | 4.7 | 4.7 | 4.7 |
| | Standard deviation | 8.2 | 3.7 | 7.7 |
| | Median | 2.0 | 5.0 | 2.0 |
| | (Q1; Q3) | (1.5; 6.0) | (1.3; 8.0) | (1.5; 6.0) |
| | (Min; Max) | (0.5; 45.0) | (1.0; 10.0) | (0.5; 45.0) |
| | p-value (Mann-Whitney U) | 0.589 | | |
| Reason for change | | | | |
| Lack of efficacy | | 25 (73.5%) | 5 (100.0%) | 30 (76.9%) |
| Tolerance | | 7 (20.6%) | 0 (0.0%) | 7 (17.9%) |
| Both | p-value (chi-squared) | 2 (5.9%) 0.423 | 0 (0.0%) | 2(5.1%) |
| Vigabatrin | Yes | 38 (13.6%) | 7 (3.8%) | 45 (9.7%) |
| Maximum dose | p-value (chi-squared) | <0.001 | | |
| | Mean | 1,990.7 | 1,400.0 | 1,898.4 |
| | Standard deviation | 861.8 | 547.7 | 842.1 |
| | Median | 1,500.0 | 1,500.0 | 1,500.0 |
| | (Q1; Q3) | (1,500.0; 3,000.0) (1,000; | (1,000.0; 1,750.0) | (1,500.0; 2,375.0) |
| | (Min; Max) | 4,500) | (500; 2,000) | (500; 4,500) |
| | p-value (Mann-Whitney U) | 0.263 | | |
| Reason for change | | | | |
| Lack of efficacy | | 28 (80.0%) | 2 (28.6%) | 30 (71.4%) |
| Tolerance | | 5 (14.3%) | 4 (57.1%) | 9 (21.4%) |

(continued)

| | | | DICE (n = 279) | CE (n = 186) | Total (n = 465) |
|---|---|---|---|---|---|
| Both | | p-value (chi-squared) | 2 (5.7%) 0.021 | 1 (14.3%) | 3 (7.1%) |
| Primidone | Yes | | 31 (11.1%) | 3 (1.6%) | 34 (7.3%) |
| | Maximum dose | p-value (chi-squared) | <0.001 | | |
| | | Mean | 671.3 | 375.0 | 644.3 |
| | | Standard deviation | 210.8 | 176.8 | 222.0 |
| | | Median | 750.0 | 375.0 | 750.0 |
| | | (Q1; Q3) | (500.0; 750.0) | - | (500.0; 750.0) |
| | | (Min; Max) | (250; 1,125) | (250; 500) | (250; 1,125) |
| | | p-value (Mann-Whitney U) | 0.104 | | |
| | Reason for change | | | | |
| | | Lack of efficacy | 24 (80.0%) | 1 (33.3%) | 25 (75.8%) |
| | | Tolerance | 3 (10.0%) | 2 (66.7%) | 5 (15.2%) |
| | | Both | 3 (10.0%) | 0 (0.0%) | 3 (9.1%) |
| | | p-value | NA | | |
| Lacosamide | Yes | | 31 (11.1%) | 2 (1.1%) | 33 (7.1 %) |
| | Maximum dose | p-value (chi-squared) | <0.001 | | |
| | | Mean | 276.9 | 250.0 | 275.0 |
| | | Standard deviation | 115.1 | 212.1 | 118.2 |
| | | Median | 300.0 | 250.0 | 300.0 |
| | | (Q1; Q3) | (200.0; 400.0) | - | (200.0; 400.0) |
| | | (Min; Max) | (50; 400) | (100; 400) | (50; 400) |
| | | p-value (Mann-Whitney U) | 0.825 | | |
| | Reason for change | | | | |
| | | Lack of efficacy | 13 (44.8%) | 1 (50.0%) | 14 (45.2%) |
| | | Tolerance | 15 (51.7%) | 1 (50.0%) | 16 (51.6%) |
| | | Both | 1 (3.4%) | 0 (0.0%) | 1 (3.2%) |
| | | p-value | NA | | |
| Tiagabine | Yes | | 27 (9.7%) | 3 (1.6%) | 30 (6.5%) |
| | Maximum dose | p-value (chi-squared) | 0.001 | | |
| | | Mean | 36.2 | 25.0 | 35.0 |
| | | Standard deviation | 12.2 | 14.1 | 12.5 |
| | | Median | 30.0 | 25.0 | 30.0 |
| | | (Q1; Q3) | (30.0; 45.0) | - | (30.0; 45.0) |
| | | (Min; Max) | (20; 60) | (15; 35) | (15; 60) |
| | | p-value (Mann-Whitney U) | 0.421 | | |
| | Reason for change | | | | |
| | | Lack of efficacy | 21 (87.5%) | 1 (33.3%) | 22 (81.5%) |
| | | Tolerance | 2 (8.3%) | 2 (66.7%) | 4 (14.8%) |
| | | Both | 1 (4.2%) | 0 (0.0%) | 1 (3.7%) |

(continued)

| | DICE (n = 279) | CE (n = 186) | Total (n = 465) |
|---|---|---|---|
| p-value | NA | | |

## 9. Current anti-epileptic treatment

The number of current treatments and the maximum dose were evaluated. In the group of CE patients, a significantly higher proportion had a single AED (monotherapy) [79.1% vs 7.6%, p < 0.001]. It was also observed that the patients in the DICE group showed a significantly higher mean number of AEDs [2.7 (0.9) vs 1.3 (0.6), p < 0.001].

Twenty-three (23) different drugs were recorded as used in the total population. Table 5 below shows the 15 AEDs analysed, as they were used by more than one patient in each group: levetiracetam, carbamazepine, lamotrigine, valproate, lacosamide, oxcarbazepine, clobazam, zonisamide, topiramate, phenobarbital, pregabalin, clonazepam, eslicarbazepine, phenytoin and gabapentin. It was observed that 11 of them were used significantly more by the group with DICE than by the group with CE (p < 0.05). Oxcarbazepine, eslicarbazepine, phenytoin and gabapentin showed similar use in both groups. Six AEDs showed mean maximum doses that were significantly higher for patients with DICE than for patients with CE: levetiracetam, carbamazepine, lamotrigine, valproate, oxcarbazepine and zonisamide (p < 0.05).

Table 5: Current anti-epileptic treatment by study group and in the total population

| | | DICE (n = 289) | CE (n = 273) | Total (n = 562) |
|---|---|---|---|---|
| Levetiracetam | Yes | 135 (46.7%) | 77 (28.2%) | 212 (37.7%) |
| | p-value (chi-squared) | <0.001 | | |
| Maximum dose | Mean | 2,477.1 | 1,607.1 | 2,158.1 |
| | Standard deviation | 884.1 | 718.0 | 926.1 |
| | Median | 2,500.0 | 1,500.0 | 2,000.0 |
| | (Q1; Q3) | (2,000.0; 3,000.0) | (1,000.0; 2,000.0) | (1,500.0; 3,000.0) |
| | (Min; Max) p-value (Student's t) | (100; 4,000) <0.001 | (500; 4,000) | (100; 4,000) |
| Carbamazepin e | Yes | 108 (37.4%) | 59 (21.6%) | 167 (29.7%) |
| | p-value (chi-squared) | <0.001 | | |
| Maximum dose | Mean | 1,044.9 | 796.6 | 956.6 |
| | Standard deviation | 327.7 | 327.6 | 347.7 |
| | Median | 1,200.0 | 800.0 | 1,000.0 |
| | (Q1; Q3) | (800.0; 1,200.0) | (600.0; 1,000.0) | (700.0; 1,200.0) |
| | (Min; Max) | (300; 2,200) | (100; 1,200) | (100; 2,200) |
| | p-value (Student's t) | <0.001 | | |
| Lamotrigine | Yes | 81 (28.0%) | 52 (19.0%) | 133 (23.7%) |
| | p-value (chi-squared) | 0.012 | | |
| Maximum dose | Mean | 319.4 | 232.2 | 285.3 |
| | Standard deviation | 146.8 | 116.6 | 141.9 |
| | Median | 300.0 | 200.0 | 275.0 |
| | (Q1; Q3) | (200.0; 400.0) | (150.0; 300.0) | (200.0; 400.0) |
| | (Min; Max) | (50; 800) | (75; 600) | (50; 800) |
| | p-value (Student's t) | 0.001 | | |
| Valproate | Yes | 69 (23.9%) | 40 (14.7%) | 109 (19.4%) |
| | p-value (chi-squared) | 0.006 | | |
| Maximum dose | Mean | 1,369.9 | 1,107.7 | 1,274.3 |
| | Standard deviation | 617.5 | 415.7 | 1,274.3 |
| | Median | 1,500.0 | 1,000.0 | 1,100.0 |

(continued)

|  |  | DICE (n = 289) | CE (n = 273) | Total (n = 562) |
|---|---|---|---|---|
|  | (Q1; Q3) | (1,000.0; 1,500.0) | (800.0; 1,500.0) | (1,000.0; 1,500.0) |
|  | (Min; Max) | (100; 3000) | (500; 2,400) | (100; 3,000) |
|  | p-value (Student's t) | 0.009 |  |  |
| Lacosamide | Yes | 78 (27.0%) | 7 (2.6%) | 85 (15.1%) |
|  | p-value (chi-squared) | <0.001 |  |  |
| Maximum dose | Mean | 316.0 | 366.7 | 319.6 |
|  | Standard deviation | 118.3 | 81.6 | 116.4 |
|  | Median | 325.0 | 400.0 | 375.0 |
|  | (Q1; Q3) | (200.0; 400.0) | (350.0; 400.0) | (200.0; 400.0) |
|  | (Min; Max) | (100; 600) | (200; 400) | (100; 600) |
|  | p-value (Mann-Whitney U) | 0.202 |  |  |
| Oxcarbazepine | Yes | 49 (17.0%) | 34 (12.5%) | 83 (14.8%) |
|  | p-value (chi-squared) | 0.133 |  |  |
| Maximum dose | Mean | 1,441.8 | 1,112.1 | 1,309.1 |
|  | Standard deviation | 458.1 | 382.8 | 456.8 |
|  | Median | 1,200.0 | 1,200.0 | 1,200.0 |
|  | (Q1; Q3) | (1,200.0; 1,800.0) | (900.0; 1,200.0) | (1,162.5; 1,800.0) |
|  | (Min; Max) | (150; 2,400) | (600; 1,800) | (150; 2,400) |
|  | p-value (Mann-Whitney U) | <0.001 |  |  |
| Clobazam | Yes | 46 (15.9%) | 12 (4.4%) | 58 (10.3%) |
|  | p-value (chi-squared) | <0.001 |  |  |
| Maximum dose | Mean | 24.0 | 25.4 | 24.3 |
|  | Standard deviation | 15.2 | 16.0 | 15.3 |
|  | Median | 20.0 | 25.0 | 20.0 |
|  | (Q1; Q3) | (10.0; 40.0) | (11.3; 37.5) | (10.0; 10.0) |
|  | (Min; Max) | (5; 60) | (5;60) | (5; 60) |
|  | p-value (Mann-Whitney U) | 0.788 |  |  |
| Zonisamide | Yes | 48 (16.6%) | 8 (2.9%) | 56 (10.0%) |
|  | p-value (chi-squared) | <0.001 |  |  |
| Maximum dose | Mean | 376.6 | 275.0 | 361.8 |
|  | Standard deviation | 117.0 | 70.7 | 116.7 |
|  | Median | 400.0 | 300.0 | 400.0 |
|  | (Q1; Q3) | (300.0; 500.0) | (200.0; 300.0) | (300.0; 400.0) |
|  | (Min; Max) | (100; 700) | (200; 400) | (100; 700) |
|  | p-value (Mann-Whitney U) | 0.014 |  |  |
| Topiramate | Yes | 36 (12.5%) | 15 (5.5%) | 51 (9.1%) |
|  | p-value (chi-squared) | 0.004 |  |  |
| Maximum dose | Mean | 291.4 | 263.3 | 283.0 |
|  | Standard deviation | 154.2 | 109.3 | 141.7 |
|  | Median | 250.0 | 200.0 | 250.0 |
|  | (Q1; 03) | (200.0; 400.0) | (200.0; 400.0) | (200.0; 400.0) |
|  | (Min; Max) | (50; 700) | (100; 400) | (50; 700) |
|  | p-value (Mann-Whitney U) | 0.658 |  |  |
| Phenobarbital | Yes | 34 (11.8%) | 7 (2.6%) | 41 (7.3%) |
|  | p-value (chi-squared) | <0.001 |  |  |
| Maximum dose | Mean | 125.8 | 100.0 | 122.4 |

(continued)

|  |  |  | DICE (n = 289) | CE (n = 273) | Total (n = 562) |
|---|---|---|---|---|---|
|  |  | Standard deviation | 73.3 | 0.0 | 68.7 |
|  |  | Median | 100.0 | 100.0 | 100.0 |
|  |  | (Q1-Q3) | (100.0; 150.0) | (100.0; 100.0) | (100.0; 150.0) |
|  |  | (Min; Max) | (25; 400) | (100; 100) | (25; 400) |
|  |  | p-value (Mann-Whitney U) | 0.615 |  |  |
| Pregabalin |  | Yes | 11 (3.8%) | 3 (1.1%) | 14 (2.5%) |
|  |  | p-value (chi-squared) | 0.044 |  |  |
|  | Maximum dose | Mean | 456.8 | 450.0 | 455.4 |
|  |  | Standard deviation | 127.5 | 259.8 | 151.3 |
|  |  | Median | 450.0 | 600.0 | 450.0 |
|  |  | (Q1; Q3) | (300.0; 600.0) | - | (300.0; 600.0) |
|  |  | (Min; Max) | (300; 600) | (150; 600) | (150; 600) |
|  |  | p-value (Mann-Whitney U) | 0.885 |  |  |
| Clonazepam |  | Yes | 24 (8.3%) | 5 (1.8%) | 29 (5.2%) |
|  |  | p-value (chi-squared) | 0.001 |  |  |
|  | Maximum dose | Mean | 5.1 | 1.8 | 4.6 |
|  |  | Standard deviation | 9.6 | 1.5 | 8.8 |
|  |  | Median | 1.8 | 1.5 | 1.5 |
|  |  | (Q1; Q3) | (1.5; 6.0) | (0.5 3.3) | (0.9; 3.5) |
|  |  | (Min; Max) | (0.3; 40.0) | (0.5; 4.0) | (0.3; 40.0) |
|  |  | p-value (Mann-Whitney U) | 0.556 |  |  |
|  | Eslicarbazepine | Yes | 6 (2.1%) | 5 (1.8%) | 11 (2.0%) |
|  |  | p-value (chi-squared) | 0.834 |  |  |
|  | Maximum dose | Mean | 666.7 | 720.0 | 690.9 |
|  |  | Standard deviation | 206.6 | 178.9 | 186.8 |
|  |  | Median | 800.0 | 800.0 | 800.0 |
|  |  | (Min; Max) | (400;0, 800.0) | (400;0, 800.0) | 800.0) |
|  |  | p-value (Mann-Whitney U) | 0.792 |  |  |
| Phenytoin |  | Yes | 23 (8.0%) | 16 (5.9%) | 39 (6.9%) |
|  |  | p-value (chi-squared) | 0.328 |  |  |
|  | Maximum dose | Mean | 307.6 | 286.7 | 299.3 |
|  |  | Standard deviation | 113.7 | 64.0 | 96.6 |
|  |  | Median | 300.0 | 300.0 | 300.0 |
|  |  | (Min; Max) | (100;0, 600.0) | (200;0, (100;0, 400.0) | 600.0) |
|  |  | p-value (Mann-Whitney U) | 0.658 |  |  |
|  | Gabapentin | Yes | 8 (2.8%) | 3 (1.1%) | 11 (2.0%) |
|  |  | p-value (chi-squared) | 0.153 |  |  |
|  | Maximum dose | Mean | 1,500.0 | 1,900.0 | 1,609.1 |
|  |  | Standard deviation | 400.0 | 458.3 | 434.6 |
|  |  | Median | 1,600.0 | 1,800.0 | 1,600.0 |
|  |  | (Min; Max) | (800.0; 2,000.0) | (1,500.0; 2,400.0) | (1,500.0; 2,400.0) |
|  |  | p-value (Mann-Whitney U) | 0.376 |  |  |

10. *Electroencephalogram (EEG)*

The number of EEGs, the days of continuous EEG and the findings were recorded. It was found that the mean number of EEGs was similar in both groups [6.3 (8.7) in the total population]. Nevertheless, a higher number of days of continuous EEG was noted for patients with DICE [4.6 (6.1) vs 2.2 (2.0), p = 0.023]. The EEG findings were: normal, focal slowness, focal epileptiform activity, multifocal epileptiform activity and secondary synchrony. It was observed that a significantly higher proportion of patients with CE reported normal activity (29.9% vs 10.6%, p < 0.001), whereas a higher proportion of patients with DICE reported focal epileptiform (50.7% vs 71.1%, p < 0.001) and multifocal epileptiform activity (9.2% vs 4.5%, p = 0.032).

11. *Cerebral magnetic resonance imaging (MRI)*

Information was also collected about cerebral MRI: protocol used, magnetic field and results. Significant differences in the protocol used were observed, with a standard protocol used for the majority of patients with CE (60.0%) and an epilepsy protocol for the majority of patients with DICE (61.4%). Only in a small proportion (1.5%) of patients with DICE were both protocols applied (p < 0.001). Regarding the magnetic field used, it was found that, for a significantly higher proportion of patents with CE, a field of 0.5 teslas was used (5.0% vs 1.4%, p = 0.018), whereas for a significantly higher proportion of patients with DICE, a field of 3.0 teslas was used (19.6% vs 6.5%, p < 0.001). A tendency was also observed, although not significant, for a field of 1.5 teslas to be used a higher proportion of patients with CE (83.1% vs 76.4%, p = 0.053). The possible results of cerebral MRI were: normal, diffuse cortical atrophy, focal atrophy, atrophy of the hippocampus, hippocampal dysplasia, focal cortical dysplasia, diffuse cortical dysplasia, tumour, cavernous haemangioma, arteriovenous malformation and infarction. A significantly higher proportion of patients with CE was found to have a normal result (53.4% vs 40.8%, p = 0.004) or infarction (11.1% vs 4.7%, p = 0.006), whereas a significantly higher proportion of patients with DICE showed atrophy of the hippocampus (26.0% vs 7.5%, p < 0.001) and diffuse cortical dysplasia (3.2% vs 0.4%, p = 0.022). The remaining results were statistically similar, with the following percentages of the total population: diffuse cortical atrophy (2.5%), focal atrophy (9.1 %), hippocampal dysplasia (1.5%), focal cortical dysplasia (8.1%), tumour (4.9%), cavernous haemangioma (2.1%) and arteriovenous malformation (3.0%).

12. *Environmental factors*

The environmental factors taken into account were: smoking, use of mobile telephones, home, employment status, smoking and alcohol consumption. Smoking was similar in both groups (64.0% of the total population were non-smokers), but the mean daily number of cigarettes was significantly higher in the group with DICE [16.3 (9.4) vs 13.3 (10.3), p = 0.013]. The patients with CE showed a significantly higher alcohol consumption (12.5% vs 2.4%, p < 0.001), but the quantity consumed per week was similar for both groups: 3.9 (4.0) units in the total population. The percentage of patients who consumed caffeine, and the mean quantities of coffee, cola and energy drinks consumed per week, were similar for both groups, 48.2% of the total population, 121.0 (481.4), 21.7 (68.7) and 5.4 (35.0) units, respectively. Concerning the number of patients who used a mobile telephone, the mean daily number of minutes of mobile use and the location of the home, the results were similar in both groups, with 82.1% of the total population using a mobile telephone, with a mean of 22.4 (40.8) minutes use per day, and 79.8% of the total population lived in urban areas. The proportion of patents with CE who worked tended to be higher, although this was not significant (48.9% vs 41.4%, p = 0.073), and the proportion of patients with CE who worked night shifts was significantly higher (13.2% vs 5.3%, p = 0.0037).

Genetic factors

The following 44 SNPs were analysed: rs3812718, rs121909673, rs121909672, rs121909674, rs1105879, rs2032582, rs5031017, rs5031016, rs12721627, rs28399433, rs28399468, CYP2B6, MDR1, AGTR1, BDKRB2, GRIN2B, DPYD, IL10, MTHFR, TNF, VKORC1, CYP1A1, CYP1A2, CYP2C19, CYP2C8, CYP2C9, CYP3A4, GSTM3, GSTP1, TPMT, ADRB1, ADRB2, DRD3, HTR2A, HTR2A.1, ADD1, AGT, BCHE, BCHE.1, COMT, ERCC2, TYMS, GSTM1 and GSTT1.

13. *Association analysis for each SNP*

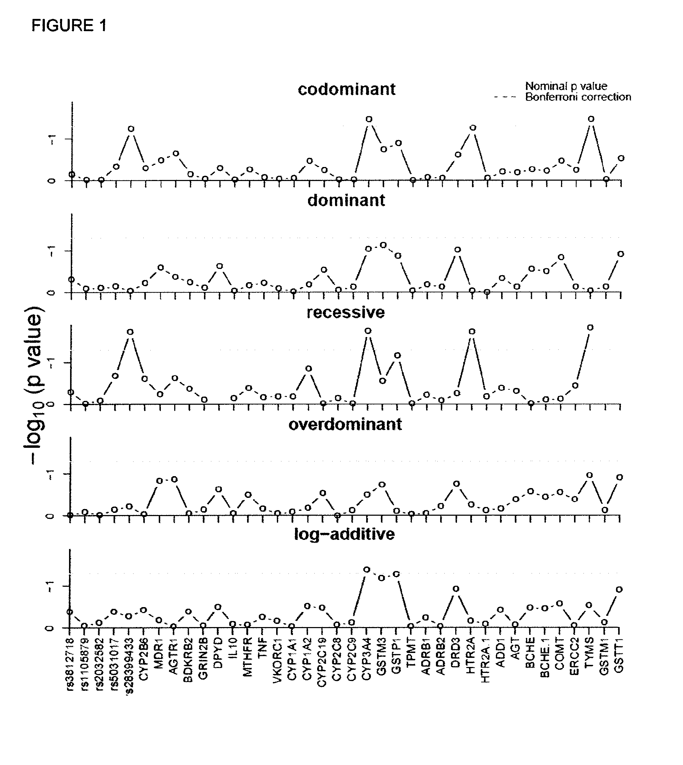

Figure 1 shows the association analysis, SNP by SNP, under distinct inheritance models: codominance, dominance, recessive, overdominance and log-additive. Four SNPs were observed to be significantly associated at the 5% level in the recessive model only but, after correction for multiple comparisons, there was no statistically significant result. A predictive model was then created on the basis of all the genotyped SNPs, assuming an additive effect of the SNP (0: homozygous normal; 1: heterozygous; 2: homozygous variant).

14. *Creation of a predictive model*

*Creation of genetic score*

From the whole set of genotyped SNPs, the set of SNPs that was best associated with the definition of the group of patients with DICE and CE was selected. Based on a stepwise process with the Akaike selection criterion (AIC), the following 5 SNPs were selected: CYP3A4, GSTM3, GSTP1, DRD3 y GSTT1. These polymorphisms constitute the model with the best predictive capacity.

It was found that the 5 SNPs (in univariate form) show a protector effect for patients with DICE, in that patients with the variant allele show a lower likelihood of suffering DICE. Table 5 shows the odds ratios (OR) [EC vs DICE] for each of the SNPs considered under each of the inheritance models. In Table 6, below, it is noted that all the OR were greater than 1, indicating a higher likelihood of presenting EC than DICE.

**Table 6:** Associated risks according to type of epilepsy (N, %): Odds ratio (OR) and 95% confidence interval for the risk of EC/DICE. The results are presented for each inheritance model (with its p-value) and each SNP.

| SNP | DICE | % | Epilepsia controlada | % | OR | IC 95% lower | IC 95% upper | p.value |
|---|---|---|---|---|---|---|---|---|
| **CYP3A4** | | | | | | | | |
| Codominant | | | | | | | | |
| A/A | 212 | 94.60 | 196 | 90.30 | 1 | | | 0.06 |
| A/B | 12 | 5.40 | 17 | 7.80 | 1.53 | 0.71 | 3.29 | |
| B/B | 0 | 0 | 4 | 1.80 | | 0 | | |
| Dominant | | | | | | | | |
| A/A | 212 | 94.60 | 196 | 90.30 | 1 | | | 0.08 |
| A/B-B/B | 12 | 5.40 | 21 | 9.70 | 1.89 | 0.91 | 3.95 | |
| Recessive | | | | | | | | |
| A/A-A/B | 224 | 100 | 213 | 98.20 | 1 | | | 0.06 |
| B/B | 0 | 0 | 4 | 1.80 | | 0 | | |
| Overdominant | | | | | | | | |
| A/A-B/B | 212 | 94.60 | 200 | 92.20 | 1 | | | 0.29 |
| A/B | 12 | 5.40 | 17 | 7.80 | 1.50 | 0.70 | 3.22 | |
| log-Additive | | | | | | | | |
| 0,1,2 | 224 | 50.80 | 217 | 49.20 | 1.99 | 1.03 | 3.86 | 0.06 |
| **GSTM3** | | | | | | | | |
| Codominant | | | | | | | | |
| A/A | 154 | 68.80 | 134 | 61.80 | 1 | | | 0.28 |
| A/B | 60 | 26.80 | 69 | 31.80 | 1.32 | 0.87 | 2 | |
| B/B | 10 | 4.50 | 14 | 6.50 | 1.61 | 0.69 | 3.74 | |
| Dominant | | | | | | | | |
| A/A | 154 | 68.80 | 134 | 61.80 | 1 | | | 0.12 |
| A/B-B/B | 70 | 31.20 | 83 | 38.20 | 1.36 | 0.92 | 2.02 | |
| Recessive | | | | | | | | |
| A/A-A/B | 214 | 95.50 | 203 | 93.50 | 1 | | | 0.36 |
| B/B | 10 | 4.50 | 14 | 6.50 | 1.48 | 0.64 | 3.40 | |
| Overdominant | | | | | | | | |
| A/A-B/B | 164 | 73.20 | 148 | 68.20 | 1 | | | 0.25 |
| A/B | 60 | 26.80 | 69 | 31.80 | 1.27 | 0.84 | 1.92 | |
| log-Additive | | | | | | | | |
| 0,1,2 | 224 | 50.80 | 217 | 49.20 | 1.30 | 0.94 | 1.78 | 0.11 |
| **GSTP1** | | | | | | | | |
| Codominant | | | | | | | | |
| A/A | 114 | 50.90 | 96 | 44.20 | 1 | | | 0.22 |

(continued)

| | | | | | | | | |
|---|---|---|---|---|---|---|---|---|
| **GSTP1** | | | | | | | | |
| A/B | 88 | 39.30 | 90 | 41.50 | 1.21 | 0.81 | 1.81 | |
| B/B | 22 | 9.80 | 31 | 14.30 | 1.67 | 0.91 | 3.08 | |
| Dominant | | | | | | | | |
| A/A | 114 | 50.90 | 96 | 44.20 | 1 | | | 0.16 |
| A/B-B/B | 110 | 49.10 | 121 | 55.80 | 1.31 | 0.90 | 1.90 | |
| Recessive | | | | | | | | |
| A/A-A/B | 202 | 90.20 | 186 | 85.70 | 1 | | | 0.15 |
| B/B | 22 | 9.80 | 31 | 14.30 | 1.53 | 0.86 | 2.74 | |
| Overdominant | | | | | | | | |
| A/A-B/B | 136 | 60.70 | 127 | 58.50 | 1 | | | 0.64 |
| A/B | 88 | 39.30 | 90 | 41.50 | 1.10 | 0.75 | 1.60 | |
| log-Additive | | | | | | | | |
| 0,1,2 | 224 | 50.80 | 217 | 49.20 | 1.27 | 0.96 | 1.67 | 0.09 |
| **DRD3** | | | | | | | | |
| Codominant | | | | | | | | |
| A/A | 115 | 51.30 | 94 | 43.30 | 1 | | | 0.24 |
| A/B | 92 | 41.10 | 103 | 47.50 | 1.37 | 0.93 | 2.03 | |
| B/B | 17 | 7.60 | 20 | 9.20 | 1.44 | 0.71 | 2.90 | |
| Dominant | | | | | | | | |
| A/A | 115 | 51.30 | 94 | 43.30 | 1 | | | 0.09 |
| A/B-B/B | 109 | 48.70 | 123 | 56.70 | 1.38 | 0.95 | 2.01 | |
| Recessive | | | | | | | | |
| A/A-A/B | 207 | 92.40 | 197 | 90.80 | 1 | | | 0.54 |
| B/B | 17 | 7.60 | 20 | 9.20 | 1.24 | 0.63 | 2.43 | |
| Overdominant | | | | | | | | |
| A/A-B/B | 132 | 58.90 | 114 | 52.50 | 1 | | | 0.18 |
| A/B | 92 | 41.10 | 103 | 47.50 | 1.30 | 0.89 | 1.89 | |
| log-Additive | | | | | | | | |
| 0,1,2 | 224 | 50.80 | 217 | 49.20 | 1.27 | 0.95 | 1.71 | 0.11 |
| **GSTT1** | | | | | | | | |
| Codominant | | | | | | | | |
| A/A | 189 | 84.40 | 171 | 78.80 | 1 | | | 0.13 |
| A/B | 35 | 15.60 | 46 | 21.20 | 1.45 | 0.89 | 2.36 | |
| log-Additive | | | | | | | | |
| 0,1,2 | 224 | 50.80 | 217 | 49.20 | 1.45 | 0.89 | 2.36 | |

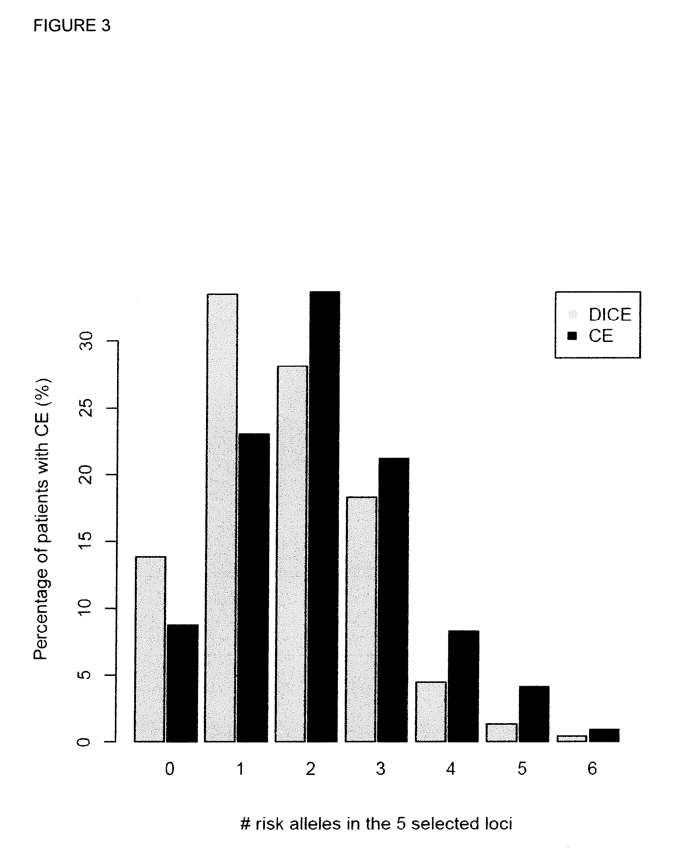

A genetic score (sum of the number of variant alleles for each polymorphism of the unweighted model) was created for the 5 SNPs considered, as well as a score in which the effect of each polymorphism was weighted by the coefficient obtained in the multivariate model [log (OR)]. It was observed that as the number of variant alleles of the selected SNPs increased, the percentage of patients with CE was higher (see Fig. 3). Based on the genetic score, the increase in the likelihood of CE was calculated for each allele that increased the genetic score, observing an increase of 32% [OR: 1.32; 95%CI (1.13; 1.55)] for each increase in a variant allele of the score. As the SNPs considered are protectors for DICE (risk for CE), the patients with a greater number of variant alleles were less prone to present DICE.

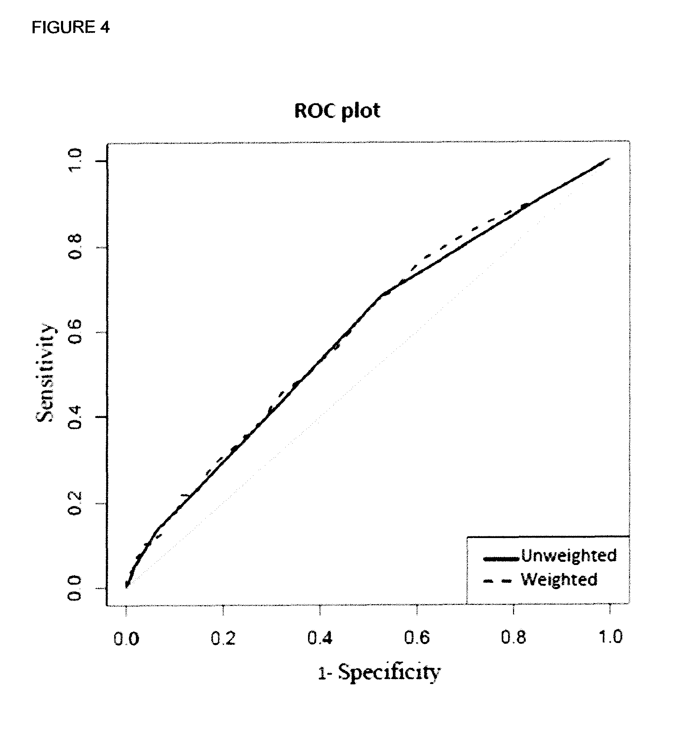

Figure 4 shows the ROC curve and the area under this curve (AUC) where the discriminative power of this model is observed, about 60%. The AUC was similar for both models, weighted and unweighted. The discriminative powers of the unweighted and weighted models, with their 95% confidence intervals, was 0.592 (0.541-0.643) and 0.599 (0.546-0.651), respectively.

15. *Validation of the score*

A validation was carried out by the re-sampling method (crossover validation), given that there was no other population of patients with DICE and CE for the validation. The re-sampling method was carried out by eliminating an individual from the sample. The model was estimated, the prediction was compared with the "new" population (the sample without the individual eliminated at random) and a predictive precision of 0.56 (possible range 0-1) was obtained, with a 95% confidence interval of (0.52-0.61).

16. *Analysis of related phenotypes*

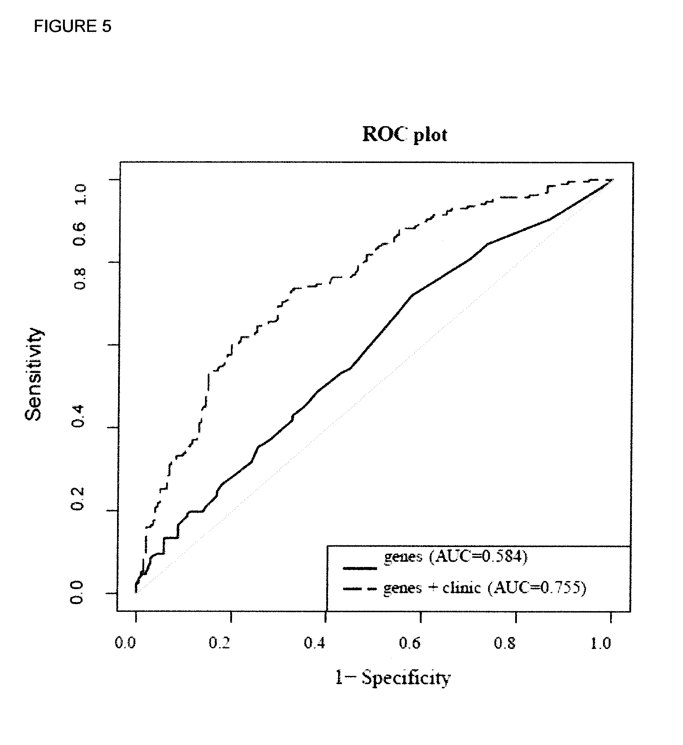

Furthermore, a model was created that took into account clinical as well as genetic factors. The 4 clinical variables that best predicted the likelihood of presenting CE were obtained by an automatic variable selection model, and were: focal seizures with secondary generalisation (CRFO), atrophy of the hippocampus or medial temporal sclerosis as seen on cerebral MRI (RES4), tumour as seen on cerebral MRI (RES8) and age at confirmed diagnosis of epilepsy (edad dx). Table 7, below, shows the risks associated with these variables.
These variables were added to the genetic model, and the discriminative power of the genetic-clinical model was 75.5% (see Fig. 5). The sensitivity and specificity of the model were also calculated at 68.3% and 68.8%, respectively.

**Table 7:** The Odds Ratio (OR) values associated with the clinical variables

|         | OR   | 2.50% | 97.50% |
|---------|------|-------|--------|
| CRFO    | 2.32 | 1.49  | 3.64   |
| RES4    | 0.29 | 0.15  | 0.53   |
| RES8    | 0.35 | 0.12  | 0.9    |
| edad_dx | 1.04 | 1.03  | 1.06   |

genes (AUC = 0.584)

genes + clinical (AUC = 0.755)    1- Specificity

17. *Nomograms*

The nomograms for the prediction of the probability of suffering CE were calculated. Figure 6 shows the clinical factors nomogram and Figure 7 shows the clinico-genetic nomogram. The nomogram with clinical and genetic factors has a greater predictive power than that obtained with only the clinical variables.

18. *Overview over candidate genes to be analysed by an ad-hoc DNA microarray*

| Gene | Polymorphism | rs |
|------|--------------|-----|
| CYP1A2 (allele *1, *1F, *7, *11) | -163A/C | rs762551 |
| | 3534G>A | rs56107638 |
| | 558C>A | - |
| *1C | -3860G>A | |
| CYP1A6 allele *4, *5;*7,*8,*9, *10) | gene deletion | |
| | Gly479Val | rs5031017 |
| | Ile471Thr | rs5031016 |
| | Arg485Leu | rs28399468 |
| | -48T>G | rs28399433 |
| | IVS5+2T>A | - |

(continued)

| Gene | Polymorphism | rs |
|---|---|---|
| CYP2C19 (allele *1, *2, *3, *4, *5, *7, *8, *9, *10) | 1A>G | rs28399504 |
| | 358T>C | rs41291556 |
| | 431G>A | rs17884712 |
| | G636A | rs4986893 |
| | G681A | rs4244285 |
| | C680T | rs6413438 |
| | 1297C>T | rs56337013 |
| | 792C>G | rs1058930 |
| CYP2C8 (allele *1, *2, *3, *4) | A805T | rs11572103 |
| | 1196A>G | rs10509681 |
| | 430C>T | rs1799853 |
| CYP2C9 (allele *1, *2, *3, *4, *5, *6) | 818delA | rs9332131 |
| | 1075A>C | rs1057910 |
| | 1076T>C | rs56165452 |
| | 1080C>G | rs28371686 |
| CYP2D6 (allele *1, *2, *3, *4, *6, *7, *8, *9, *10, *11, *14A, *14B, *15, *17, *18, *19, *20, *25, *29, *31, | -1584C>G | rs1080985 |
| | 31A/G | rs769258 |
| *35, *41) | 100C>T | rs1065852 |
| | 138insT | - |
| | 883C/G | rs5030863 |
| | 1023C>T | rs28371706 |
| | 1039C/T | rs1081003 |
| | G1661C | rs1058164 |
| | 1707T>del | rs5030655 |
| | 1758G>A | rs11568728 |
| | 1758G>T | rs5030865 |
| | 1847A/G | rs3892097 |
| | 1973insG | rs34035805 |
| | 2539delAACT | - |
| | 2549A>del | - |
| | 2613delAGA | rs28371720 |
| | 2850C>T | rs16947 |
| | 2935A/C | rs5030867 |
| | 2988 G>A | rs28371725 |
| | 3183G>A | rs59421388 |
| | 3198C>G | - |
| | 4042G>A | |
| | 4125insGTGCCCACT | |

(continued)

| Gene | Polymorphism | rs |
|---|---|---|
| | 4180G>C | rs1135840 |
| CYP3A4 (allele *1, *1B) *16 | -392A/G | rs2740574 |
| | Thr185Ser | rs12721627 |
| CYP3A5 (allele *1, *3, *6, *9, *10) | 6986G>A | rs776746 |
| | 14690G>A | rs10264272 |
| | 19386G>A | rs28383479 |
| | 29753T>C | rs41279854 |
| ABCB1 (MDR1) | C3435T | rs1045642 |
| | G2677T | rs2032582 |
| ABCC2 (MRP2) | -24C>T | |
| SCN1A | IVS5-91 G>A | |
| | IVS5N+5 G>A | |
| SCN2A | G56A (Arg19Lys) | rs17183814 |
| | IVS7-32A>G | rs2304016 |
| OCTN1 (Leu503Phe) | 1507C>T | rs1050152 |
| GABA(A) | 245G>A (R43Q) | rs28933070 |
| | 529C>G (R139G) | |
| UGT1A6 (allele *3) | 552A>C (R184S) | rs1105879 |
| ADD1 | Gly460Trp | rs4961 |
| ADRB1 | Arg389Gly | rs1801253 |
| ADRB2 | Arg16Gly | rs1042713 |
| AGT | Met235Thr | rs699 |
| AGTR1 | A1166C | rs5186 |
| BCHE | Asp70Gly | rs1799807 |
| BCHE | Ala539Thr | rs1803274 |
| BDKRB2 | C-58T | rs1799722 |
| COMT | Val108Met | rs4680 |
| CYP1A1 | Ile462Val | rs1048943 |
| CYP2B6 | G516T | rs3745274 |
| DPYD | IVS14+1G>A | rs3918290 |
| DRD3 | Ser9Gly | rs6280 |
| ERCC2 | Lys751Gln | rs13181 |
| GRIN2B | C2664T | rs1806201 |
| GSTM1 | null allele | |
| GSTM3 | (-/AGG) | rs1799735 |
| GSTP1 | Ile105Val | rs1695 |
| GSTT1 | null allele | |

(continued)

| Gene | Polymorphism | rs |
|---|---|---|
| HTR2A | His452Tyr | rs6314 |
| | T102C | rs6313 |
| IL10 | -1082G>A | rs1800896 |
| MTHFR | C677T | rs1801133 |
| NAT2 (allele *4, *5A, *5B, *5C, *5D, *6A, *6B, *7A, *7B, *11A, *12A, *12B, *12C, *13, *14A, *14B) | 341C>T | rs1801280 |
| | 481C>T | rs1799929 |
| | 803A>G | rs1208 |
| | 282C>T | rs1041983 |
| | 590G>A | rs1799930 |
| | 857G>A | rs1799931 |
| | 191G>A | rs1801279 |
| TNF | -308G>A | rs1800629 |
| TPMT (allele *1, *2, *3A, *3B, *3C, *3D) | A719G | rs1142345 |
| | G238C | rs1800462 |
| TYMS | 460G/A | rs1800460 |
| VKORC1 | -1639G/A | rs9923231 |

## Example 5

### *Conclusions*

This study was designed with the primary objective of determining the clinical, environmental and genetic factors related to the risk of suffering Difficult-to-Control Epilepsy (DICE). Sixty-two (62) centres participated in the study, recruiting a total of 567 patients, 564 of whom met all the selection criteria and had an analysable DNA sample; in total, the study included 290 patients (51.4%) with DICE and 274 patients (48.6%) with CE.

In general, the groups of patients with DICE and with CE can be classified as homogeneous in terms of biodemographic characteristics and anthropometric data, as they do not show any significant differences in age, sex, weight or height. It was also observed that the groups were similar in terms of birth history, problems and medication during pregnancy, and perinatal problems such as APGAR score, prematurity, postmaturity, hypoglycaemia, neonatal seizures, periventricular haemorrhage and resuscitation. Nevertheless, differences were observed in psychomotor development and perinatal distress, which were proportionally higher in the group of patients with DICE.

In general, concomitant illnesses and treatments were similar among both groups. On analysis of the two subgroups, it was observed that the patients with DICE were prone to illnesses related to the nervous system: depression, psychiatric disorders and treatments for nervous system disorders, whereas the patients with CE were prone to other illnesses such as: cardiac disorders, vascular disorders, treatments of the cardiovascular system, metabolic and nutritional disorders, endocrine disorders, treatments of the digestive tract and metabolism, and treatments of the blood and haematopoietic organs.

The primary variables of the study were clinical, environmental and genetic factors. Concerning environmental factors, the patients were homogeneous in caffeine consumption, use of mobile telephones and location of home (the majority in urban areas). Smoking and employment status were also similar in both groups, but the daily number of cigarettes was higher in the group with DICE and employment on night shifts was higher in patients with CE. More of the patients with CE consumed alcohol, but the quantities consumed per week were similar for both groups.

Concerning clinical factors, the following were evaluated: history of epilepsy, history for febrile seizures and other risk factors, aetiology and type of epilepsy, medical care, status epilepiticus and cognitive status, family history, previous and current anti-epileptic treatment, electroencephalogram (EEG), and cerebral MRI. It was observed that the two groups of patients differed in their history of epilepsy, as patients with DICE presented a longer period since the first seizure, a longer period since the first diagnosis of epilepsy, a higher number of seizures prior to diagnosis, and a higher proportion of complex partial seizures for the first seizure. In relation to this last point, the patients with CE showed a higher proportion of simple partial seizures and generalised seizures for the first seizure. Also, in terms of the different types of seizure that a patient has suffered during the illness, a higher proportion of patients with CE had suffered a generalised seizure or a focal partial seizure with secondary generalisation, whereas a higher proportion of patients with DICE had suffered a complex partial seizure. Furthermore, the group with DICE presented a higher number of seizures in the last year, both partial and generalised, compared with the group with CE. The different types of seizure occurred at the same time of day and in the same cerebral locations for both groups, except for complex partial seizures, where a higher proportion of patients with DICE presented the seizure at any time of day (daytime or while sleeping), and simple partial seizures, which are located in the temporal lobe in a higher proportion of patients with DICE, as opposed to the location in the frontal lobe for patients with CE.

The groups also differed in terms of history of febrile seizures, as a greater number of patients with DICE had presented a seizure of this type. Nevertheless, no differences were found in terms of age at the first and last febrile seizure, or the total number and type of febrile seizures suffered. The two groups were also similar in relation to head injury and brain surgery. However, a higher proportion of patients with CE was observed to have history of stroke or haemorrhage, whereas history of meningitis was observed in a higher proportion of patients with DICE. The temporal type of epilepsy was more frequent in patients with DICE. Also, the groups were different with respect to cause of epilepsy, with the symptomatic aetiology occurring in a greater proportion of patients with DICE, whereas the patients with CE showed a higher proportion of cryptogenic and idiopathic aetiologies. Within symptomatic aetiology, the group with DICE showed a higher proportion with sclerosis of the hippocampus as the symptom, whereas the group with CE showed a higher proportion with vascular and post-traumatic symptoms. The higher proportion of patients with DICE with symptoms of hippocampal sclerosis and of having suffered meningitis could be related to the tendency of this group to suffer nervous system problems. On the other hand, a higher proportion of patients with CE presented epilepsy with vascular symptomology, as well as having suffered stroke and haemorrhage, which could be related to the group's tendency to suffer cardiovascular problems.

It was also observed that the group with DICE more frequently visited the neurologist and the emergency room, as well as being more frequently admitted to hospital. Likewise, it was found that a higher proportion of patients with DICE presented status epilepticus, learning difficulties and were neuropsychologically assessed. There was no difference between the two groups in the percentage of patients with a family history of epilepsy in terms of brothers and sisters, parents, aunts and uncles, grandparents and cousins.

With regards to the quantity and type of pharmacological treatment used by the patient over the course of the illness, maximum dose and reasons for change, the group of patients with DICE presented a larger number of previous anti-epileptic drugs (AEDs). It was also observed that a higher percentage of patients with CE controlled the onset of epileptic seizures with the first AED tried. Likewise, it was observed that all the AEDs had been used by a higher proportion of patients with DICE. Differences in maximum dose between the two groups were only found for 24% of the AEDs, with the dose being higher for patients with DICE. For more than 50% of the AEDs, the reason for change was the lack of efficacy (patients with DICE) or tolerance (patients with CE). On evaluating the number of current treatments and the maximum dose, it was found that the group of patients with CE had a higher proportion of patients on monotherapy. It was also observed that 73% of the AEDs were used more by the group with DICE, and that 40% of the AEDs were used in higher dose for patients with DICE.

In relation to EEG sessions, it was noted that the number of EEGs was similar in both groups, but that the number of days of continuous EEG was higher for the group with DICE. It was also observed that a significantly higher proportion of patients with CE reported normal activity, whereas a higher proportion of patients with DICE reported focal and multifocal epileptiform activity. With regard to cerebral MRI, it was observed that, for the majority of patients with CE, this was carried out with a standard protocol, and for the majority of patients with DICE with an epilepsy protocol. Low magnetic fields (0.5, 1.0 and 1.5 teslas) were used for a higher proportion of patients with CE, whereas high magnetic fields (3.0 teslas) were used in a higher proportion of patients with DICE. The following results of cerebral MRI were similar in both groups: diffuse cortical atrophy, focal atrophy, hippocampal dysplasia, focal cortical dysplasia, tumour, cavernous haemangioma and arteriovenous malformation. Nevertheless, a higher proportion of patients with CE presented a normal result or infarction, whereas a higher proportion of patients with DICE presented atrophy of the hippocampus and diffuse cortical dysplasia. In general, the patients with CE had normal EEG and MRI results, as well as a higher proportion of cases of infarction, possibly related to the vascular system problems mentioned earlier. On the contrary, a higher proportion of patients with DICE was observed to have atrophy of the hippocampus and diffuse cortical dysplasia by MRI, which, along with status epilepticus, learning difficulties and neuropsychological assessment, could be related to the tendency of this group to suffer nervous system problems.

A total of 44 SNPs were analysed in the study. They were selected for relevance to the treatment and aetiology of the illness, with the aim of creating a genetic score capable of predicting type of epilepsy. A multivariate model was obtained that included 5 polymorphisms (CYPP3A4, GSTM3, GSTP1, DRD3 and GSTT1) which were protectors for DICE, presenting a greater risk for CE. A genetic score was constructed with the 5 SNPs, which had a predictive power of 60%. The validation of this score (by resampling) indicated that its capacity to predict the type of epilepsy was 56%.

The most relevant clinical variables for the CE and DICE phenotypes were added to the genetic model, increasing the predictive power of the model to 75%, showing a greater predictive capacity for clinical variables than genetic variables. The inclusion of SNPs in the model for predicting type of epilepsy increased its predictive capacity by approximately 5-10% (6% in validation by resampling). The inclusion of clinical variables substantially improved the results, as the weight of the genetic variables was much lower than that of the clinical variables.

On the basis of this study, it can be concluded that there are certain clinical factors that characterise patients with DICE, such as history of epilepsy and febrile seizures, that they visit the doctor more often, as well as a greater use of AEDs and a greater difficulty in finding effective AEDs. It was observed that the patients with DICE presented a symptomatic aetiology related to disorders of the nervous system, whereas the patients with CE presented a cryptogenic or idiopathic aetiology and a relationship with vascular disorders.

**Claims**

1. A group of biomarkers wherein said group of biomarkers comprises:

    i) SNP rs2740574;
    ii) SNP rs1799735;
    iii) SNP rs1695;
    iv) SNP rs6280; and
    v) the locus of the human glutathione S-transferase theta-1 (GSTT1) gene.

2. The group of biomarkers of claim 1, wherein

    i) SNP rs2740574 is represented by a single polymorphic change at position 21 of SEQ ID NO: 1, where in one or two alleles the wildtype nucleotide A is replaced by indicator nucleotide G;
    ii) SNP rs1799735 is represented by a single polymorphic change at position 21 of SEQ ID NO: 2, where in one or two alleles indicator nucleotides AGG are inserted;
    iii) SNP rs1695 is represented by a single polymorphic change at position 21 of SEQ ID NO: 3, where in one or two alleles wildtype nucleotide A is replaced by indicator nucleotide G;
    iv) SNP rs6280 is represented by a single polymorphic change at position 21 of SEQ ID NO: 4, where in one or two alleles wildtype nucleotide C is replaced by indicator nucleotide T; and
    v) the human glutathione S-transferase theta-1 (GSTT1) gene in one or two alleles is completely deleted, partially deleted and/or non functional.

3. The group of biomarkers of claim 1 or 2, constituting a marker for protection against drug-resistant epilepsy.

4. The group of biomarkers of any one of claims 1 to 3, additionally comprising at least one clinical marker selected from the group comprising:

    i) a focal seizure with secondary generalization;
    ii) age at confirmed diagnosis of epilepsy;
    iii) atrophy of hippocampus or medial temporal sclerosis; and
    iv) the presence of a brain tumor.

5. A method for detecting protection against drug-resistant epilepsy in a subject comprising the steps of:

    i) isolating a nucleic acid from a subject's sample; and
    ii) determining the status of a biomarker as defined in claim 1 or 2;

    wherein the presence of indicator nucleotides, and the presence of a deleted, partially deleted and/or non-functional GSTT1 gene as defined in claim 2 is indicative for a protection against drug-resistant epilepsy.

6. The method of claim 5, wherein said subject has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor.

7. The method of claim 6, wherein said subject has an age of more than 30 years, more than 40 years, more than 45 years, more than 50 years, more than 55 years or more than 60 years at a confirmed diagnosis of epilepsy.

8. The method of claim 5, wherein the method comprises as additional step the determination of a focal seizure with secondary generalization in a subject, the determination of atrophy of hippocampus or medial temporal sclerosis in a subject, the determination of the presence of a brain tumor in a subject and/or the determination of the subject's age at confirmed diagnosis of epilepsy.

9. The method of any one of claims 5 to 8, wherein said determination of the nucleotide sequence and/or molecular structure is carried out through allele-specific oligonucleotide (ASO)-dot blot analysis, primer extension assays, iPLEX SNP genotyping, Dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, SNP microarray based analysis, restriction enzyme fragment length polymorphism (RFLP) analysis, targeted resequencing analysis and/or whole genome sequencing analysis.

10. The method of any one of claims 5 to 9, wherein said method comprises the determination of the haplotype for two copies of the chromosome comprising the SNPs rs2740574; rs1799735; rs1695; and rs6280 and the GSTT1 gene in a subject.

11. A method for monitoring epilepsy therapy comprising the step of determining the status of a biomarker as defined in claim 1 or 2 before and during an epilepsy treatment of a subject, optionally also after an epilepsy treatment.

12. The method of claim 11, wherein said subject has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor.

13. The method of claim 12, wherein said subject has an age of more than 30 years, more than 40 years, more than 45 years, more than 50 years, more than 55 years or more than 60 years at a confirmed diagnosis of epilepsy.

14. The method of claim 11, further comprising the determination of a focal seizure with secondary generalization in a subject, the determination of atrophy of hippocampus or medial temporal sclerosis in a subject, and/or the determination of the presence of a brain tumor in a subject.

15. The method of any one of claims 11 to 14, wherein said treatment is a treatment with at least one anticonvulsant selected from the group of lacosamide, levetiracetam, brivaracetam, phenobarbital, primidone, midazolam, clonazepam, topiramate, carbamazepine, oxcarbazepine, eslicarbazepine, mesuximide, ethosuximide, valproic acid and salts thereof, tiagabine, vigabatrine, gabapentin, pregabalin, phenytoin, lamotrigine, sultiam, felbamate, retigabine, perampanel and zonisamide, or combinations thereof.

16. A method of identifying an individual as eligible for an aggressive epilepsy therapy comprising:

    i) determining in a sample obtained from an individual the status of a biomarker as defined in claim 1 or 2;
    ii) optionally determining a focal seizure with secondary generalization in a subject, determining atrophy of hippocampus or medial temporal sclerosis in a subject, determining the presence of a brain tumor in a subject and/or determining the subject's age at confirmed diagnosis of epilepsy;
    iii) identifying the individual as eligible to receive an aggressive epilepsy therapy where the individual's sample is classified as showing the presence of indicator nucleotides, and the occurrence of a deleted, partially deleted and/or non-functional GSTT1 gene as defined in claim 2; optionally also showing focal seizure with secondary generalization in a subject, showing no atrophy of hippocampus or medial temporal sclerosis in a subject, showing no brain tumor in a subject and/or wherein the subject's age at confirmed diagnosis of epilepsy is above 45 years.

17. The method of any one of claims 5 to 16, wherein said sample is a mixture of tissues, organs, cells and/or fragments thereof, or a tissue or organ specific sample, such as a tissue biopsy from tongue, pancreas, liver, spleen, ovary, muscle, joint tissue, neural tissue, gastrointestinal tissue, or a body fluid such as blood, or saliva.

18. A composition for detecting protection against drug-resistant epilepsy in a subject, comprising a nucleic acid affinity ligand for a biomarker as defined in claim 1 or 2.

19. The composition of claim 18, wherein said oligonuceotide is a oligonucleotide specific for an indicator nucleotide as defined in claim 2 or the corresponding wildtype nucleotide, or specific for the GSTT1 gene, preferably an oligonucleotide having a sequence complementary to an indicator nucleotide as defined in claim 2, or to the corresponding wildtype nucleotide.

20. A kit for detecting protection against drug-resistant epilepsy in a subject, comprising a nucleic acid affinity ligand for a biomarker as defined in claim 1 or 2.

21. The kit of claim 20, further comprising an enzyme for primer elongation, nucleotides and/or labeling agents.

22. A microarray for the analysis of protection against drug-resistant epilepsy in a subject, comprising at least one probe selective for an indicator nucleotide or the corresponding wildtype nucleotide, and the GSTT1 gene as defined in claim 2.

23. Use of a group of biomarkers as defined in claim 1 or 2 for detecting protection against drug-resistant epilepsy in a subject, or for screening a population of subjects for the presence of drug-resistant epilepsy.

24. The use of claim 23, wherein said subject or population of subjects has been diagnosed or can be diagnosed (i) as being afflicted by a focal seizure with secondary generalization, and/or (ii) as not being afflicted by atrophy of hippocampus or medial temporal sclerosis and/or (iii) as not being afflicted by a brain tumor.

25. The use of claim 24 wherein said subject has an age of more than 30 years, more than 40 years, more than 45 years, more than 50 years, more than 55 years or more than 60 years at a confirmed diagnosis of epilepsy.

FIGURE 1

FIGURE 2

Genotype missing data

FIGURE 3

# risk alleles in the 5 selected loci

FIGURE 4

ROC plot

FIGURE 5

ROC plot

FIGURE 6

**Nomogram: Clinical variables**

FIGURE 7

# Nomogram: Clinical and genetic variables

Europäisches
Patentamt

European
Patent Office

Office européen
des brevets

# EUROPEAN SEARCH REPORT

Application Number

EP 13 16 4508

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PURANIK YOGITA GHODKE ET AL: "Association of carbamazepine major metabolism and transport pathway gene polymorphisms and pharmacokinetics in patients with epilepsy", PHARMACOGENOMICS, ASHLEY PUBLICATIONS, GB, vol. 14, no. 1, 1 January 2013 (2013-01-01), pages 35-45, XP008163573, ISSN: 1462-2416 | 18,19 | INV. C12Q1/68 |
| Y | * page 37 * <br> * page 40 * <br> * page 36, last paragraph * <br> ----- | 1-17, 20-25 | |
| Y | ERCEGOVAC M ET AL: "GLUTATHIONE TRANSFERASE POLYMORPHISMS IN PATIENTS WITH PROGRESSIVE MYOCLONIC EPILEPSY", EPILEPSIA; 10TH EUROPEAN CONGRESS ON EPILEPTOLOGY, RAVEN PRESS LTD, NEW YORK, US; LONDON, UK, vol. 53, no. Suppl. 5 Special issue, 4 September 2012 (2012-09-04), page 21, XP008163527, ISSN: 0013-9580, DOI: 10.1111/J.1528-1167.2012.03677.X [retrieved on 2012-09-11] * abstract * <br> ----- <br> -/-- | 1-25 | TECHNICAL FIELDS SEARCHED (IPC) <br><br> C12Q |

The present search report has been drawn up for all claims

| Place of search | Date of completion of the search | Examiner |
|---|---|---|
| The Hague | 18 July 2013 | Reuter, Uwe |

EPO FORM 1503 03.82 (P04C01)

## EUROPEAN SEARCH REPORT

**Application Number**

EP 13 16 4508

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OJOPI E B ET AL: "NON-RESPONSE TO ANTI-EPILEPTIC DRUG THERAPY WITH VALPROATE IS ASSOCIATED TO CYP2C9*2 AND*3 GENOTYPE IN YOUNG PATIENTS WITH EPILEPSY", EPILEPSIA, RAVEN PRESS LTD, NEW YORK, US, vol. 50, no. Supplement 11, 8 December 2009 (2009-12-08), page 480, XP008163543, ISSN: 0013-9580, DOI: 10.1111/J.1528-1167.2009.02377_1.X [retrieved on 2009-10-06] * abstract * | 1-25 | |
| Y | DATABASE BIOSIS [Online] BIOSCIENCES INFORMATION SERVICE, PHILADELPHIA, PA, US; October 2011 (2011-10), LI YAN-MEI ET AL: "Relationship between single nucleotide polymorphisms of CYP3A4 and drug resistant epilepsy in Han Chinese children", XP002701565, Database accession no. PREV201200058542 * abstract * & ZHONGGUO XINYAO YU LINCHUANG ZAZHI, vol. 30, no. 10, October 2011 (2011-10), pages 740-745, ISSN: 1007-7669 | 1-25 | **TECHNICAL FIELDS SEARCHED (IPC)** |

-/--

The present search report has been drawn up for all claims

| Place of search | Date of completion of the search | Examiner |
|---|---|---|
| The Hague | 18 July 2013 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 13 16 4508

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FUKUSHIMA ET AL: "Glutathione-S-transferase (GST) M1 null genotype and combined GSTM1 and GSTT1 null genotypes are risk factors for increased serum gamma-glutamyltransferase in valproic acid-treated patients", CLINICA CHIMICA ACTA, ELSEVIER BV, AMSTERDAM, NL, vol. 389, no. 1-2, 29 January 2008 (2008-01-29), pages 98-102, XP022438010, ISSN: 0009-8981, DOI: 10.1016/J.CCA.2007.11.035 * page 100 * | 1-25 | |
| X | ALMOGUERA BERTA ET AL: "Evaluating a newly developed pharmacogenetic array: screening in a Spanish population.", PHARMACOGENOMICS NOV 2010, vol. 11, no. 11, November 2010 (2010-11), pages 1619-1625, XP002702941, ISSN: 1744-8042 * abstract * * page 1623, paragraph "conclusion" * | 1-4, 18-22 | |
| | -/-- | | |

|  |  |  |
|---|---|---|
| TECHNICAL FIELDS SEARCHED (IPC) | | |

The present search report has been drawn up for all claims

| Place of search | Date of completion of the search | Examiner |
|---|---|---|
| The Hague | 18 July 2013 | Reuter, Uwe |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

# EP 2 792 754 A1

## EUROPEAN SEARCH REPORT

Application Number

EP 13 16 4508

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Clarice Chemello: "Atención farmacéutica al paciente con insusficiencia renal crónica: seguimiento farmacoterapéutico y farmacogenética", Universidad de Granada, tesis doctoral , 31 December 2011 (2011-12-31), XP002702942, Granada, Spain Retrieved from the Internet: URL:http://hera.ugr.es/tesisugr/1966428x.pdf [retrieved on 2013-07-16] * table 5.4 * | 1-4, 18-22 | |
| X | SARUWATARI J ET AL: "Update on the genetic polymorphisms of drug-metabolizing enzymes in antiepileptic drug therapy", PHARMACEUTICALS 2010 MOLECULAR DIVERSITY PRESERVATION INTERNATIONAL CHE, vol. 3, no. 8, 2010, pages 2709-2732, XP002702943, ISSN: 1424-8247 * abstract; table 3 * | 1-25 | TECHNICAL FIELDS SEARCHED (IPC) |

The present search report has been drawn up for all claims

| Place of search | Date of completion of the search | Examiner |
|---|---|---|
| The Hague | 18 July 2013 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 792 754 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FORSGREN et al.** *Eur J Neurol,* 2005, vol. 12, 245-53
- **GLAUSER et al.** *Epilepsia,* 2006, vol. 47 (7), 1094-120
- **FRENCH et al.** *Neurology,* 2004, vol. 62, 1261-73
- **TOMSON et al.** *ancet Neurol,* 2008, vol. 7, 1021-31
- **KWAN et al.** *Epilepsia,* 2009
- **KWAN et al.** *N Engl J Med,* 2000, vol. 342, 314-9
- **SHORVON.** *Epilepsia,* 1996, vol. 37, S1-S3
- **PERUCCA.** *CNS Drugs,* 1998, vol. 10, 171-9
- **FRENCH.** *Epilepsia,* 2007, vol. 48, S3-S7
- **SIDDIQUI et al.** *N Engl J Med,* 2003, vol. 348, 1442-8
- **TATE et al.** *Proc Natl Acad Sci,* 2005, vol. 102, 5507-12
- **ESCAYG et al.** *Am J Hum Genet,* 2001, vol. 68, 866-73
- **PIRMOHAMED et al.** *Neurology,* 2001, vol. 56, 890-6
- **SARUWATARI et al.** *Pharmaceuticals,* 2010, vol. 3, 2709-2732
- Intracranial EEG and localization studies. **BEN-BADIS et al.** The treatment of epilepsy: principles and practice. Lippincott Williams & Wilkins, 2001
- **YASUDA et al.** *Expert Rev Neurother.,* 2010, vol. 10 (6), 975-984
- *Tripartite Harmonised ICH Guidelines for Good Clinical Practice,* 1996
- International Guidelines for Ethical Review of Epidemiological Studies. Spanish Epidemiology Society, 1991